# EUROPÄISCHE PATENTSCHRIFT

(11) **EP 1 987 026 B1**
(45) Veröffentlichungstag und Bekanntmachung des Hinweises auf die Patenterteilung: **26.08.2009**
(21) Anmeldenummer: 07703379.3
(22) Anmeldetag: 06.02.2007
(51) Int. Cl.: C07D 413/14, A61P 7/02, A61K 31/422

(54) **AMINOACYL-PRODRUG DERIVATE UND ARZNEIMITTEL ZUR BEHANDLUNG VON THROMBOEMBOLISCHEN ERKRANKUNGEN**
AMINOACYL PRODRUG DERIVATIVES AND MEDICAMENTS FOR THE TREATMENT OF THROMBOEMBOLITIC DISORDERS
DÉRIVÉS PROMÉDICAMENTEUX AMINOACYLES ET MÉDICAMENTS DESTINÉS AU TRAITEMENT DE MALADIES THROMBOEMBOLIQUES

(30) Priorität: 16.02.2006 DE 102006007146
(43) Veröffentlichungstag der Anmeldung: 05.11.2008
(73) Patentinhaber: Bayer HealthCare AG, 51368 Leverkusen (DE)
(72) Erfinder: LERCHEN, Hans-Georg, 51375 Leverkusen (DE); KRENZ, Ursula, 42799 Leichlingen (DE); SCHLEMMER, Karl-Heinz, 42113 Wuppertal (DE); PERZBORN, Elisabeth, 42327 Wuppertal (DE); KELDENICH, Joerg, 42113 Wuppertal (DE)
(86) Internationale Anmeldenummer: PCT/EP2007/001135
(87) Internationale Veröffentlichungsnummer: WO 2007/093328

(56) Entgegenhaltungen:
- WO-A-01/00622
- WO-A-20/05028473
- ROEHRIG S ET AL: "Discovery of the Novel Antithrombotic Agent 5-Chloro-N-(((5S)-2-oxo-3 [4-(3-oxomorpholin-4-yl)phenyl]-1,3-oxazol idin-5-yl)methyl)thiophene 2-carboxamide (BAY 59-7939): An Oral, Direct Factor Xa Inhibitor" JOURNAL OF MEDICINAL CHEMISTRY, AMERICAN CHEMICAL SOCIETY. WASHINGTON, US, Bd. 48, 22. September 2005 (2005-09-22), Seiten 5900-5908, XP002418821 ISSN: 0022-2623 in der Anmeldung erwähnt
- KAHNS A H ET AL: "N ACYL DERIVATIVES AS PRODRUG FORMS FOR AMIDES CHEMICAL STABILITY AND ENZYMATIC HYDROLYSIS OF VARIOUS N ACYL AND N ALKOXYCARBONYL AMIDE DERIVATIVES" INTERNATIONAL JOURNAL OF PHARMACEUTICS (KIDLINGTON), Bd. 71, Nr. 1-2, 1991, Seiten 31-44, XP002440260 ISSN: 0378-5173

## Beschreibung

Die vorliegende Anmeldung betrifft Prodrug-Derivate von 5-Chlor-*N-*({(5S)-2-oxo-3-[4-(3-oxo-morpholin-4-yl)phenyl]-1,3-oxazolidin-5-yl}methyl)thiophen-2-carboxamid, Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere von thromboembolischen Erkrankungen.

Prodrugs sind Derivate eines Wirkstoffs, die *in vivo* eine ein- oder mehrstufige Biotransformation enzymatischer und/oder chemischer Art durchlaufen, bevor der eigentliche Wirkstoff freigesetzt wird. Ein Prodrug-Rest wird in der Regel genutzt, um das Eigenschaftsprofil des zu Grunde liegenden Wirkstoffs zu verbessern [P. Ettmayer et al., J. Med. Chem. 47, 2393 (2004)). Um ein optimales Wirkprofil zu erreichen, muss dabei das Design des Prodrug-Restes ebenso wie der angestrebte Freisetzungsmechanismus sehr genau auf den individuellen Wirkstoff, die Indikation, den Wirkort und die Applikationsroute abgestimmt werden. Eine große Zahl von Arzneimitteln wird als Prodrugs verabreicht, die gegenüber dem zu Grunde liegenden Wirkstoff eine verbesserte Bioverfügbarkeit aufweisen, beispielsweise erzielt durch eine Verbesserung des physikochemischen Profils, speziell der Löslichkeit, der aktiven oder passiven Absorptionseigenschaften oder der gewebsspezifischen Verteilung. Aus der umfangreichen Literatur über Prodrugs sei beispielhaft genannt: H. Bundgaard (Ed.), Design of Prodrugs: Bioreversible derivatives for various functional groups and chemical entities, Elsevier Science Publishers B.V., 1985.

5-Chlor-*N-*({(5S)-2-oxo-3-[4-(3-oxomorpholin-4-yl)phenyl]-1,3-oxazolidin-5-yl}methyl)thiophen-2-carboxamid [BAY 59-7939, Verbindung (A)] ist ein oral wirksamer, direkter Inhibitor der Serin-Protease Faktor Xa, welche eine essentielle Funktion bei der Regulation der Blutgerinnung ausübt. Die Verbindung, befindet sich gegenwärtig in vertiefter klinischer Prüfung als möglicher neuer Arzneimittelwirkstoff für die Prävention und Therapie thromboembolischer Erkrankungen [S. Roehrig et al., J. Med. Chem. 48, 5900 (2005)].

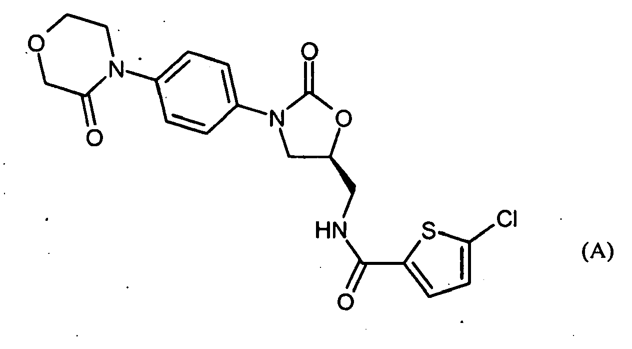

Verbindung (A) weist jedoch nur eine begrenzte Löslichkeit in Wasser und physiologischen Medien auf, was beispielsweise eine intravenöse Applikation des Wirkstoffs erschwert. Aufgabe der vorliegenden Erfindung war daher die Identifizierung von Derivaten oder Prodrugs von Verbindung (A), die eine verbesserte Löslichkeit in den genannten Medien besitzen und gleichzeitig nach Applikation eine kontrollierte Freisetzung des Wirkstoffs (A) im Körper des Patienten erlauben.

In WO 2005/028473 werden Acyloxymethylcarbamat-Prodrugs von Oxazolidinonen beschrieben, die einer Erhöhung der oralen Bioverfügbarkeit dienen. In WO 01/00622 werden Acyl-Prodrugs von Carbamat-Inhibitoren der Inosin-5'-monophosphat-Dehydrogenase offenbart. Eine weitere Art von Amid-Prodrugs für Oxazolidinone, die über einen mehrstufigen Aktivierungsmechanismus den zu Grunde liegenden Wirkstoff freisetzen, ist in WO 03/006440 beschrieben.

### Gegenstand der vorliegenden Erfindung sind Verbindungen der allgemeinen Formel (I)

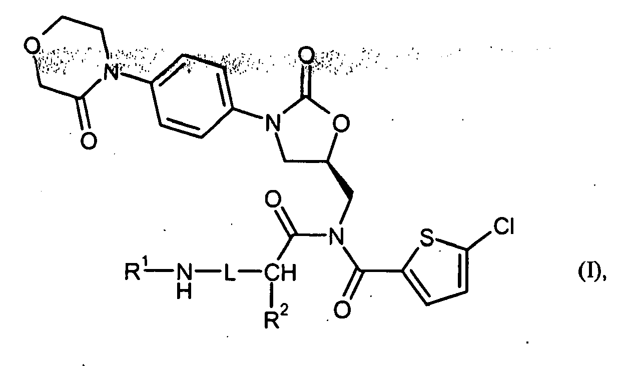

in welcher
- R¹: für Wasserstoff oder (C₁-C₄)-Alkyl, das mit Hydroxy oder (C₁-C₄)-Alkoxy substituiert sein kann, steht,
- R²: für Wasserstoff oder (C₁-C₄)-Alkyl steht
und
- L: für eine (C₁-C₄)-Alkandiyl-Gruppe, in welcher eine CH₂-Gruppe gegen ein O-Atom ausgetauscht sein kann, oder für eine Gruppe der Formel 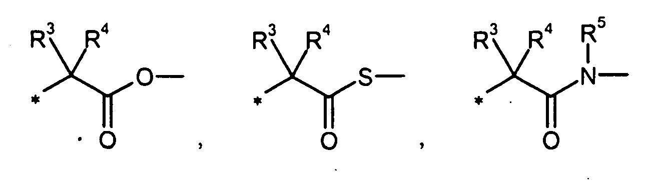
oder 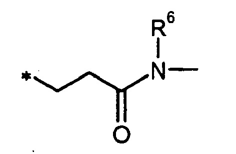 steht, worin
- *: die Verknüpfungsstelle mit dem N-Atom bedeutet,
- R³: die Seitengruppe einer natürlichen α-Aminosäure oder ihrer Homologe oder Isomere bedeutet
oder
- R³: mit R¹ verknüpft ist und beide zusammen eine (CH₂)₃- oder (CH₂)₄-Gruppe bilden,
- R⁴: Wasserstoff oder Methyl bedeutet,
- R⁵: (C₁-C₄)-Alkyl bedeutet
und
- R⁶: Wasserstoff oder (C₁-C₄)-Alkyl bedeutet,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Im Rahmen der vorliegenden Erfindung haben die Substituenten, soweit nicht anders spezifiziert, die folgende Bedeutung:

C₁-C₄)-Alkyl und (C₁-C₃)-Alkyl stehen im Rahmen der Erfindung für einen geradkettigen oder verzweigten Alkylrest mit 1 bis 4 bzw. 1 bis 3 Kohlenstoffatomen. Bevorzugt ist ein geradkettiger Alkylrest mit 1 bis 3 Kohlenstoffatomen. Beispielhaft und vorzugsweise seien genannt: Methyl, Ethyl, n-Propyl, Isopropyl, n-Butyl, Isobutyl, sec.-Butyl, *tert.*-Butyl.

(C₁-C₄)-Alkoxy steht im Rahmen der Erfindung für einen geradkettigen oder verzweigten Alkoxyrest mit 1 bis 4 Kohlenstoffatomen. Beispielhaft und vorzugsweise seien genannt: Methoxy, Ethoxy, n-Propoxy, Isopropoxy, n-Butoxy, *tert*.-Butoxy.

(C₁-C₄)-Alkandiyl steht im Rahmen der Erfindung für einen geradkettigen oder verzweigten divalenten Alkylrest mit 1 bis 4 Kohlenstoffatomen. Bevorzugt ist ein geradkettiger Alkandiylrest mit 2 bis 4 Kohlenstoffatomen. Beispielhaft und vorzugsweise seien genannt: Methylen, 1,2-Ethylen, Ethan-1,1-diyl, 1,3-Propylen, Propan-1,1-diyl, Propan-1,2-diyl, Propan-2,2-diyl, 1,4-Butylen, Butan-1,2-diyl, Butan-1,3-diyl, Butan-2,3-diyl.

Die Seitengruppe einer α-Aminosäure in der Bedeutung von R³ umfasst sowohl die Seitengruppen der natürlich vorkommenden α-Aminosäuren als auch die Seitengruppen von Homologen und Isomeren dieser α-Aminosäuren. Die α-Aminosäure kann hierbei sowohl in der L- als auch in der D-Konfiguration oder auch als Gemisch der L- und D-Form vorliegen. Als Seitengruppen seien beispielhaft genannt: Wasserstoff (Glycin), Methyl (Alanin), Propan-2-yl (Valin), Propan-1-yl (Norvalin), 2-Methylpropan-1-yl (Leucin), 1-Methylpropan-1-yl (Isoleucin), Butan-1-yl (Norleucin), Phenyl (2-Phenylglycin), Benzyl (Phenylalanin), p-Hydroxybenzyl (Tyrosin), Indol-3-ylmethyl (Tryptophan), Imidazol-4-ylmethyl (Histidin), Hydroxymethyl (Serin), 2-Hydroxyethyl (Homoserin), 1-Hydroxyethyl (Threonin), Mercaptomethyl (Cystein), Methylthiomethyl (S-Methylcystein), 2-Mercaptoethyl (Homocystein), 2-Methylthioethyl (Methionin), Carbamoylmethyl (Asparagin), 2-Carbamoylethyl (Glutamin), Carboxymethyl (Asparaginsäure), 2-Carboxyethyl (Glutaminsäure), 4-Aminobutan-1-yl (Lysin), 4-Amino-3-hydroxybutan-1-yl (Hydroxylysin), 3-Aminopropan-1-yl (Omithin), 3-Guanidinopropan-1-yl (Arginin), 3-Ureidopropan-1-yl (Citrullin) Bevorzugte α-Aminosäure-Seitengruppen in der Bedeutung von R³ sind Wasserstoff (Glycin), Methyl (Alanin), Propan-2-yl (Valin), Propan-1-yl (Norvalin), Imidazol-4-ylmethyl (Histidin), Hydroxymethyl (Serin), 1-Hydroxyethyl (Threonin), Carbamoylmethyl (Asparagin), 2-Carbamoylethyl (Glutamin), 4-Aminobutan-1-yl (Lysin), 3-Aminopropan-1-yl (Ornithin), 3-Guanidino-propan-1-yl (Arginin). Bevorzugt ist jeweils die L-Konfiguration.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit einem oder zwei gleichen oder verschiedenen Substituenten ist bevorzugt. Ganz besonders bevorzugt ist die Substitution mit einem Substituenten.

Bevorzugt sind Verbindungen der Formel (I), in welcher
- R¹: für Wasserstoff oder (C₁-C₄)-Alkyl steht,
- R²: für Wasserstoff steht
und
- L: für eine (C₂-C₄)-Alkandiyl-Gruppe oder für eine Gruppe der Formel 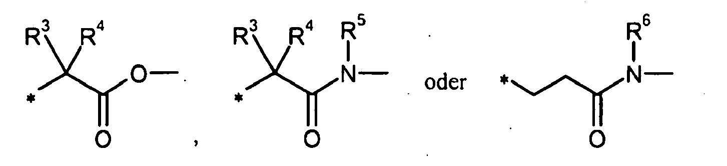
steht, worin
- *: die Verknüpfungsstelle mit dem N-Atom bedeutet,
- R³: Wasserstoff, Methyl, Propan-2-yl, Propan-1-yl, Imidazol-4-ylmethyl, Hydroxymethyl, 1-Hydroxyethyl, Carbamoylmethyl, 2-Carbamoylethyl, 4-Aminobutan-1-yl, 3-Aminopropan-1-yl oder 3-Guanidinopropan-1-yl bedeutet
oder
- R³: mit R¹ verknüpft ist und beide zusammen eine (CH₂)₃- oder (CH₂)₄-Gruppe bilden,
- R⁴: Wasserstoff oder Methyl bedeutet,
- R⁵: Methyl bedeutet
und
- R⁶: Wasserstoff oder Methyl bedeutet,
sowie ihre Salze, Solvate und Solvate der Salze.

Von besonderer Bedeutung hierbei sind Verbindungen der Formel (I), in welcher
- R¹: für Wasserstoff oder (C₁-C₃)-Alkyl steht. Von besonderer Bedeutung sind auch Verbindungen der Formel (I), in welcher
- L: für eine geradkettige (C₂-C₄)-Alkandiyl-Gruppe steht. Besonders bevorzugt sind Verbindungen der Formel (I), in welcher
- R¹: für Wasserstoff, Methyl oder n-Butyl steht,
- R²: für Wasserstoff steht
und
- L: für eine CH₂CH₂-Gruppe oder für eine Gruppe der Formel 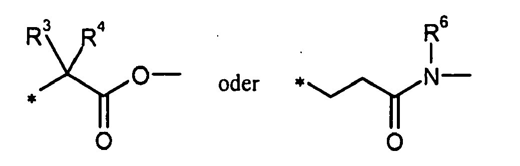
steht, worin
- *: die Verknüpfungsstelle mit dem N-Atom bedeutet,
- R³: Wasserstoff, Methyl, Propan-2-yl, Propan-1-yl, Imidazol-4-ylmethyl, Hydroxymethyl, 1-Hydroxyethyl, Carbamoylmethyl, 2-Carbamoylethyl, 4-Aminobutan-1-yl, 3-Aminopropan-1-yl oder 3-Guanidinopropan-1-yl bedeutet
oder
- R³: mit R¹ verknüpft ist und beide zusammen eine (CH₂)₃- oder (CH₂)₄-Gruppe bilden,
- R⁴: Wasserstoff oder Methyl bedeutet
und
- R⁶: Wasserstoff oder Methyl bedeutet,
sowie ihre Salze, Solvate und Solvate der Salze.

Von besonderer Bedeutung hierbei sind Verbindungen der Formel (I), in welcher
- R¹: für Wasserstoff oder Methyl steht. Von besonderer Bedeutung sind auch Verbindungen der Formel (I), in welcher
L für eine CH₂CH₂-Gruppe steht.

Weiterer Gegenstand der Erfindung ist ein Verfahren zur Herstellung der erfindungsgemäßen Verbindungen der Formel (I), dadurch gekennzeichnet, dass man entweder

### [A] die Verbindung (A)

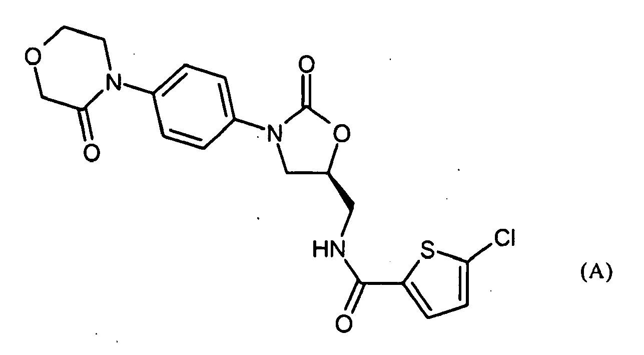

zunächst in einem inerten Lösungsmittel in Gegenwart einer Base mit einer Verbindung der Formel (II) 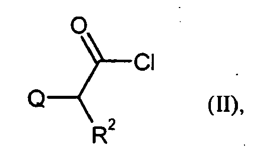 in welcher R² die oben angegebene Bedeutung hat
und
Q für eine Abgangsgruppe wie beispielsweise Chlor, Brom oder Iod steht,
in eine Verbindung der Formel (III) 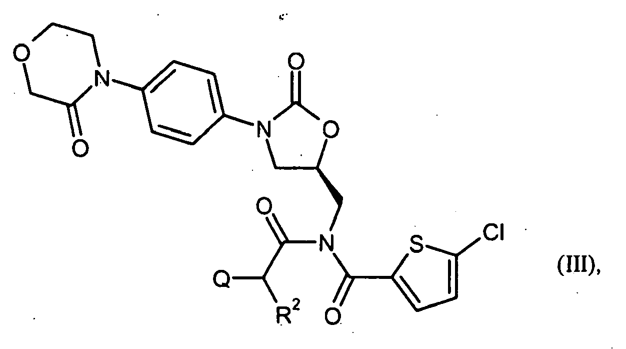 in welcher Q und R² die oben angegebenen Bedeutungen haben,
überführt, diese dann in einem inerten Lösungsmittel mit dem Caesiumsalz einer α-Aminocarbonsäure oder α-Aminothiocarbonsäure der Formel (IV) 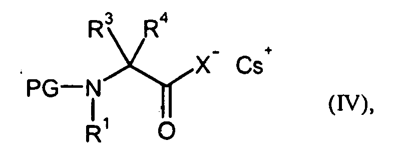 in welcher R', R³ und R⁴ jeweils die oben angegebenen Bedeutungen haben,
PG für eine Amino-Schutzgruppe wie beispielsweise *tert*.-Butoxycarbonyl (Boc) oder Benzyloxycarbonyl (Z) steht
und
X für O oder S steht,
zu einer Verbindung der Formel (V) 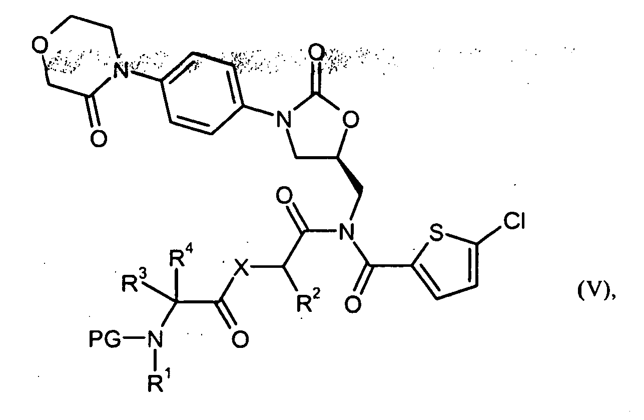 in welcher R¹, R², R³, R⁴, PG und X jeweils die oben angegebenen Bedeutungen haben,
umsetzt und nachfolgend die Schutzgruppe PG nach üblichen Methoden unter Erhalt einer Verbindung der Formel (I-A) 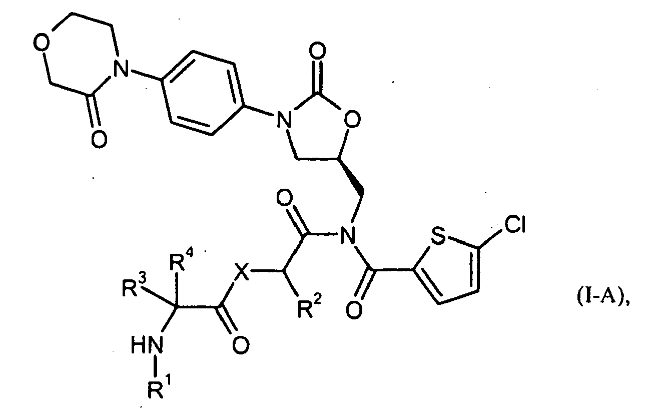 in welcher R¹, R², R³, R⁴ und X jeweils die oben angegebenen Bedeutungen haben, entfernt
oder

### [B] Verbindung (A) in einem inerten Lösungsmittel in Gegenwart einer Base mit einer Verbindung der Formel (VI)

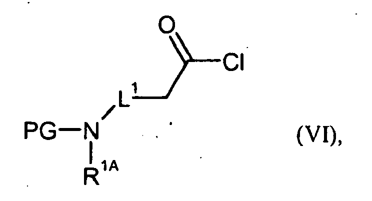

in welcher PG die oben angegebene Bedeutung hat,
R^{1A} für (C₁-C₄)-Alkyl, das mit Hydroxy oder (C₁-C₄)-Alkoxy substituiert sein kann, steht
und
L¹ für eine (C₁-C₄)-Alkandiyl-Gruppe, in welcher eine CH₂-Gruppe gegen ein O-Atom ausgetauscht sein kann, steht,
zu einer Verbindung der Formel (VII) 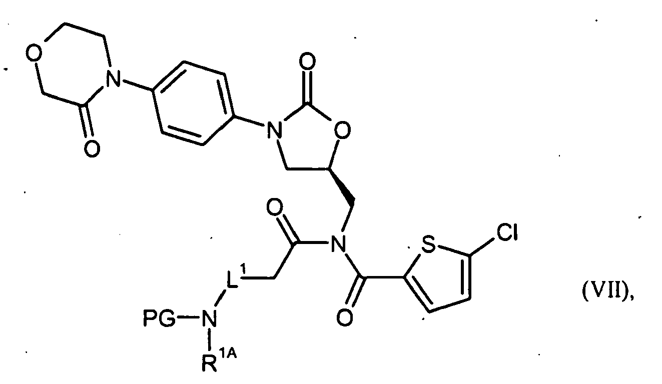 in welcher R^{1A}, L¹ und PG jeweils die oben angegebenen Bedeutungen haben,
umsetzt und anschließend die Schutzgruppe PG nach üblichen Methoden unter Erhalt einer Verbindung der Formel (I-B) 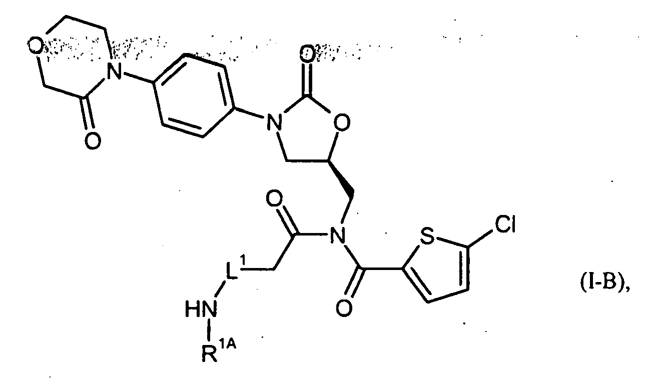 in welcher R^{1A} und L¹ die oben angegebenen Bedeutungen haben, entfernt
oder

### [C] die Verbindung (B)

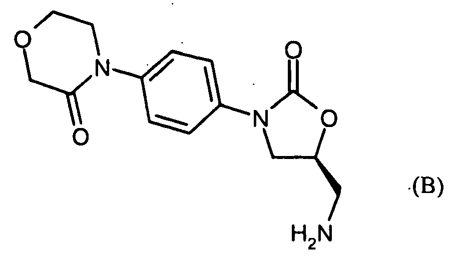

zunächst nach Standardmethoden der Peptidchemie in eine Verbindung der Formel (VIII) 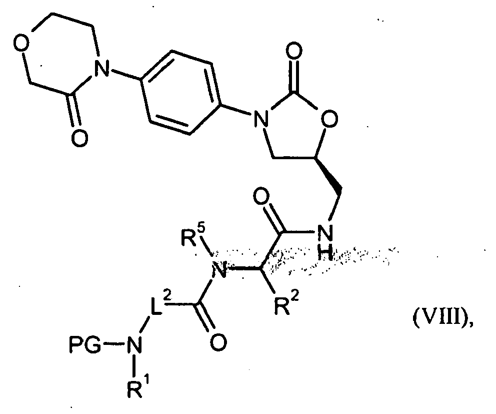 in welcher PG, R¹, R² und R⁵ jeweils die oben angegebenen Bedeutungen haben
und
L² für eine (CH₂)₂- oder CR³R⁴-Gruppe steht, worin R³ und R⁴ jeweils die oben angegebenen Bedeutungen haben,
überführt, diese dann in einem inerten Lösungsmittel in Gegenwart einer Base mit einer Verbindung der Formel (IX) 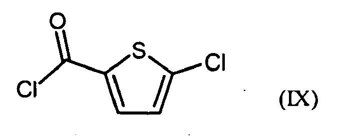 zu einer Verbindung der Formel (X) 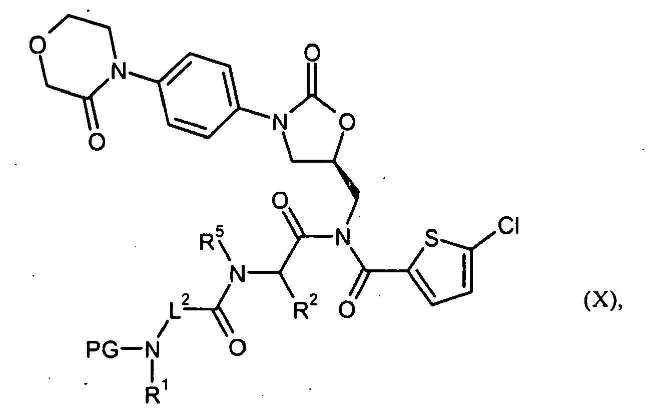 in welcher PG, L², R¹, R² und R⁵ jeweils die oben angegebenen Bedeutungen haben,
umsetzt und nachfolgend die Schutzgruppe PG nach üblichen Methoden unter Erhalt einer Verbindung der Formel (I-C) 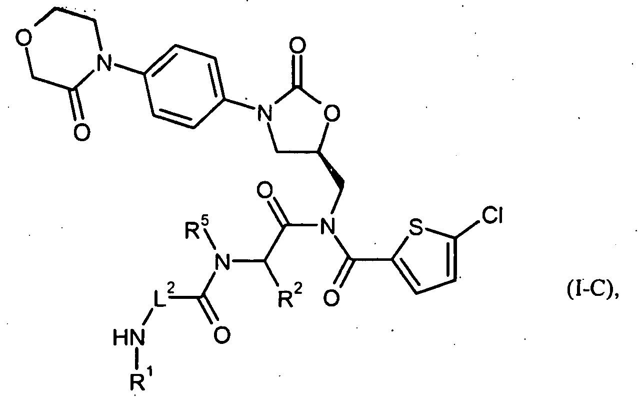 in welcher L², R¹, R² und R⁵ jeweils die oben angegebenen Bedeutungen haben, entfernt
oder

### [D] Verbindung (A) in einem inerten Lösungsmittel in Gegenwart einer Base mit einer Verbindung der Formel (XI)

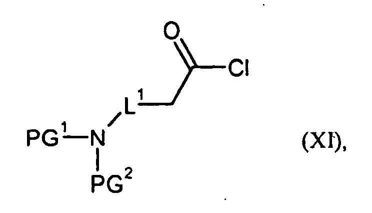

in welcher
L¹ für eine (C₁-C₄)-Alkandiyl-Gruppe, in welcher eine CH₂-Gruppe gegen ein O-Atom ausgetauscht sein kann, steht
und
PG¹ und PG² unabhängig voneinander für eine Amino-Schutzgruppe wie beispielsweise tert-Butoxycarbonyl (Boc), Benzyloxycarbonyl (Z) oder p-Methoxybenzyl (PMB) stehen und gleich oder verschieden sein können,
zu einer Verbindung der Formel (XII) 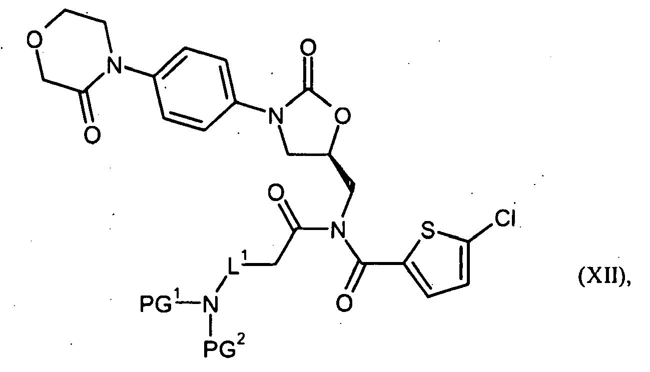 in welcher L¹, PG¹ und PG² jeweils die oben angegebenen Bedeutungen haben,
umsetzt und anschließend die Schutzgruppen PG¹ und PG² nach üblichen Methoden, gleichzeitig oder sequentiell, unter Erhalt einer Verbindung der Formel (I-D) 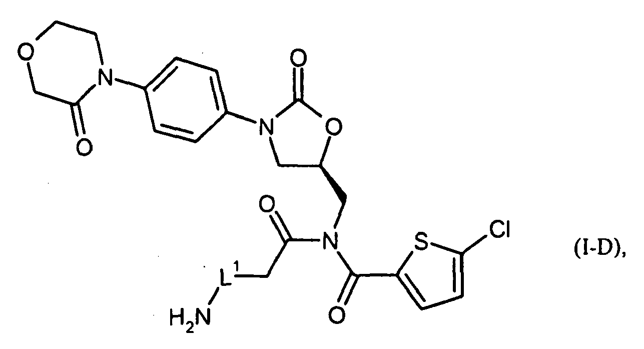 in welcher L¹ die oben angegebene Bedeutung hat, entfernt
und die jeweils resultierenden Verbindungen der Formel (I-A), (I-B), (I-C) bzw. (I-D) gegebenenfalls mit den entsprechenden (i) Lösungsmitteln und/oder (ii) Säuren in ihre Solvate, Salze und/oder Solvate der Salze überführt.

Die Verbindungen der Formeln (I-A), (I-B), (I-C) und (I-D) können bei der Herstellung nach den oben beschriebenen Verfahren auch direkt in Form ihrer Salze entstehen. Diese Salze können gegebenenfalls durch Behandlung mit einer Base in einem inerten Lösungsmittel, über chromatographische Methoden oder mittels Ionenaustauscherharzen in die jeweiligen freien Basen überführt werden.

In den Resten R¹, R^{1A} und/oder R³ gegebenenfalls vorhandene funktionelle Gruppen können bei den zuvor beschriebenen Reaktionssequenzen, falls zweckmäßig oder erforderlich, auch in temporär geschützter Form vorliegen. Die Einführung und Entfernung solcher Schutzgruppen wie auch der Schutzgruppen PG, PG¹ und PG² erfolgt hierbei nach üblichen, aus der Peptidchemie bekannten Methoden [siehe z.B. T.W. Greene und P.G.M. Wuts, Protective Groups in Organic Synthesis, Wiley, New York, 1999; M. Bodanszky und A. Bodanszky, The Practice of Peptide Synthesis, Springer-Verlag, Berlin, 1984].

Solche in R¹, R^{1A} und/oder R³ gegebenenfalls vorhandenen Schutzgruppen können hierbei gleichzeitig mit der Abspaltung von PG oder in einem separaten Reaktionsschritt vor oder nach der Abspaltung von PG entfernt werden.

Als Amino-Schutzgruppe PG, PG¹ bzw. PG² wird bei den obigen Verfahren bevorzugt *tert.-*Butoxycarbonyl (Boc), Benzyloxycarbonyl (Z) oder p-Methoxybenzyl (PMB) verwendet. Die Abspaltung dieser Schutzgruppen wird nach üblichen Methoden, vorzugsweise durch Reaktion mit einer starken Säure wie Chlorwasserstoff, Bromwasserstoff oder Trifluoressigsäure in einem inerten Lösungsmittel wie Dioxan, Dichlormethan oder Essigsäure durchgeführt; gegebenenfalls kann die Abspaltung auch ohne ein zusätzliches inertes Lösungsmittel durchgeführt werden.

Die Transformation (B) → (VIII) erfolgt nach Standardmethoden der Peptidchemie entweder durch Acylierung der Verbindung (B) mit einem geeignet geschützten Dipeptid-Derivat oder durch sequentielle Kupplung der einzelnen, gegebenenfalls geeignet geschützten Aminosäure-Komponenten [vgl. z.B. M. Bodanszky, Principles of Peptide Synthesis, Springer-Verlag, Berlin, 1993; H.-D. Jakubke und H. Jeschkeit, Aminosäuren, Peptide, Proteine, Verlag Chemie, Weinheim, 1982].

Als inerte Lösungsmittel werden in den Verfahrensschritten (A) + (II) → (III), (A) + (VI) → (VII), (VIII) + (IX) → (X) und (A) + (XI) → (XII) vorzugsweise Tetrahydrofuran, *N*,*N-*Dimethylformamid oder Dimethylsulfoxid verwendet; besonders bevorzugt ist *N,N-*Dimethylformamid. Als Base ist bei diesen Reaktionen insbesondere Natriumhydrid geeignet. Die genannten Umsetzungen werden im Allgemeinen in einem Temperaturbereich von 0°C bis +40°C bei Normaldruck durchgeführt.

Der Verfahrensschritt (III) + (IV) → (V) erfolgt bevorzugt in *N,N-*Dimethylformamid als Lösungsmittel. Im Allgemeinen wird die Reaktion in einem Temperaturbereich von 0°C bis +50°C, bevorzugt bei +20°C bis +50°C, bei Normaldruck durchgeführt. Die Umsetzung kann auch vorteilhaft unter Ultraschall-Behandlung ausgeführt werden.

Die Verbindungen der Formeln (II), (IV), (VI), (IX) und (XI) sind kommerziell erhältlich, literaturbekannt oder können nach literaturüblichen Verfahren hergestellt werden. Die Herstellung der Verbindungen (A) und (B) ist in S. Roehrig et al., J. Med. Chem. 48, 5900 (2005) beschrieben.

Die Herstellung der erfindungsgemäßen Verbindungen kann durch die folgenden Syntheseschemata veranschaulicht werden:

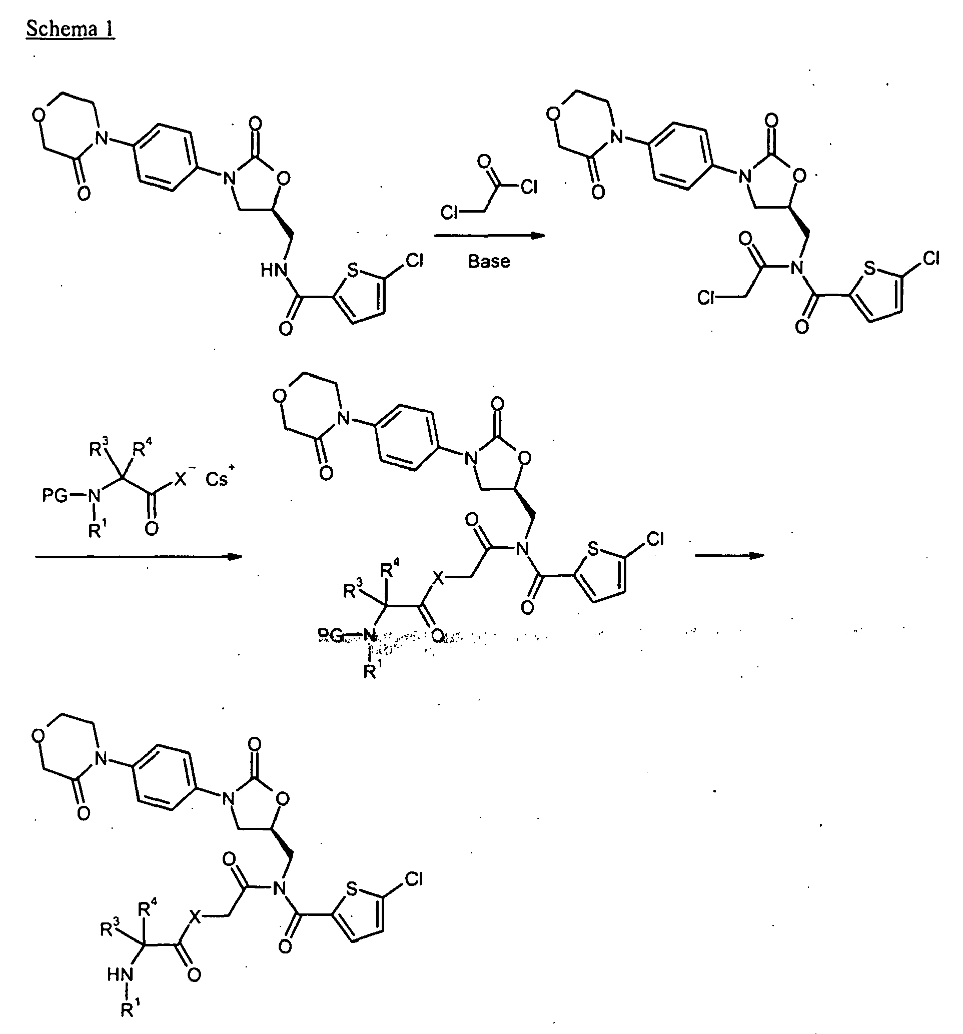

### [X = O oder S; PG = Amino-Schutzgruppe, z.B. tert.-Butoxycarbonyl (Boc) oder Benzyloxycarbonyl (Z)].

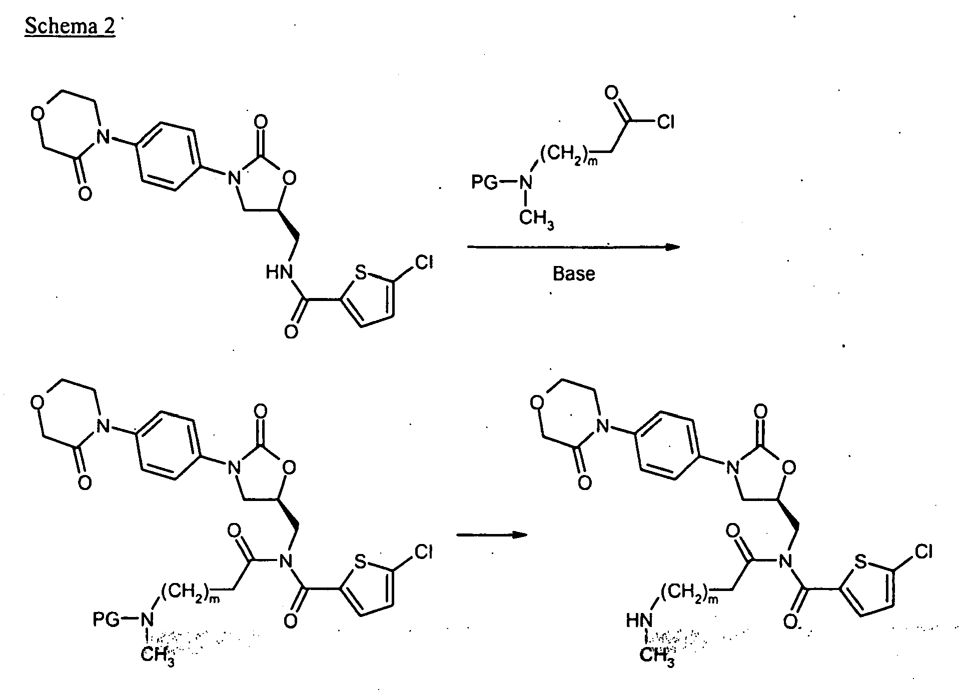

### [m = 1-4; PG = Amino-Schutzgruppe, z.B. tert.-Butoxycarbonyl (Boc) oder Benzyloxycarbonyl (Z)].

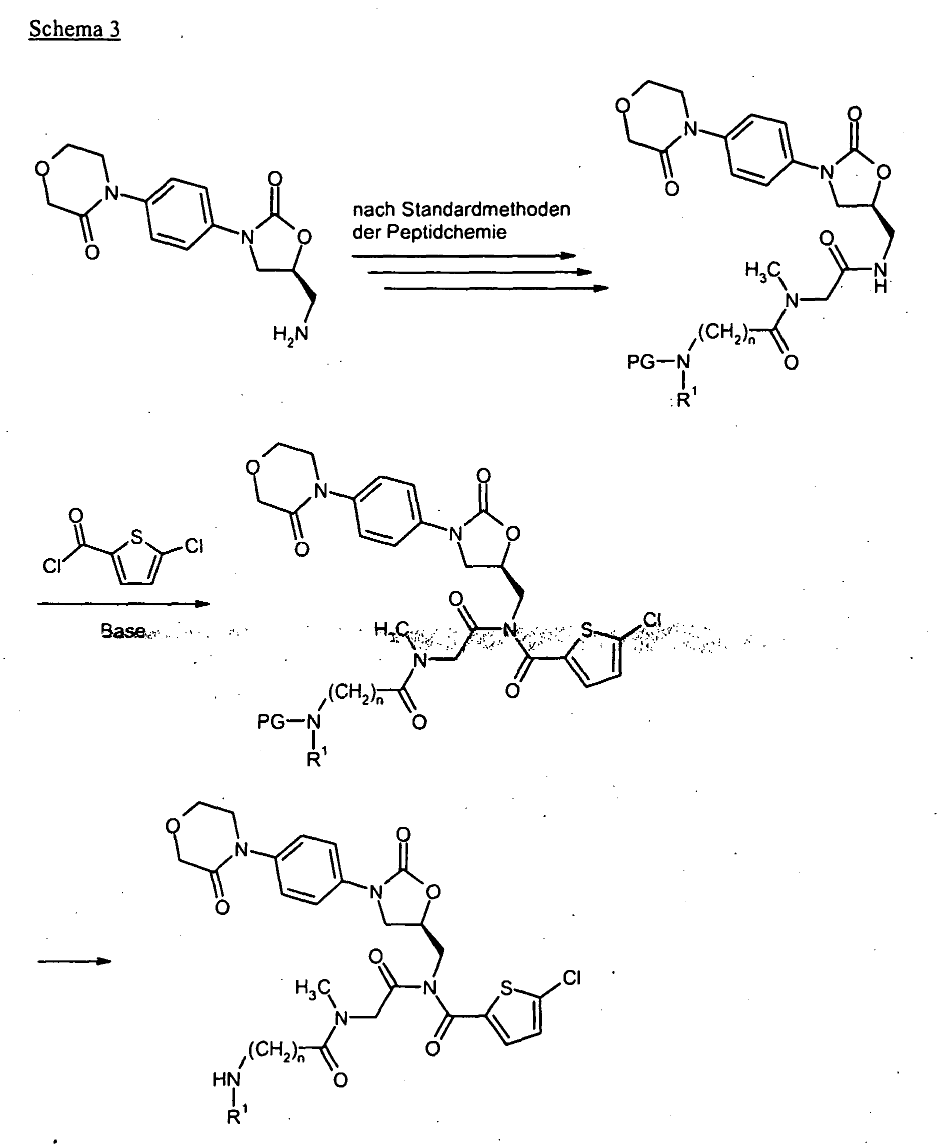

### [n = 1 oder 2; PG = Amino-Schutzgruppe, z.B. tert.-Butoxycarbonyl (Boc) oder Benzyloxycarbonyl (Z)].

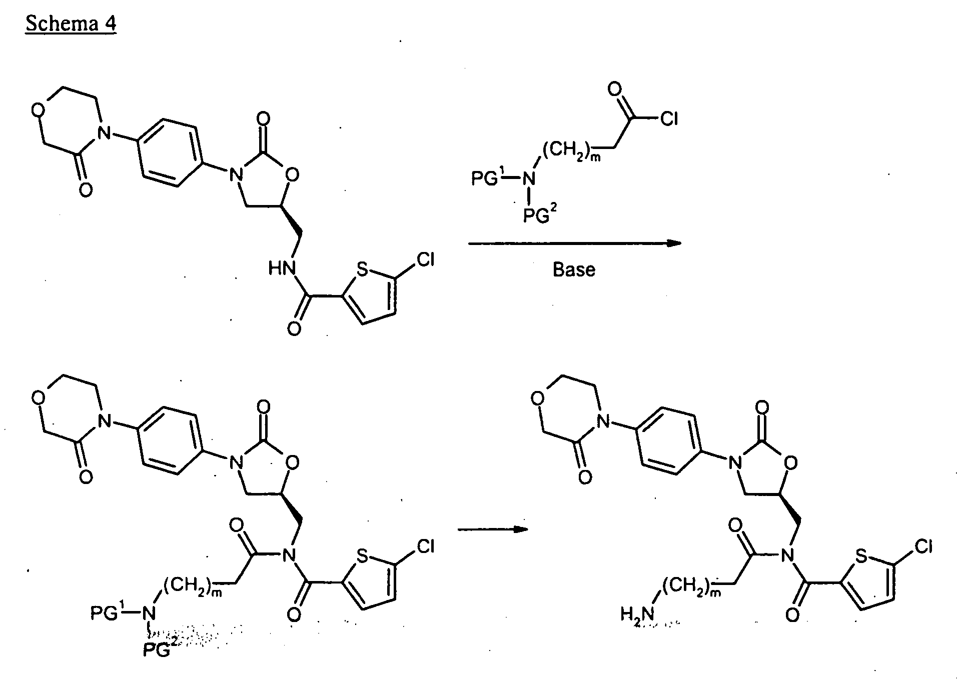

### [m = 1-4; Pol, PG² = Amino-Schutzgruppen, z.B. tert.-Butoxycarbonyl (Boc), Benzyloxycarbonyl (Z) oder p-Methoxybenzyl (PMB)].

Die erfindungsgemäßen Verbindungen und ihre Salze stellen nützliche Prodrugs der Wirkstoff-Verbindung (A) dar. Sie weisen einerseits eine gute Stabilität bei pH 4 auf und zeigen andererseits eine effiziente Konversion zur Wirkstoff-Verbindung (A) bei einem physiologischen pH-Wert und *in vivo.* Darüber hinaus besitzen die erfindungsgemäßen Verbindungen eine gute Löslichkeit in Wasser und anderen physiologisch verträglichen Medien, was sie für die therapeutische Anwendung insbesondere bei intravenöser Applikation geeignet macht.

Weiterer Gegenstand der vorliegenden Erfindung ist der Einsatz der erfindungsgemäßen Verbindungen zur Behandlung und/oder Prophylaxe von Erkrankungen, vorzugsweise von thromboembolischen Erkrankungen und/oder thromboembolischen Komplikationen.

Zu den "thromboembolischen Erkrankungen" im Sinne der vorliegenden Erfindung zählen insbesondere Erkrankungen wie Herzinfarkt mit ST-Segment-Erhöhung (STEMI) und ohne ST-Segment-Erhöhung (non-STEMI), stabile Angina Pectoris, instabile Angina Pectoris, Reokklusionen und Restenosen nach Koronarinterventionen wie Angioplastie oder aortokoronarem Bypass, periphere arterielle Verschlusskrankheiten, Lungenembolien, tiefe venöse Thrombosen und Nierenvenenthrombosen, transitorische ischämische Attacken sowie thrombotischer und thromboembolischer Hirnschlag.

Die Substanzen eignen sich daher auch zur Prävention und Behandlung von kardiogenen Thromboembolien, wie beispielsweise Hirn-Ischämien, Schlaganfall und systemischen Thromboembolien und Ischämien, bei Patienten mit akuten, intermittierenden oder persistierenden Herzarrhythmien, wie beispielsweise Vorhofflimmern, und solchen, die sich einer Kardioversion unterziehen, ferner bei Patienten mit Herzklappen-Erkrankungen oder mit künstlichen Herzklappen. Darüber hinaus sind die erfindungsgemäßen Verbindungen zur Behandlung der disseminierten intravasalen Gerinnung (DIC) geeignet.

Thromboembolische Komplikationen treten ferner auf bei mikroangiopathischen hämolytischen Anämien, extrakorporalen Blutkreisläufen, wie Hämodialyse, sowie Herzklappenprothesen.

Außerdem kommen die erfindungsgemäßen Verbindungen auch für die Prophylaxe und/oder Behandlung von atherosklerotischen Gefäßerkrankungen und entzündlichen Erkrankungen wie rheumatische Erkrankungen des Bewegungsapparats in Betracht, darüber hinaus ebenso für die Prophylaxe und/oder Behandlung der Alzheimer'schen Erkrankung. Außerdem können die erfindungsgemäßen Verbindungen zur Inhibition des Tumorwachstums und der Metastasenbildung, bei Mikroangiopathien, altersbedingter Makula-Degeneration, diabetischer Retinopathie, diabetischer Nephropathie und anderen mikrovaskulären Erkrankungen sowie zur Prävention und Behandlung thromboembolischer Komplikationen, wie beispielsweise venöser Thromboembolien, bei Tumorpatienten, insbesondere solchen, die sich größeren chirurgischen Eingriffen oder einer Chemo- oder Radiotherapie unterziehen, eingesetzt werden.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden .Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung sind Arzneimittel, enthaltend eine erfindungsgemäße Verbindung und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
- Lipidsenker, insbesondere HMG-CoA-(3-Hydroxy-3-methylglutaryl-Coenzym A)-Reduktase-Inhibitoren;
- Koronartherapeutika/Vasodilatatoren, insbesondere ACE-(Angiotensin-Converting-Enzyme)-Inhibitoren; AII-(Angiotensin II)-Rezeptor-Antagonisten; β-Adrenozeptor-Antagonisten; alpha-1-Adrenozeptor-Antagonisten; Diuretika; Calciumkanal-Blocker; Substanzen, die eine Erhöhung von cyclischem Guanosinmonophosphat (cGMP) bewirken, wie beispielsweise Stimulatoren der löslichen Guanylatcyclase;
- Plasminogen-Aktivatoren (Thrombolytika/Fibrinolytika) und die Thrombolyse/Fibrinolyse steigernde Verbindungen wie Inhibitoren des Plasminogen-Aktivator-Inhibitors (PAI-Inhibitoren) oder Inhibitoren des Thrombin-aktivierten Fibrinolyse-Inhibitors (TAFI-Inhibitoren);
- antikoagulatorisch wirksame Substanzen (Antikoagulantien),
- plättchenaggregationshemmende Substanzen (Plättchenaggregationshemmer, Thrombozytenaggregationshemmer);
- Fibrinogen-Rezeptor-Antagonisten (Glycoprotein-IIb/IIIa-Antagonisten);
- sowie Antiarrhythmika.

Weiterer Gegenstand der vorliegenden Erfindung sind Arzneimittel, die mindestens eine erfindungsgemäße Verbindung, üblicherweise zusammen mit einem oder mehreren inerten, nicht-toxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal oder nasal. Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. inhalative Arzneiformen wie Pulverinhalatoren oder Nebulizer, oder nasal applizierbare Arzneiformen wie Tropfen, Lösungen oder Sprays.

Bevorzugt ist die parenterale Applikation, insbesondere die intravenöse Applikation.

Die erfindungsgemäß Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- abs.: absolut
- Boc: *tert*.-Butoxycarbonyl
- DC: Dünnschichtchromatographie
- DMF: *N,N-*Dimethylformamid
- DMSO: Dimethylsulfoxid
- d. Th.: der Theorie (bei Ausbeute)
- h: Stunde(n)
- HPLC: Hochdruck-, Hochleistungsflüssigchromatographie
- LC-MS: Flüssigchromatographie-gekoppelte Massenspektrometrie
- min: Minute(n)
- MS: Massenspektrometrie
- NMR: Kernresonanzspektrometrie
- p: para
- Pd/C: Palladium auf Aktivkohle
- PMB: p-Methoxybenzyl
- quant.: quantitativ (bei Ausbeute)
- R_{f}: Retentionsindex (bei DC)
- RT: Raumtemperatur
- R,: Retentionszeit (bei HPLC)
- UV: Ultraviolett-Spektrometrie
- v/v: Volumen-zu-Volumen-Verhältnis (einer Lösung)
- Z: Benzyloxycarbonyl

### LC-MS- und HPLC-Methoden:

### Methode 1 (präparative HPLC):

Säule: VP 250/21 Nukleodur 100-5 C18 ec, Macherey & Nagel Nr. 762002; Eluent A: Wasser / 0.01% Trifluoressigsäure, Eluent B: Acetonitril / 0.01% Trifluoressigsäure; Gradient: 0 min 0% B → 20 min 20% B → 40 min 20% B → 60 min 30% B → 80 min 30% B → 90 min 100% B → 132 min 100% B; Fluss: 5 ml/min; Temperatur: RT; UV-Detektion: 210 nm.

### Methode 2 (analytische HPLC):

Säule: XTerra 3.9 x 150 WAT 186000478; Eluent A: 10 ml 70%-ige Perchlorsäure in 2.5 Liter Wasser, Eluent B: Acetonitril; Gradient: 0.0 min 20% B → 1 min 20% B → 4 min 90% B → 9 min 90% B; Temperatur: RT; Fluss: 1 ml/min.

### Methode 3 (LC-MS):

Instrument: Micromass LCT mit HPLC Agilent Serie 1100; Säule: Waters Symmetry C18, 3.5 µm, 50 mm x 2.1 mm; Eluent A: 1 l Wasser + 1 ml 98-100%-ige Ameisensäure, Eluent B: 1 1 Acetonitril + 1 ml 98-100%-ige Ameisensäure; Gradient: 0 min 100% A → 1 min 100% A → 6 min 10% A → 8 min 0% A → 10 min 0% A → 10.1 min 100% A → 12 min 100% A; Fluss: 0-10 min 0.5 ml/min → 10.1 min 1 ml/min → 12 min 0.5 ml/min; Temperatur: 40°C; UV-Detektion DAD: 208-500 nm.

### Methode 4 (LC-MSI:

Instrument, Micromass ZQ mit HPLC HP 1100 Series; UV DAD; Säule: Phenomenex Synergi 2µ Hydro-RP Mercury 20 mm x 4 mm; Eluent A: 1 l Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Temperatur: 50°C; UV-Detektion: 210 nm.

### Methode 5 (LC-MS):

Instrument: Micromass Quattro LCZ mit HPLC Agilent Series 1100; Säule: Phenomenex Synergi 2µ Hydro-RP Mercury 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Temperatur: 50°C; UV-Detektion: 208-400 nm.

### Methode 6 i(LC-MS):

Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Temperatur: 50°C; UV-Detektion: 210 nm.

### Methode 7 (chirale HPLC, analytisch):

Chirale Silicagel-Phase (250 mm x 4.6 mm) basierend auf Poly(*N-*methacryloyl-L-leucin-dicyclo-propylmethylamid); Eluent: Isohexan/Essigsäureethylester 35:65 (v/v); Temperatur: 24°C; Fluss: 2 ml/min; UV-Detektion: 270 nm.

### Methode 8 (chirale HPLC, analytisch):

Chirale Silicagel-Phase (250 mm x 4.6 mm) basierend auf Poly(*N-*methacryloyl-L-leucin-*tert.-*butylamid); Eluent: Isohexan/Essigsäureethylester 35:65 (v/v); Temperatur: 24°C; Fluss: 2 ml/min; UV-Detektion: 270 nm.

### Methode 9 (chirale HPLC, analytisch):

Chirale Silicagel-Phase (250 mm x 4.6 mm) basierend auf Poly(*N-*methacryloyl-L-leucin-*tert.-*butylamid); Eluent: Isohexan/Essigsäureethylester 65:35 (v/v); Temperatur: 24°C; Fluss: 2 ml/min; UV-Detektion: 270 nm.

### Methode 10 (chirale HPLC, präparativ):

Chirale Silicagel-Phase (670 mm x 40 mm) basierend auf Poly(*N-*methacryloyl-L-leucin-dicyclo-propylmethylamid); Eluent: Isohexan/Essigsäureethylester 25:75 (v/v); Temperatur: 24°C; Fluss: 80 ml/min; UV-Detektion: 270 nm.

### Methode 11 (chirale HPLC, präparativ):

Chirale Silicagel-Phase (670 mm x 40 mm) basierend auf Poly(*N-*methacryloyl-L-leucin-*tert.-*butylamid); Eluent: Isohexan/Essigsäureethylester 65:35 (v/v); Temperatur: 24°C; Fluss: 50 ml/min; UV-Detektion: 260 nm.

### Methode 12 (LC-MS):

Instrument: Micromass Quattro LCZ mit HPLC Agilent Serie 1100; Säule: Phenomenex Onyx Monolithic C18, 100 mm x 3 mm; Eluent A: 1 l Wasser+ 0.5 ml 50%-ige Ameisensäure, Eluent B: 1 l Acetonitril + 0.5 ml 50%-ige Ameisensäure; Gradient: 0.0 min 90% A → 2 min 65% A → 4.5 min 5% A → 6 min 5% A; Fluss: 2 ml/min; Ofen: 40°C; UV-Detektion: 208-400 nm.

### Methode 13 (LC-MS):

Instrument: Micromass Platform LCZ mit HPLC Agilent Serie 1100; Säule: Thermo Hypersil GOLD 3µ, 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 100% A → 0.2 min 100% A → 2.9 min 30% A → 3.1 min 10% A → 5.5 min 10% A; Ofen: 50°C; Fluss: 0.8 ml/min; UV-Detektion: 210 nm.

### NMR-Spektrometrie:

NMR-Messungen wurden bei einer Protonenfrequenz von 400.13 MHz durchgerührt. Die Proben wurden üblicherweise in DMSO-d₆ gelöst; Temperatur: 302 K.

### Ausgangsverbindungen und Intermediate:

Als Ausgangsmaterialien werden 5-Chlor-*N-*({(5*S*)-2-oxo-3-[4-(3-oxomorpholin-4-yl)phenyl]-1,3-oxazolidin-5-yl}methyl)thiophen-2-carboxamid [Verbindung (A)] und 4-{4-[(5*S*)-5-(Aminomethyl)-2-oxo-1,3-oxazolidin-3-yl]phenyl}morpholin-3-on [Verbindung (B)] verwendet, deren Herstellung an anderer Stelle beschrieben ist [S. Roehrig et al., J. Med. Chem. 48, 5900 (2005)].

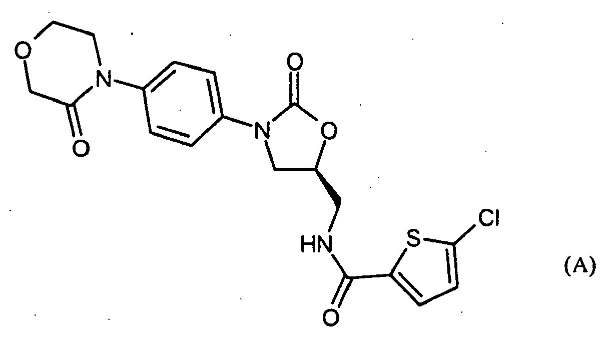

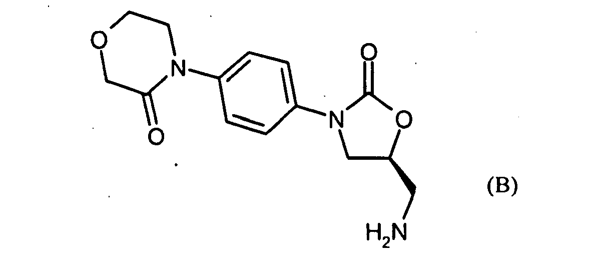

### Allgemeine Vorschrift 1 zur Acylierung der Aminogruope in Verbindung (B):

Die eingesetzten Carboxylkomponenten (in den meisten Fällen geeignet geschützte Aminosäure- oder Peptid-Derivate) sind entweder kommerziell erhältlich oder werden nach Standardmethoden hergestellt. Bevorzugt wird 4-{4-[(5*S*)-5-(Aminomethyl)-2-oxo-1,3-oxazolidin-3-yl]phenyl}morpholin-3-on [Verbindung (B)] direkt mit geeignet geschützten Peptid-Derivaten acyliert. Alternativ kann in einer sequentiellen Verfahrensweise auch zuerst mit einem Aminosäure-Derivat verknüpft, anschließend gegebenenfalls entschützt und dann mit weiteren geeignet geschützten Aminosäure- oder Peptid-Derivaten nach Standardmethoden umgesetzt werden.

2.3 mmol der entsprechenden Carboxylkomponente werden in 30 ml DMF gelöst und mit 2.3 mmol 1-Hydroxy-1*H*-benzotriazol, 2 mmol *N-*(3-Dimethylaminopropyl)-*N'*-ethylcarbodiimid-Hydrochlorid (EDC), 4.5 mmol *NN-*Diisopropylethylamin sowie anschließend mit 1.5 mmol der Amin-Komponente, 4-{4-[(5*S*)-5-(Aminomethyl)-2-oxo-1,3-oxazolidin-3-yl]phenyl}morpholin-3-on [Verbindung (B)], versetzt. Nach 3 h Rühren bei Raumtemperatur wird das Reaktionsgemisch eingeengt, der Rückstand in Dichlormethan aufgenommen und zweimal mit 5%-iger Zitronensäure, zweimal mit 5%-iger Natriumhydrogencarbonat-Lösung und zweimal mit Wasser ausgeschüttelt. Die organische Phase wird eingeengt und der Rückstand durch Flash-Chromatographie an Kieselgel mit Acetonitril/Wasser 30:1 als Eluent gereinigt. Die entsprechenden Fraktionen werden vereinigt und das Lösungsmittel entfernt. Der verbleibende Rückstand wird in Dichlormethan/Methanol gelöst und das Produkt mit Diethylether gefällt und im Hochvakuum getrocknet

### Intermediat 1A

### 5-Chlor-N-(chloracetyl)-N-({(5S)-2-oxo-3-[4-(3-oxomorpholin-4-yl)phenyl]-1,3-oxazolidin-5-yl}-methyl)thiophen-2-carboxamid

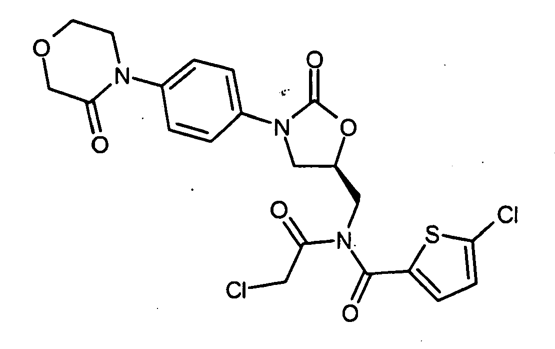

1 g (2.3 mmol) 5-Chlor-*N-*({(5*S*)-2-oxo-3-[4-(3-oxomorpholin-4-yl)phenyl]-1,3-oxazolidin-5-yl}-methyl)thiophen-2-carboxamid [Verbindung (A)] wird unter Argon in 170 ml abs. DMF gelöst. Man setzt 110 mg (4.6 mmol) Natriumhydrid zu und lässt 20 min bei RT rühren. Dann werden 3.5 g (31 mmol) Chloracetylchlorid zugesetzt, wobei die Reaktionstemperatur auf RT gehalten wird. Nach 30 min setzt man unter Kühlung 25 ml Wasser zu und lässt das Gemisch zwei Tage bei RT stehen. Dann wird das Lösungsmittel im Vakuum entfernt, wobei die Temperatur nicht über +25°C ansteigen sollte. Der Rückstand wird in 500 ml Dichlormethan aufgenommen und fünfmal mit 200 ml Wasser ausgeschüttelt. Die organische Phase wird über Magnesiumsulfat getrocknet, filtriert und auf ein Volumen von ca. 50 ml eingeengt. Unter Rühren werden 200 ml Diethylether zugesetzt und der Niederschlag (zum großen Teil nicht umgesetztes Ausgangsmaterial) anschließend abfiltriert. Die Mutterlauge wird eingeengt und der Rückstand durch Flash-Chromatographie an Kieselgel mit Toluol/Ethanol 10:1 1 als Eluent gereinigt. Die entsprechenden Fraktionen werden vereinigt und das Lösungsmittel entfernt. Der Rückstand wird aus Dioxan lyophilisiert.

### Ausbeute: 111 mg (9.5% d. Th.)

HPLC (Methode 2): Rₜ = 5.09 min;
LC-MS (Methode 4): Rₜ = 2.31 min; m/z = 512 (M+H)⁺.

### Intermediat 2A

### N-[(Benzyloxy)carbonyl]-N-methylglycyl-N²-methyl-N-({(5S)-2-oxo-3-[4-(3-oxomorpholin-4-yl)-phenyl]-1,3-oxazolidin-5-yl}methyl)-glycinamid.

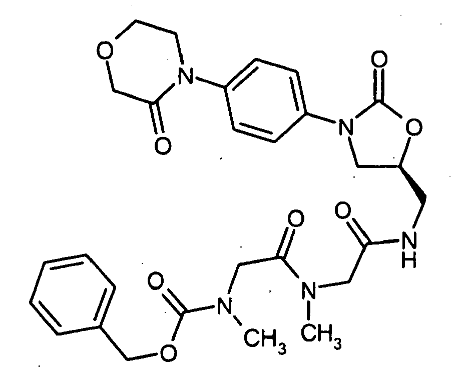

Im ersten Schritt werden 511 mg (1.5 mmol) Z-Sarcosin als Carboxylkomponente nach der Allgemeinen Vorschrift 1 mit Verbindung (B) umgesetzt (Ausbeute: 697 mg, 92% d. Th.).

Von 200 mg (0.4 mmol) dieser Zwischenstufe wird anschließend nach Standardmethoden die Benzyloxycarbonyl-Schutzgruppe hydrogenolytisch über Pd/C entfernt (Ausbeute: 130 mg, 89% d. Th.).

Die so erhaltene Verbindung wird dann in einem dritten Schritt erneut mit 120 mg (0.54 mmol) Z-Sarcosin nach der Allgemeinen Vorschrift 1 verknüpft (Ausbeute: 201 mg, 98% d. Th.).

HPLC (Methode 2): Rₜ = 4.21 min;

LC-MS (Methode 6): Rₜ = 1.54 min; m/z = 568 (M+H)⁺.

### Intermediat 3A

### N-[(Benzyloxy)carbonyl]-N-methylglycyl-N²-methyl-N-({(5S)-2-oxo-3-[4-(3-oxomorpholin-4-yl)-phenyl]-1,3-oxazolidin-5-yl}methyl)-L-valinamid

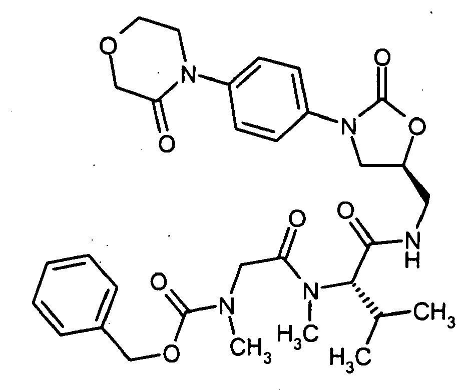

Im ersten Schritt werden 529 mg (2.3 mmol) Boc-*N-*Methylvalin als Carboxylkomponente nach der Allgemeinen Vorschrift 1 mit Verbindung (B) umgesetzt (Ausbeute: 750 mg, 97% d. Th.).

Von 750 mg (1.5 mmol) dieser Zwischenstufe wird anschließend- nach Standardmethode mit Trifluoressigsäure in Dichlormethan die tert.-Butoxycarbonyl-Schutzgruppe entfernt (Ausbeute: 740 mg, 96% d. Th.).

200 mg (0.39 mmol) der so erhaltenen Verbindung werden dann in einem dritten Schritt mit 129 mg (0.58 mmol) Z-Sarcosin nach der Allgemeinen Vorschrift 1 verknüpft (Ausbeute: 206 mg, 88% d. Th.).
HPLC (Methode 2): Rₜ = 4.68 min;
LC-MS (Methode 5): Rₜ = 1.96 min; m/z = 610 (M+H)⁺.

### Intermediat 4A

### Benzyl methyl-{5-oxo-5-[({(5S)-2-oxo-3-[4-(3-oxomorpholin-4-yl)phenyl]-1,3-oxazolidin-5-yl}-methyl)amino]pentyl}-carbamat

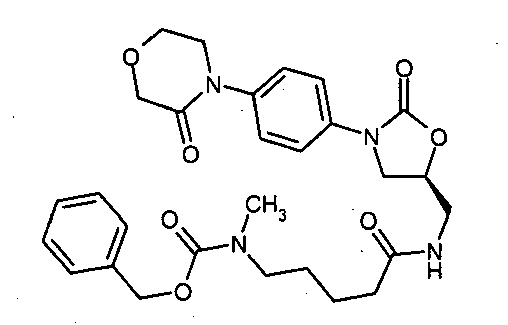

32 mg (0.119 mmol) 5-[[(Benzyloxy)carbonyl](methyl)amino]valeriansäure werden als Carboxylkomponente nach der Allgemeinen Vorschrift 1 mit Verbindung (B) umgesetzt.
Ausbeute: 45 mg (91% d. Th.)
HPLC (Methode 2): Rₜ = 4.51 min;
LC-MS (Methode 4): Rₜ = 2.02 min; m/z = 539 (M+H)⁺.

### Intermediat 5A

### Benzyl methyl-{5-oxo-5-[({5S)-2-oxo-3-[4-(3-oxomorpholin-4-yl)phenyl]-1,3-oxazolidin-5-yl)-methyl)amino]butyl}-carbamat

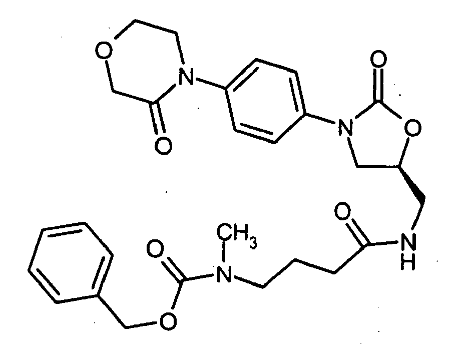

313 mg (0.96 mmol) 5-[[(Benzyloxy)carbonyl](methyl)amino]buttersäure werden als Carboxylkomponente nach der Allgemeinen Vorschrift 1 mit Verbindung (B) umgesetzt.
Ausbeute: 298 mg (71% d. Th.)
HPLC (Methode 2): Rₜ = 4.42 min;
LC-MS (Methode 4): Rₜ = 1.89 min; m/z = 525 (M+H)⁺.

### Intermediat 6A

### N-[(Benzyloxy)carbonyl]-N-methyl-β-alanyl-N²-methyl-N-({(5S)-2-oxo-3-[4-(3-oxomorpholin-4-yl)phenyl]-1,3-oxazolidin-5-yl}methyl)-glycinamid

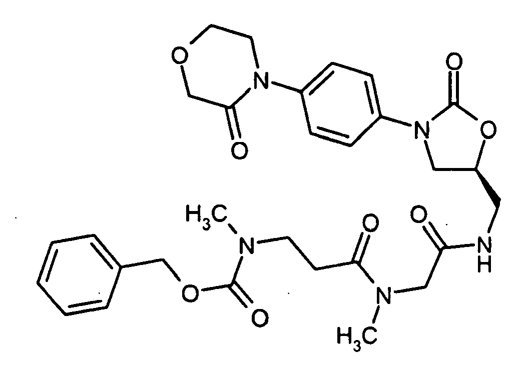

Im ersten Schritt werden 511 mg (1.5 mmol) Z-Sarcosin als Carboxylkomponente nach der Allgemeinen Vorschrift 1 mit Verbindung (B) umgesetzt (Ausbeute 697 mg 92% d. Th.).

Von 200 mg (0.4 mmol) dieser Zwischenstufe wird anschließend nach Standardmethoden die Benzyloxycarbonyl-Schutzgruppe hydrogenolytisch über Pd/C entfernt (Ausbeute: 130 mg, 89% d. Th.).

100 mg der so erhaltenen Verbindung werden dann in einem dritten Schritt mit 98.2 mg (0.41 mmol) Z-*N-*Methyl-β-alanin nach der Allgemeinen Vorschrift 1 verknüpft (Ausbeute: 87 mg, 54% d. Th.).
HPLC (Methode 2): Rₜ = 4.28 min;
LC-MS (Methode 6): Rₜ = 1.60 min; m/z = 582 (M+H)⁺.

### Intermediat 7A

### Benzyl (4-chlor-4-oxobutyl)methyl-carbamat

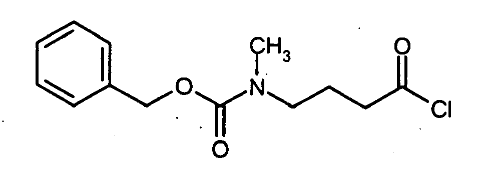

Zunächst wird 4-[[(Benzyloxy)carbonyl](methyl)amino]buttersäure nach Literaturvorschrift [Y. Aramaki et al., Chem. Pharm. Bull. 52, 258 (2004)] aus kommerziell erhältlicher 4-{[(Benzyl-oxy)carbonyl]amino}buttersäure hergestellt. Alternativ kann die Herstellung auch über die Einführung der Benzyloxycarbonyl-Schutzgruppe in ω-*N*-Methylaminoalkylcarbonsäuren, die nach P. Quitt et al. [Helv. Chim. Acta 46, 327 (1963)] erhältlich sind, erfolgen.

1.74 g (6.92 mmol) 4-[[(Benzyloxy)carbonyl](methyl)amino]buttersäure werden in 35 ml Dichlormethan gelöst und mit 3.5 ml (48 mmol) Thionylchlorid versetzt. Man erhitzt die Mischung 1 h lang unter Rückfluss. Danach wird im Vakuum eingeengt, der Rückstand erneut mit Dichlormethan versetzt und abermals eingeengt. Zurück bleibt ein viskoses Öl, das im Hochvakuum getrocknet wird. Man erhält 1.8 g (96% d. Th.) der Zielverbindung, welche ohne weitere Aufreinigung und Charakterisierung weiter umgesetzt wird.

### Intermediat 8A

### Benzyl (5-chlor-5-oxopentyl)methyl-carbamat

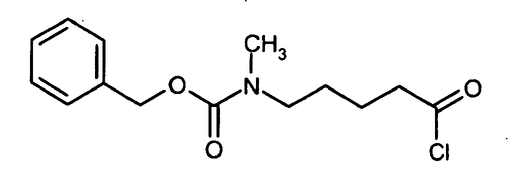

Zunächst wird 5-[[(Benzyloxy)carbonyl](methyl)amino]valeriansäure nach bekannten Methoden analog zu Intermediat 7A hergestellt.

1.97 g (7.43 mmol) 5-[[(Benzyloxy)carbonyl](methyl)amino]valeriansäure werden in 30 ml Dichlormethan gelöst und mit 4.9 ml (67.3 mmol) Thionylchlorid versetzt. Man erhitzt die Mischung 1 h lang unter Rückfluss. Danach wird im Vakuum eingeengt, der Rückstand erneut mit Dichlormethan versetzt und abermals eingeengt. Zurück bleibt ein viskoses Öl, das im Hochvakuum getrocknet wird. Man erhält 2 g (95% d. Th.) der Zielverbindung, welche ohne weitere Aufreinigung und Charakterisierung weiter umgesetzt wird.

### Intermediat 9A

### Benzyl (3-chlor-3-oxopropyl)methyl-carbamat

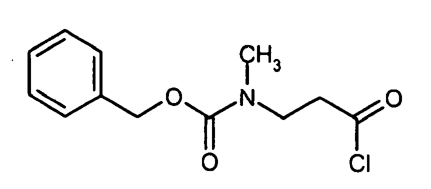

Zunächst wird 3-[[(Benzyloxy)carbonyl](methyl)amino]propionsäure nach Literaturvorschrift [Y. Aramaki et al., Chem. Pharm. Bull. 52, 258 (2004)] aus kommerziell erhältlicher 3-{[(Benzyl-oxy)carbonyl]amino}propionsäure hergestellt. Alternativ kann die Herstellung auch über die Einführung der Benzyloxycarbonyl-Schutzgruppe in ω-*N*-Methylaminoalkylcarbonsäuren, die nach P. Quitt et al. [Helv. Chim. Acta 46, 327 (1963)] erhältlich sind, erfolgen.

850 mg (3.58 mmol) 3-[[(Benzyloxy)carbonyl](methyl)amino]propionsäure werden in 15 ml Dichlormethan gelöst und mit 1.5 ml Oxalylchlorid versetzt. Man erhitzt die Mischung 3 h lang unter Rückfluss. Danach wird im Vakuum eingeengt, der Rückstand erneut mit Dichlormethan versetzt und abermals eingeengt. Zurück bleibt ein viskoses Öl, das im Hochvakuum getrocknet wird. Man erhält 915 mg (quant.) der Zielverbindung, welche ohne weitere Aufreinigung und Charakterisierung weiter umgesetzt wird.

### Intermediat 10A

### Benzyl (6-chlor-6-oxohexyl)methyl-carbamat

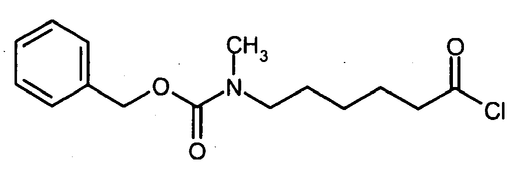

Zunächst wird 6-[[(Benzyloxy)carbonyl](methyl)amino]capronsäure nach Literaturvorschrift [Y. Aramaki et al., Chem. Pharm. Bull. 52, 258 (2004)] aus kommerziell erhältlicher 6-{[(Benzyl-oxy)carbonyl]amino}capronsäure hergestellt. Alternativ kann die Herstellung auch über die Einführung der Benzyloxycarbonyl-Schutzgruppe in ω-*N-*Methylaminoalkylcarbonsäuren, die nach P. Quitt et al. [Helv. Chim. Acca 46, 327 (1963)] erhältlich sind, erfolgen.

3850 mg (13.8 mmol) 6-[[(Benzyloxy)carbonyl](methyl)amino]capronsäure werden in 60 ml Dichlormethan gelöst und mit 4 ml Oxalylchlorid versetzt. Man erhitzt die Mischung 3 h lang unter Rückfluss. Danach wird im Vakuum eingeengt, der Rückstand erneut mit Dichlormethan versetzt und abermals eingeengt. Zurück bleibt ein viskoses Öl, das im Hochvakuum getrocknet wird.

Man erhält 4.1 g (quant.) der Zielverbindung, welche ohne weitere Aufreinigung und Charakterisierung weiter umgesetzt wird.

### Intermediat 11A

### 5-Chlorthiophen-2-carbonylchlorid

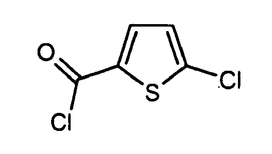

480 mg (2.95 mmol) 5-Chlorthiophen-2-carbonsäure werden in 24 ml Dichlormethan gelöst und mit 2.4 ml (27.5 mmol) Oxalylchlorid versetzt. Man erhitzt die Mischung 16 h lang unter Rückfluss. Danach wird im Vakuum eingeengt, der Rückstand erneut mit Dichlormethan versetzt und abermals eingeengt. Zurück bleibt ein viskoses Öl, das im Hochvakuum getrocknet wird. Man erhält 534 mg (quant.) der Zielverbindung, welche ohne weitere Aufreinigung und Chärakterisierung weiter umgesetzt wird.

### Intermediat 12A

Benzyl (5-chlor-5-oxopentyl)(4-methoxybenzyl)-carbamat

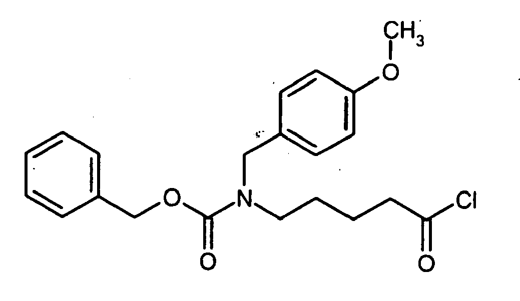

### Stufe a):

10 g (85.4 mmol) 5-Aminovaleriansäure, 17.4 g (128 mmol) p-Anisaldehyd und 10.3 g (85.4 mmol) Magnesiumsulfat werden in 330 ml Ethanol aufgenommen und 1 h unter Rückfluss erhitzt.
Man filtriert danach ab, wäscht mit Ethanol nach und versetzt anschließend das Filtrat portionsweise innerhalb von 15 min mit insgesamt 1.94 g (51.2 mmol) Natriumborhydrid. Man setzt zunächst 10 ml Wasser zu und dann 128 ml einer 2 M Natronlauge. Nach 5 min wird mit 300 ml Wasser verdünnt und anschließend dreimal mit je 200 ml Ethylacetat ausgeschüttelt. Die wässrige Phase wird mit 4 M Salzsäure auf pH 2 eingestellt und im Vakuum eingeengt. Der Rückstand wird durch Flash-Chromatographie an Kieselgel mit Acetonitril/Wasser/Essigsäure 5:1:0.1 als Eluent gereinigt. Die Produktfraktionen werden eingeengt und mit Ethylacetat und Diethylether verrührt. Der Rückstand wird dann abgesaugt und im Hochvakuum getrocknet. Man erhält 9.1 g (45% d. Th.) der p-Methoxybenzyl-geschützten 5-Aminovaleriansäure.

### Stufe b):

Das so erhaltene 5-Aminovaleriansäure-Derivat wird in Dioxan/Wasser (1:1) aufgenommen, mit Natronlauge auf pH 10 eingestellt und anschließend tropfenweise mit 12.97 g (76 mmol) Benzylchlorcarbonat versetzt. Nach 1 min Rühren bei RT wird das Dioxan im Vakuum entfernt und die verbleibende Lösung mit 2 M Salzsäure auf pH 2 eingestellt. Man extrahiert mit Ethylacetat und wäscht die organische Phase anschließend zweimal mit Wasser. Die organische Phase wird dann eingeengt und der Rückstand im Hochvakuum getrocknet. Anschließend erfolgt eine Reinigung durch Flash-Chromatographie an Kieselgel mit Acetonitril als Eluent. Die Produktfraktionen werden eingeengt und der Rückstand im Hochvakuum getrocknet. Man erhält 5.6 g (38% d. Th.) der Z-geschützten Aminosäure.

### LC-MS (Methode 3): Rₜ = 2.47 min; m/z = 372 (M+H)⁺.

### Stufe c):

5.6 g (15 mmol) der so erhaltenen 5-{((Benzyloxy)carbonyl](4-methoxybenzyl)amino}valeriansäure werden in 60 ml Dichlormethan gelöst und mit 2.2 ml Thionylchlorid versetzt. Man erhitzt die Mischung 30 min lang unter Rückfluss. Danach wird im Vakuum eingeengt, der Rückstand erneut mit Dichlormethan versetzt und abermals eingeengt. Zurück bleibt ein viskoses Öl, das im Hochvakuum getrocknet wird. Man erhält 5.7 g (98% d. Th.) der Zielverbindung, welche ohne weitere Aufreinigung und Charakterisierung weiter umgesetzt wird.

### Intermediat 13A

### Benzyl (6-chlor-6-oxohexyl)(4-methoxybenzyl)-carbamat

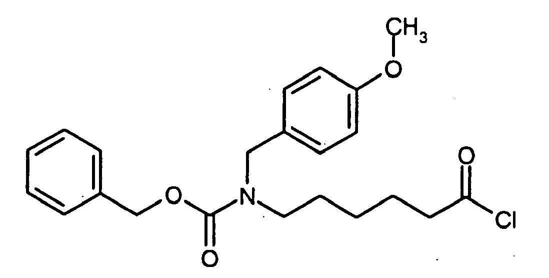

Die Titelverbindung wird in Analogie zu Intermediat 12A ausgehend von 6-Aminocapronsäure hergestellt.

### Intermediat 14A

### Benzyl (4-chlor-4-oxobutyl)(4-methoxybenzyl)-carbamat

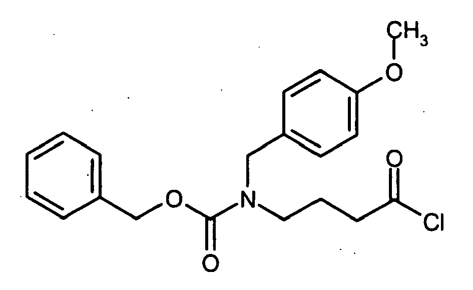

Die Titelverbindung wird in Analogie zu Intermediat 12A ausgehend von 4-Aminobuttersäure hergestellt.

### Intermediat 15A

### Benzyl butyl(4-chlor-4-oxobutyl)-carbamat

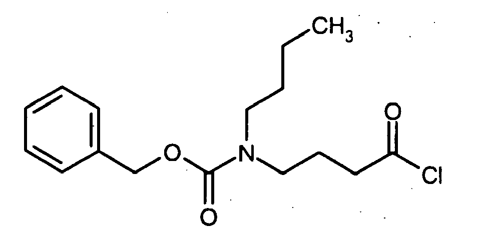

Zunächst wird 4-{[(Benzyloxy)carbonyl](butyl)amino}buttersäure nach Literaturvorschrift [Org. Prep. Proc. Int. 9 (2), 49 (1977)] in Verbindung mit nachfolgender Einführung der Z-Schutzgruppe hergestellt. Das korrespondierende Säurechlorid wird dann wie bei Intermediat 7A beschrieben hergestellt.

### Intermediat 16A

### Benzyl [2-(2-chlor-2-oxoethoxy)ethyl](4-methoxybenzyl)-carbamat

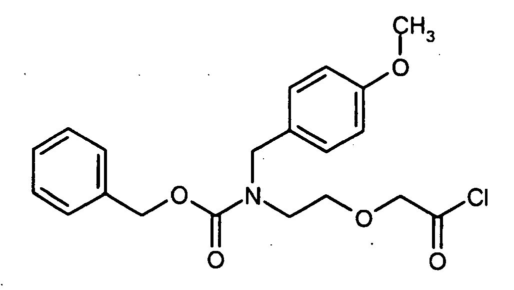

### Stufe a):

12 g (74.4 mmol) *tert*.-Butyl (2-hydroxyethyl)-carbamat werden mit 43.6 g (223.3 mmol) *tert.-*Butylbromacetat in einem Gemisch aus 400 ml Toluol und 20 g konzentrierter Natronlauge in Gegenwart von 200 mg (0.59 mmol) Tetrabutylammoniumhydrogensulfat 1 h lang bei RT gerührt. Danach werden nochmals 15 g *tert*.-Butylbromacetat zugesetzt und die Mischung eine weitere Stunde bei RT gerührt. Man verdünnt dann mit Toluol und Wasser und trennt die Phasen. Die organische Phase wird getrocknet und im Vakuum eingeengt. Der Rückstand wird mit 100 ml wasserfreier Trifluoressigsäure versetzt und 90 min bei RT gerührt. Man engt danach ein und behandelt den harzigen Rückstand mit Diethylether. Man dekantiert ab und trocknet das verbleibende Harz im Hochvakuum. Man erhält so 10.8 g (62% d. Th.) der Zwischenverbindung (2-Amino-ethoxy)essigsäure (als Trifluoracetat).
DC (Acetonitril/Wasser/Eisessig 5:1:0.2): R_{f} = 0.08.

### Stufe b):

Die oben erhaltene Aminosäure wird anschließend, wie für Intermediat 12A beschrieben, zu (2-{[(Benzyloxy)carbonyl](4-methoxybenzyl)amino}ethoxy)essigsäure umgesetzt.
Ausbeute: 2.56 g (13% d. Th.)
HPLC (Methode 2): Rₜ = 5.16 min;
LC-MS (Methode 12): Rₜ = 3.1 min; m/z = 374 (M+H)⁺.

### Stufe c):

2.56 g (6.86 mmol) der erhaltenen (2-{[(Benzyloxy)carbonyl](4-methoxybenzyl)amino}ethoxy)-essigsäure werden dann, wie bei Intermediat 12A beschrieben, mit Thionylchlorid zur Titelverbindung umgesetzt.

Ausbeute: 2.65 g (99% d. Th.) HPLC (Methode 2): Rₜ = 5.11 min.

### Ausführungsbeispiele:

### Allgemeine Vorschrift 2 zur Herstellung von Caesium-Salzen von Carbonsäuren oder geeignet geschützten Aminosäure-Derivaten:

1 mmol der entsprechenden Carbonsäure wird in einer Mischung aus 10 ml Dioxan und 10 ml Wasser gelöst und mit 0.5 mmol Caesiumcarbonat versetzt. Anschließend wird lyophilisiert.

### Beispiel 1

### 2-[[(5-Chlor-2-thienyl)carbonyl]({(5S)-2-oxo-3-[4-(3-oxomorpholin-4-yl)phenyl]-1,3-oxazolidin-5-yl}methyl)amino]-2-oxoethyl-glycinat-Hydrochlorid

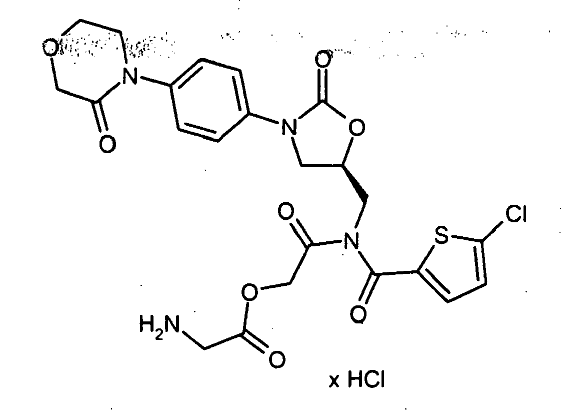

### Stufe a):

11 mg (21 µmol) von Intermediat 1A werden mit 7.9 mg (26 µmol) des Caesiumsalzes von Boc-Glycin (hergestellt aus Boc-Glycin nach der Allgemeinen Vorschrift 2) in 5 ml DMF gelöst. Nach 3 h Rühren bei RT wird durch präparative HPLC (Methode 1) aufgereinigt. Die entsprechenden Fraktionen werden eingeengt und im Hochvakuum getrocknet.
Ausbeute: 7 mg (50% d. Th.)
HPLC (Methode 2): Rₜ = 5.2 min;
LC-MS (Methode 6): Rₜ = 2.11 min; m/z = 651 (M+H)⁺.

### Stufe b):

7 mg (11 µmol) der oben erhaltenen geschützten Zwischenstufe werden mit 3 ml einer 22%-igen Lösung von Chlorwasserstoff in Dioxan versetzt. Nach 30 min wird das Gemisch im Vakuum bei ≤ 25°C eingeengt und der Rückstand aus Dioxan lyophilisiert.
Ausbeute: 4.5 mg (72% d. Th.)
HPLC (Methode 2): Rₜ = 4.2 min;
LC-MS (Methode 5): Rₜ = 1.41 min; m/z = 551 (M+H)⁺.

### Beispiel 2

### 2-[[(5-Chlor-2-thienyl)carbonyl]({(5S)-2-oxo-3-[4-(3-oxomorpholin-4-yl)phenyl]-1,3-oxazolidin-5-yl}methyl)amino]-2-oxoethyl-2-methylalaninat-Hydrochlorid

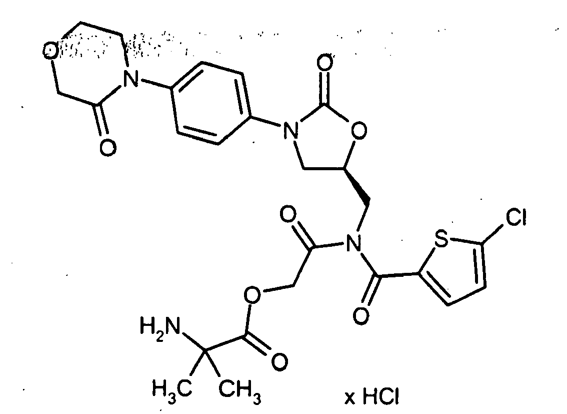

### Stufe a):

38 mg (74 µmol) von Intermediat 1A werden mit 32.3 mg (96 µmol) des Caesiumsalzes von *N-*Boc-2-methylalanin (hergestellt aus *N-*Boc-2-methylalanin nach der Allgemeinen Vorschrift 2) in 38 ml DMF gelöst. Nach 3 h Rühren bei RT unter Ultraschallbehandlung wird durch präparative HPLC (Methode 1) aufgereinigt. Die entsprechenden Fraktionen werden eingeengt und im Hochvakuum getrocknet.
Ausbeute: 29 mg (58% d. Th.)
HPLC (Methode 2): Rₜ = 5.38 min;
LC-MS (Methode 6): Rₜ = 2.27 min; m/z = 679 (M+H)⁺.

### Stufe b:

16.3 mg (24 µmol) der oben erhaltenen geschützten Zwischenstufe werden mit 3 ml einer 22%-igen Lösung von Chlorwasserstoff in Dioxan versetzt. Nach 30 min wird das Gemisch im Vakuum bei ≤ 25°C eingeengt und der Rückstand aus Dioxan lyophilisiert.
Ausbeute: 13 mg (88% d. Th.)
HPLC (Methode 2): Rₜ = 4.3 min;
LC-MS (Methode 6): Rₜ =1.27 min; m/z = 579 (M+H)⁺.

### Beispiel 3

### 2-[[(5-Chlor-2-thienyl)carbonyl]({(5S)-2-oxo-3-[4-(3-oxomorpholin-4-yl)phenyl]-1,3-oxazolidin-5-yl}methyl)amino]-2-oxoethyl-L-valinat-Hydrochlorid

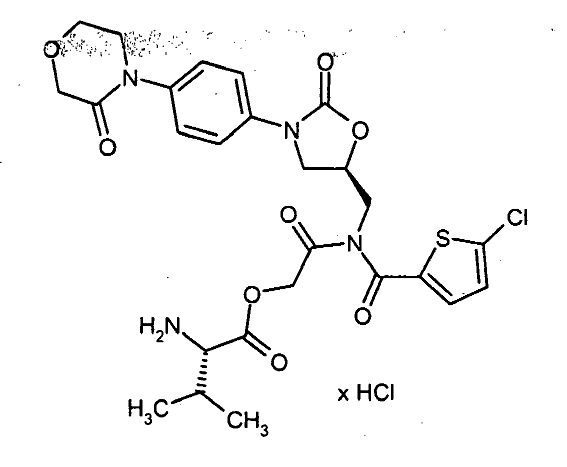

### Stufe a):

19 mg (37 µmol) von Intermediat 1A werden mit 16.8 mg (48 µmol) des Caesiumsalzes von *N-*Boc-Valin (hergestellt aus *N-*Boc-Valin nach der Allgemeinen Vorschrift 2) in 10 ml DMF gelöst. Nach 1.5 h Rühren bei RT unter Ultraschallbehandlung wird durch präparative HPLC (Methode 1) aufgereinigt. Die entsprechenden Fraktionen werden eingeengt und im Hochvakuum getrocknet.
Ausbeute: 13.5 mg (53% d. Th.)
HPLC (Methode 2): Rₜ = 5.45 min;
LC-MS (Methode 4): Rₜ = 2.69 min; m/z = 693 (M+H)⁺.

### Stufe b):

13.5 mg (19 µmol) der oben erhaltenen geschützten Zwischenstufe werden mit 3 ml einer 22%-igen Lösung von Chlorwasserstoff in Dioxan versetzt. Nach 30 min wird das Gemisch im Vakuum bei ≤ 25°C eingeengt und der Rückstand aus Dioxan lyophilisiert.
Ausbeute: 12 mg (98% d. Th.)
HPLC (Methode 2): Rₜ = 4.43 min;
LC-MS (Methode 5): Rₜ = 1.54 min; m/z = 593 (M+H)⁺.

### Beispiel 4

### 2-[[(5-Chlor-2-thienyl)carbonyl]({(5S)-2-oxo-3-[4-(3-oxomorpholin-4-yl)phenyl]-1,3-oxazolidin-5-yl}methyl)amino]-2-oxoethyl-L-prolinat-Hydrochlorid

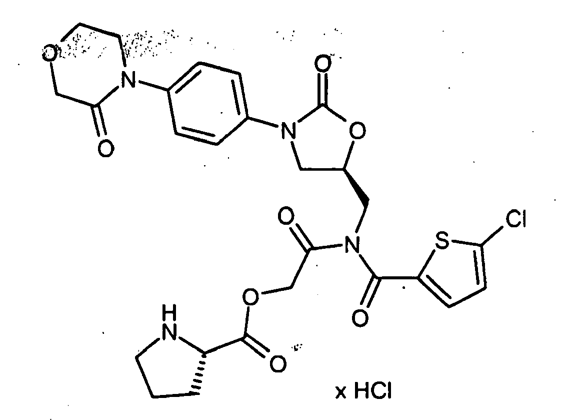

### Stufe a):

20 mg (39 µmol) von Intermediat 1A werden mit 17.6 mg (51 µmol) des Caesiumsalzes von *N-*Boc-Prolin (hergestellt aus *N-*Boc-Prolin nach der Allgemeinen Vorschrift 2) in 5 ml DMF gelöst. Nach 2 h Rühren bei RT unter Ultraschallbehandlung wird durch präparative HPLC (Methode 1) aufgereinigt. Die entsprechenden Fraktionen werden eingeengt und im Hochvakuum getrocknet.
Ausbeute: 11.4 mg (42% d. Th.)
HPLC (Methode 2): Rₜ = 5.44 min;
LC-MS (Methode 4): Rₜ =2.61 min; m/z = 691 (M+H)⁺.

### Stufe b):

11.3 mg (16 µmol) der oben erhaltenen geschützten Zwischenstufe werden mit 2.5 ml einer 22%-igen Lösung von Chlorwasserstoff in Dioxan versetzt. Nach 30 min wird das Gemisch im Vakuum bei ≤ 25°C eingeengt und der Rückstand aus Dioxan lyophilisiert.
Ausbeute: 10 mg (97% d. Th.)
HPLC (Methode 2): Rₜ = 4.34 min;
LC-MS (Methode 6): Rₜ = 1.26 min; m/z = 591 (M+H)⁺.

### Beispiel 5

### 2-[[(5-Chlor-2-thienyl)carbonyl]({(5S)-2-oxo-3-[4-(3-oxomorpholin-4-yl)phenyl]-1,3-oxazolidin-5-yl}methyl)amino]-2-oxoethyl-N-methylglycinat-Hydrochlorid

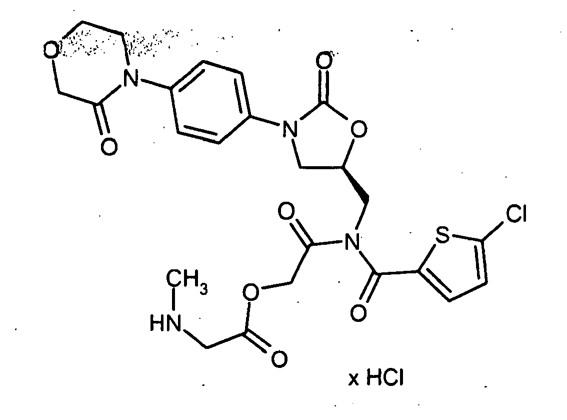

### Stufe a):

20 mg (39 µmol) von Intermediat 1A werden mit 17.5 mg (55 µmol) des Caesiumsalzes von *N-*Boc-Sarcosin (hergestellt aus *N-*Boc-Sarcosin nach der Allgemeinen Vorschrift 2) in 4 ml DMF gelöst. Nach 3 h Rühren bei RT unter Ultraschallbehandlung wird durch präparative HPLC (Methode 1) aufgereinigt. Die entsprechenden Fraktionen werden eingeengt und im Hochvakuum getrocknet.
Ausbeute: 11 mg (42% d. Th.)
HPLC (Methode 2): Rₜ = 5.35 min;
LC-MS (Methode 5): Rₜ = 2.43 min; m/z =665 (M+H)⁺.

### Stufe b):

11 mg (16 µmol) der oben erhaltenen geschützten Zwischenstufe werden mit 2 ml einer 22%-igen Lösung von Chlorwasserstoff in Dioxan versetzt. Nach 30 min wird das Gemisch im Vakuum bei ≤ 25°C eingeengt und der Rückstand aus Dioxan lyophilisiert.
Ausbeute: 9.5 mg (96% d. Th.)
HPLC (Methode 2): Rₜ = 4.24 min;
LC-MS (Methode 4): Rₜ =1.57 min; m/z = 565 (M+H)⁺.

### Beispiel 6

### 2-[[(5-Chlor-2-thienyl)carbonyl]({(5S)-2-oxo-3-[4-(3-oxomorpholin-4-yl)phenyl]-1,3-oxazolidin-5-yl}methyl)amino]-2-oxoethyl-D-valinat-Hydrochlorid

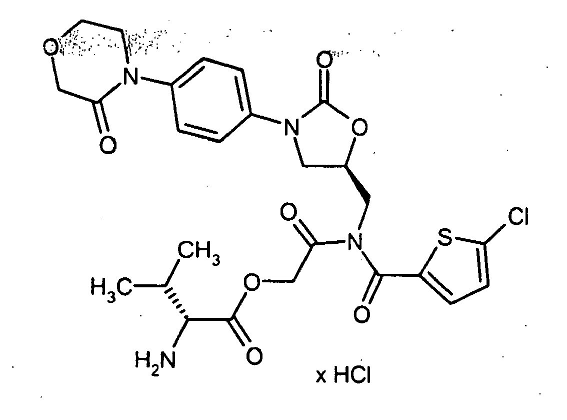

### Stufe a):

20 mg (40 µmol) von Intermediat 1 A werden mit 19 mg (55 µmol) des Caesiumsalzes von Boc-D-Valin (hergestellt aus Boc-D-Valin nach der Allgemeinen Vorschrift 2) in 4 ml DMF gelöst. Nach 2 h Rühren bei RT wird durch präparative HPLC (Methode 1) aufgereinigt. Die entsprechenden Fraktionen werden eingeengt und im Hochvakuum getrocknet.
Ausbeute: 23 mg (85% d. Th.)
HPLC (Methode 2): Rₜ = 5.6 min;
LC-MS (Methode 4): Rₜ = 2.72 min; m/z =693 (M+H)⁺.

### Stufe b):

23 mg (33 µmol) der oben erhaltenen geschützten Zwischenstufe werden mit 3 ml einer 22%-igen Lösung von Chlorwasserstoff in Dioxan versetzt. Nach 30 min wird das Gemisch im Vakuum bei ≤ 25°C eingeengt und der Rückstand aus Dioxan lyophilisiert.
Ausbeute: 20 mg (96% d. Th.)
HPLC (Methode 2): Rₜ = 4.5 min;
LC-MS (Methode 6): Rₜ = 1.3 min; m/z =593 (M+H)⁺.

### Beispiel 7

### 2-[[(5-Chlor-2-thienyl)carbonyl]({(5S)-2-oxo-3-[4-(3-oxomorpholin-4-yl)phenyl]-1,3-oxazolidin-5-yl}methyl)amino]-2-oxoethyl-L-lysinat-Dihydrochlorid

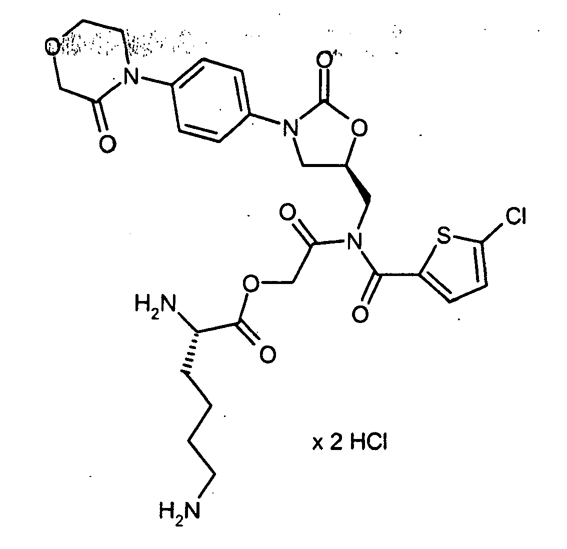

### Stufe a):

20 mg (40 µmol) von Intermediat 1 A werden mit 26 mg (55 µmol) des Caesiumsalzes von *N,N'-*bis-Boc-Lysin (hergestellt aus *N,N'*-bis-Boc-Lysin nach der Allgemeinen Vorschrift 2) in 4 ml DMF gelöst. Nach 2 h Rühren bei RT wird durch präparative HPLC (Methode 1) aufgereinigt. Die entsprechenden Fraktionen werden eingeengt und im Hochvakuum getrocknet.
Ausbeute: 14 mg (43% d. Th.)
HPLC (Methode 2): Rₜ = 5.63 min;
LC-MS (Methode 5): Rₜ =2.66 min; m/z =822 (M+H)⁺.

### Stufe b):

14 mg (17 µmol) der oben erhaltenen geschützten Zwischenstufe werden mit 2.5 ml einer 22%-igen Lösung von Chlorwasserstoff in Dioxan versetzt. Nach 30 min wird das Gemisch im Vakuum bei ≤ 25°C eingeengt und der Rückstand aus Dioxan lyophilisiert.
Ausbeute: 10 mg.(86% d. Th.)
HPLC (Methode 2): Rₜ = 4.1 min;
LC-MS (Methode 6): Rₜ = 0.92 min; m/z =622 (M+H)⁺.

### Beispiel 8

### 2-[[(5-Chlor-2-thienyl)carbony]({(5S)-2-oxo-3-[4-(3-oxomorphin-4-yl)phenyl]-1,3-oxazolidin-5-yl}methyl)amino]-2-oxoethyl-L-histidinat-Dihydrochlorid

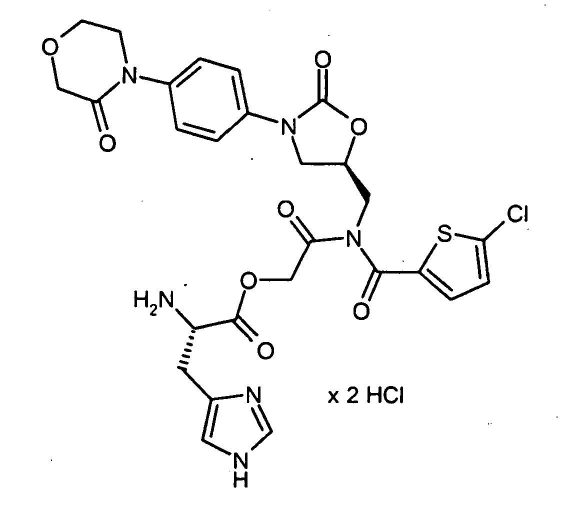

### Stufe a):

20 mg (40 µmol) von Intermediat 1A werden mit 21 mg (55 µmol) des Caesiumsalzes von Boc-L-Histidin (hergestellt aus Boc-L-Histidin nach der Allgemeinen Vorschrift 2) in 4 ml DMF gelöst. Nach 2.5 h Rühren bei RT wird das Lösungsmittel im Vakuum entfernt. Der Rückstand wird in Acetonitril/Wasser (1:1) aufgenommen und durch präparative HPLC (Methode 1) gereinigt. Die entsprechenden Fraktionen werden eingeengt und im Hochvakuum getrocknet.
Ausbeute: 21.9 mg (77% d. Th.)
HPLC (Methode 2): Rₜ = 4.64 min;
LC-MS (Methode 5): Rₜ =1.67 min; m/z =731 (M+H)⁺.

### Stufe b):

21.9 mg (30 µmol) der oben erhaltenen geschützten Zwischenstufe werden mit 6 ml einer 22%-igen Lösung von Chlorwasserstoff in Dioxan versetzt. Nach 90 min wird das Gemisch im Vakuum bei ≤ 25°C eingeengt. Der Rückstand wird in Acetonitril/Wasser (1:1) aufgenommen und durch präparative HPLC (Methode 1) gereinigt. Die entsprechenden Fraktionen werden eingeengt und der Rückstand aus Dioxan lyophilisiert.
Ausbeute: 7.2 mg (34% d. Th.)
HPLC (Methode 2): Rₜ = 4.11 min;
LC-MS (Methode 6): Rₜ = 2.56 min; m/z = 631 (M+H)⁺.

### Beispiel 9

### 2-[[(5-Chlor-2-thienyl)carbonyl]({(5S)-2-oxo-3-[4-(3-oxomorpholin-4-yl)phenyl]-1,3-oxazolidin-5-yl}methyl)amino]-2-oxoethyl-D-histidinat-Dihydrochlorid

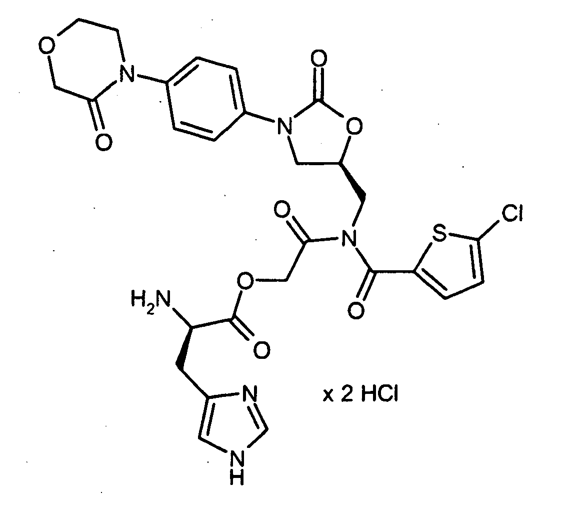

### Stufe a):

25 mg (49 µmol) von Intermediat 1A werden mit 26 mg (68 µmol) des Caesiumsalzes von Boc-D-Histidin (hergestellt aus Boc-D-Histidin nach der Allgemeinen Vorschrift 2) in 5 ml DMF gelöst. Nach 16 h Rühren bei RT wird das Lösungsmittel im Vakuum entfernt. Der Rückstand wird in Acetonitril/Wasser (1:1) aufgenommen und durch präparative HPLC (Methode 1) gereinigt. Die entsprechenden Fraktionen werden eingeengt und im Hochvakuum getrocknet.
Ausbeute: 18.3 mg (51% d. Th.)
HPLC (Methode 2): Rₜ =4.62 min.

### Stufe b):

18 mg (25 µmol) der oben erhaltenen geschützten Zwischenstufe werden mit 2 ml einer 22%-igen Lösung von Chlorwasserstoff in Dioxan versetzt. Nach 4 h wird das Gemisch im Vakuum bei ≤ 25°C eingeengt. Der Rückstand wird in Acetonitril/Wasser (1:1) aufgenommen und durch präparative HPLC (Methode I) gereinigt. Die entsprechenden Fraktionen werden eingeengt und der Rückstand aus Dioxan lyophilisiert.
Ausbeute: 6 mg (37% d. Th.)
HPLC (Methode 2): Rₜ = 4.1 min;
LC-MS (Methode 5): Rₜ = 1.15 min; m/z = 631 (M+H)⁺.

### Beispiel 10

### S-{2-[[(5-Chlor-2-thienyl)carbonyl]({(5S)-2-oxo-3-[4-(3-oxomorpholin-4-yl)phenyl]-1,3-oxazolidin-5-yl}methyl)amino]-2-oxoethyl}(2S)-2-amino-3-methylbutanthioat-Hydrobromid

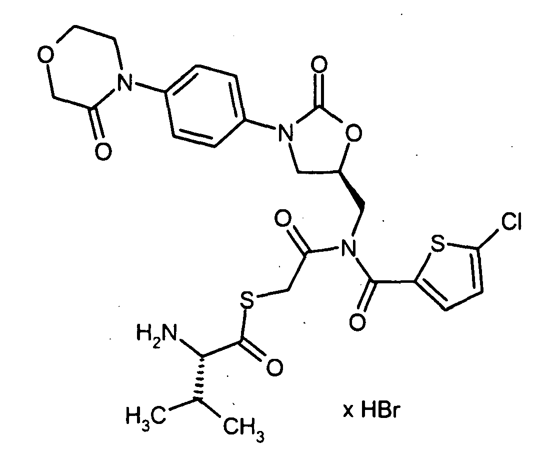

### Stufe a):

### (2S)-2-{[(Benzyloxy)carbonyl]amino}-3-methylbutanthio-S-säure

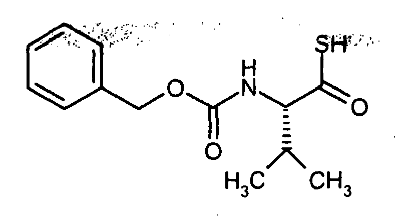

Die Titelverbindung wird aus Z-Valin analog literaturbekannter Vorschrift [R. Michelot et al., Bioorg. Med. Chem. 1996, 4, 2201] hergestellt.

### Stufe b):

200 mg (748 µmol) (2*S*)-2-{[(Benzyloxy)carbonyl]amino}-3-methylbutanthio-S-säure werden in 10 ml Dioxan und 10 ml Wasser aufgenommen und mit 110 mg (337 µmol) Caesiumcarbonat versetzt. Sobald eine klare Lösung entstanden ist, wird lyophilisiert. Man erhält 300 mg des Caesiumsalzes in quantitativer Ausbeute.

27 mg (68 µmol) dieses Caesiumsalzes sowie 25 mg (49 µmol) von Intermediat 1A werden in 5 ml DMF gelöst. Nach 2 h Rühren bei RT wird das Lösungsmittel im Vakuum entfernt. Der Rückstand wird in Acetonitril/Wasser (1:1) aufgenommen und durch präparative HPLC (Methode 1) gereinigt. Die entsprechenden Fraktionen werden eingeengt und im Hochvakuum getrocknet.
Ausbeute: 24 mg (66% d. Th.)
HPLC (Methode 2): Rₜ = 5.62 min;
LC-MS (Methode 6): Rₜ = 2.47 min; m/z = 743 (M+H)⁺.

### Stufe c):

24 mg (32.3 µmol) der oben erhaltenen geschützten Zwischenstufe werden mit 2 ml einer 33%-igen Lösung von Bromwasserstoff in Eisessig versetzt. Nach 1 h Rühren bei RT wird das Gemisch im Vakuum bei ≤ 25°C eingeengt und der Rückstand aus Dioxan/Wasser lyophilisiert.
Ausbeute: 21 mg (94% d. Th.)
HPLC (Methode 2): Rₜ = 4.43 min;
LC-MS (Methode 4): Rₜ = 1.63 min; m/z = 609 (M+H)⁺.

### Beispiel 11

### S-{2-[[(5-Chlor-2-thienyl)carbonyl]({(5S)-2-oxo-3-[4-(3-oxomorpholin-4-yl)phenyl]-1,3-oxazolidin-5-yl}methyl)amino]-2-oxoethyl}(2S)-2-amino-3-methylbutanthioat-Hydrochlorid

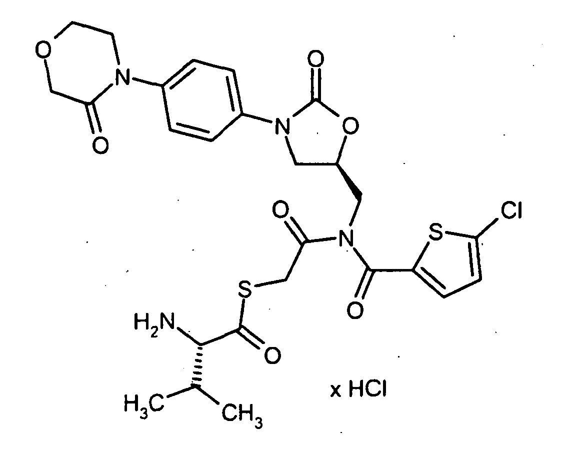

### Stufe a):

### (2S)-2-[(tert.-Butoxycarbonyl)amino]-3-methylbutanthio-S-säure

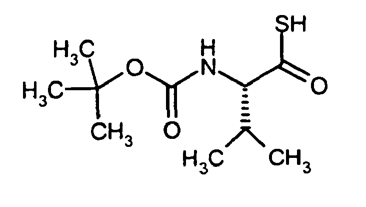

Die Titelverbindung wird aus Boc-Valin analog literaturbekannter Vorschrift [R. Michelot et al., Bioorg. Med. Chem. 1996, 4, 2201) hergestellt.

### Stufe b):

200 mg (857 µmol) (2*S*)-2-[(*tert.*-Butoxycarbonyl)amino]-3-methylbutanthio-S-säure werden in 10 ml Dioxan und 10 ml Wasser aufgenommen und mit 126 mg (386 µmol) Caesiumcarbonat versetzt. Sobald eine klare Lösung entstanden ist, wird lyophilisiert. Man erhält 310 mg des Caesiumsalzes in quantitativer Ausbeute.

25 mg (68 µmol) dieses Caesiumsalzes sowie 25 mg (49 µmol) von Intermediat 1 A werden in 4 ml DMF gelöst. Nach 1 h Rühren bei RT wird das Lösungsmittel im Vakuum entfernt. Der Rückstand wird in Acetonitril/Wasser (1:1) aufgenommen und durch präparative HPLC (Methode 1) gereinigt. Die entsprechenden Fraktionen werden eingeengt und im Hochvakuum getrocknet.
Ausbeute: 23 mg (65% d. Th.)
HPLC (Methode 2): Rₜ=5.64 min;
LC-MS (Methode 4): Rₜ =2.76 min; m/z =709 (M+H)⁺.

### Stufe c):

23 mg (32 µmol) der oben erhaltenen geschützten Zwischenstufe werden mit 4 ml einer 22%-igen Lösung von Chlorwasserstoff in Dioxan versetzt. Nach 1 h Rühren bei RT wird das Gemisch im Vakuum bei ≤ 25°C eingeengt und der Rückstand aus Dioxan lyophilisiert.
Ausbeute: 20 mg (97% d. Th.)
HPLC (Methode 2): Rₜ = 4.47 min;
LC-MS (Methode 6): Rₜ = 1.35 min; m/z = 609 (M+H)⁺.

### Beispiel 12

### 5-Chlor-N-[4-(methylamino)butanoyl]-N-({(5S)-2-oxo-3-[4-(3-oxomorpholin-4-yl)phenyl]-1,3-oxazolidin-5-yl}methyl)thiophen-2-carboxamid-Hydrobromid

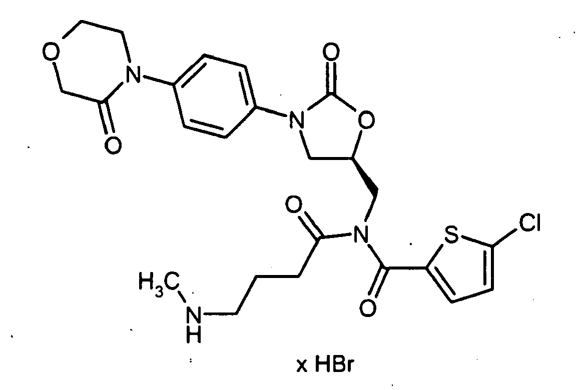

### Stufe a):

145.4 mg (0.334 mmol) der Verbindung (A) werden in 25 ml DMF gelöst, mit 24 mg (1 mmol) Natriumhydrid versetzt, und die Mischung 30 min bei RT gerührt. Dann gibt man 1.8 g (6.67 mmol) des Intermediats 7A hinzu. Man rührt weitere 15 min bei RT und versetzt den Ansatz dann mit einigen Tropfen Wasser. Danach wird eingeengt und der Rückstand in 300 ml Dichlormethan aufgenommen. Man schüttelt zunächst mit Wasser und dann dreimal mit 300 ml einer 5%-igen Natriumhydrogencarbonat-Lösung aus. Die Dichlormethan-Phase wird abgetrennt und eingeengt. Der Rückstand wird mit einer Mischung aus 15 ml Dichlormethan und 10 ml Diethylether verrührt. Man filtriert vom Ungelösten ab und engt die verbleibende Lösung ein. Der Rückstand wird durch präparative HPLC (Methode 1) gereinigt. Die entsprechenden Fraktionen, die ein bis-acyliertes Nebenprodukt, das nach Enolisierung der Mono-Acylverbindung entsteht, enthalten, werden eingeengt und im Hochvakuum getrocknet. Man erhält eine saubere Fraktion von 41 mg (13.6% d. Th.) sowie eine leicht verunreinigte Fraktion von 59 mg (19.6% d. Th.), die zusammen in der Folgestufe eingesetzt werden.
HPLC (Methode 2): Rₜ = 6.06 min;
LC-MS (Methode 5): Rₜ = 2.88 min; m/z =902 (M+H)⁺.

### Stufe b):

100 mg (0.11 mmol) der oben erhaltenen Z-geschützten Zwischenstufe werden in 3.3 ml Eisessig aufgenommen und mit 17 ml einer 33%-igen Lösung von Bromwasserstoff in Eisessig versetzt. Unter diesen Bedingungen kommt es auch zur Spaltung des Enolesters. Nach 5 min Rühren bei RT ist die Umsetzung zur Zielverbindung abgeschlossen und der Ansatz wird im Hochvakuum eingeengt. Der Rückstand wird zweimal aus Acetonitril eingeengt und anschließend durch präparative HPLC gereinigt.
Ausbeute: 29.6 mg (73.5% d. Th.)
HPLC (Methode 2): Rₜ = 4.2 min;
LC-MS (Methode 6): Rₜ = 1.19 min; m/z = 535 (M+H)⁺.

### Beispiel 13

### 5-Chlor-N-[4-(methylamino)butanoyl]-N-({(5S)-2-oxo-3-[4-(3-oxomorpholin-4-yl)phenyl]-1,3-oxazolidin-5-yl}methyl)thiophen-2-carboxamid-Hydrochlorid

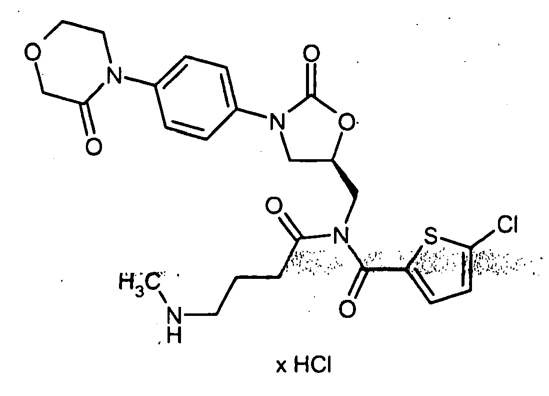

59 mg (0.096 mmol) der Verbindung aus Beispiel 12 werden in 40 ml Acetonitril/Wasser (1:1) gelöst und mit 1 g (4.8 mmol) Pyrokohlensäure-di-*tert*.-butylester ("Boc-anhydrid") versetzt. Anschließend werden portionsweise 37 µl *N,N-*Diisopropylethylamin zugegeben, wobei ein pH-Wert von 7.8 nicht überschritten wird. Nach Beendigung der Reaktion nach etwa 15 min wird mit Essigsäure ein pH-Wert zwischen 3 und 4 eingestellt und im Vakuum eingeengt. Der Rückstand wird in Acetonitril aufgenommen und durch präparative HPLC (Methode 1) gereinigt. Die entsprechenden Fraktionen werden eingeengt und im Vakuum getrocknet. Man erhält 15.5 mg (26% d. Th.) der Boc-geschützten Zwischenstufe. 12.5 mg (0.02 mmol) hiervon werden sodann mit 5 ml einer 22%-igen Lösung von Chlorwasserstoff in Dioxan behandelt. Nach 10 min wird das Gemisch im Vakuum bei ≤ 25°C eingeengt. Der Rückstand wird in Wasser aufgenommen und mit Dichlormethan durchgeschüttelt. Die wässrige Phase wird mit Salzsäure auf pH 4 eingestellt und anschließend lyophilisiert.
Ausbeute: 3.5 mg (31 % d. Th.)
HPLC (Methode 2): Rₜ = 4.24 min;
LC-MS (Methode 5): Rₜ = 1.43 min; m/z = 535 (M+H)⁺.

### Beispiel 14

### 5-Chlor-N-[5-(methylamino)pentanoyl]-N-({(5S)-2-oxo-3-[4-(3-oxomorpholin-4-yl)phenyl]-1,3-oxazolidin-5-yl}methyl)thiophen-2-carboxamid-Hydrobromid

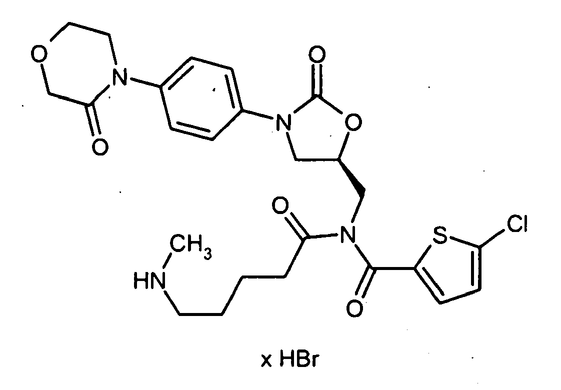

### Stufe a):

153.6 mg (0.352 mmol) der Verbindung (A) werden in 25 ml DMF gelöst, mit 25 mg (1 mmol) Natriumhydrid versetzt, und die Mischung 30 min bei RT gerührt. Dann gibt man 2 g (7.05 mmol) des Intermediats 8A, gelöst in 5 ml DMF, hinzu. Man rührt weitere 15 min bei RT und versetzt den Ansatz dann mit einigen Tropfen Wasser. Danach wird eingeengt und der Rückstand in 500 ml Dichlormethan aufgenommen. Man schüttelt zweimal mit 300 ml einer 5%-igen Natriumhydrogencarbonat-Lösung aus. Die Dichlormethan-Phase wird abgetrennt und eingeengt. Der Rückstand wird durch präparative HPLC (Methode 1) gereinigt. Die entsprechenden Fraktionen, die ein bis-acyliertes Nebenprodukt, das nach Enolisierung der Mono-Acylverbindung entsteht, enthalten, werden eingeengt und im Hochvakuum getrocknet. Man erhält 50 mg (15.2% d. Th.) einer leicht verunreinigten Fraktion, die als solche in der Folgestufe eingesetzt wird.
HPLC (Methode 2): Rₜ =6.23 min.

### Stufe b):

50 mg (0.027 mmol) der oben erhaltenen Z-geschützten Zwischenstufe werden in 1 ml Eisessig aufgenommen und mit 5 ml einer 33%-igen Lösung von Bromwasserstoff in Eisessig versetzt. Unter diesen Bedingungen kommt es auch zur Spaltung des Enolesters. Nach 10 min Rühren bei RT ist die Umsetzung zur Zielverbindung abgeschlossen und der Ansatz wird im Hochvakuum eingeengt. Der Rückstand wird zweimal aus Acetonitril eingeengt und anschließend durch mehrmalige präparative HPLC gereinigt.
Ausbeute: 0.5 mg (3% d. Th.)
HPLC (Methode 2): Rₜ = 4.32 min;
LC-MS (Methode 4): Rₜ = 1.31 min; m/z = 549 (M+H)⁺.

### Beispiel 15

### N-Methylglycyl-N-[(5-chlor-2-thienyl)carbonyl]-N²-methyl-N-({(5S)-2-oxo-3-[4-(3-oxomorpholin-4-yl)phenyl]-1,3-oxazolidin-5-yl}methyl)glycinamid-Hydrobromid

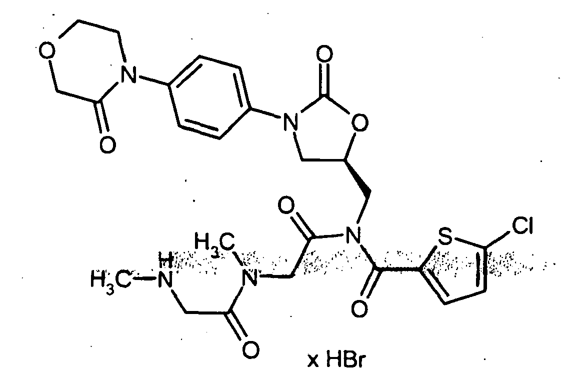

### Stufe a):

108 mg (0.19 mmol) des Intermediats 2A werden in 25 ml DMF gelöst, mit 14 mg (0.57 mmol) Natriumhydrid versetzt, und die Mischung 15 min bei RT gerührt. Dann gibt man 534 mg (2.95 mmol) des Intermediats 11A, gelöst in 5 ml DMF, hinzu. Man rührt weitere 10 min bei RT und versetzt den Ansatz dann mit einigen Tropfen Wasser. Danach wird eingeengt und der Rückstand in 500 ml Dichlormethan aufgenommen. Man schüttelt dreimal mit 300 ml einer 5%-igen Natriumhydrogencarbonat-Lösung aus. Die Dichlormethan-Phase wird abgetrennt und eingeengt. Der Rückstand wird durch zweimalige präparative HPLC (Methode 1) gereinigt. Die entsprechenden Fraktionen werden eingeengt und im Hochvakuum getrocknet.
Ausbeute: 31 mg (23% d. Th.)
HPLC (Methode 2): Rₜ = 5.15 min;
LC-MS (Methode 5): Rₜ =2.28 min; m/z = 712 (M+H)⁺.

### Stufe b):

31 mg (0.044 mmol) der oben erhaltenen Z-geschützten Zwischenstufe werden in 1 ml Eisessig aufgenommen und mit 3 ml einer 33%-igen Lösung von Bromwasserstoff in Eisessig versetzt. Nach 45 min Rühren bei RT wird im Hochvakuum eingeengt. Der Rückstand wird durch mehrmalige präparative HPLC gereinigt.
Ausbeute: 1 mg (3.3% d. Th.)
HPLC (Methode 2): Rₜ = 4.23 min;
LC-MS (Methode 4): Rₜ = 1.32 min; m/z = 578 (M+H)⁺.

### Beispiel 16

### N-Methyl-β-alanyl-N-[(5-chlor-2-thienyl)carbonyl]-N²-methyl-N-({(5S)-2-oxo-3-[4-(3-oxomorpho-lin-4-yl)phenyl]-1,3-oxazolidin-5-yl}methyl)glycinamid-Hydrobromid

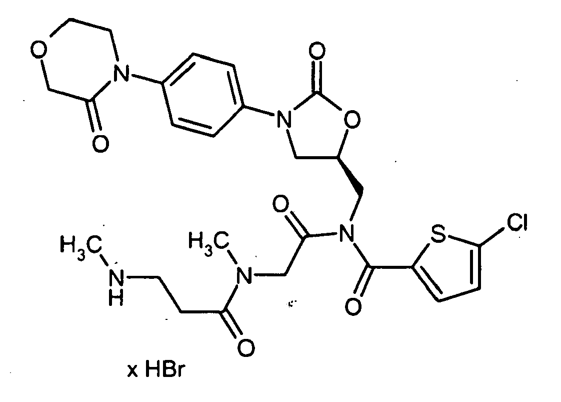

### Stufe a):

40 mg (0.069 mmol) des Intermediats 6A werden in 10 ml DMF gelöst, mit 5 mg (0.21 mmol) Natriumhydrid versetzt, und die Mischung 15 min bei RT gerührt. Dann gibt man 249 mg (1.38 mmol) des Intermediats 11 A, gelöst in 5 ml DMF, hinzu. Man rührt weitere 15 min bei RT und versetzt den Ansatz dann mit einigen Tropfen Wasser. Danach wird eingeengt und der Rückstand in 500 ml Dichlormethan aufgenommen. Man schüttelt zweimal mit 50 ml einer 5%-igen Natriumhydrogencarbonat-Lösung aus. Die Dichlormethan-Phase wird abgetrennt und eingeengt. Der Rückstand wird durch zweimalige präparative HPLC (Methode 1) gereinigt. Die entsprechenden Fraktionen werden eingeengt und im Hochvakuum getrocknet.
Ausbeute: 4.6 mg (9.2% d. Th.)
HPLC (Methode 2): Rₜ = 5.16 min;
LC-MS (Methode 6): Rₜ = 2.17 min; m/z = 726 (M+H)⁺.

### Stufe b):

4.2 mg (0.006 mmol) der oben erhaltenen Z-geschützten Zwischenstufe werden mit 1 ml einer 33%-igen Lösung von Bromwasserstoff in Eisessig versetzt. Nach 30 min Rühren bei RT wird im Hochvakuum eingeengt. Der Rückstand wird in Wasser aufgenommen, mit Dichlormethan ausgeschüttelt und die wässrige Phase anschließend lyophilisiert.
Ausbeute: 2 mg (51% d. Th.)
HPLC (Methode 2): Rₜ = 4.25 min;
LC-MS (Methode 6): Rₜ = 1.16 min; m/z.= 592 (M+H)⁺.

### Beispiel 17

### 5-Chlor-N-[5-(methylamino)pentanoyl]-N-({(5S)-2-oxo-3-[4-(3-oxomorpholin-4-yl)phenyl]-1,3-oxazolidin-5-yl}methyl)thiophen-2-carboxamid-Hydrochlorid

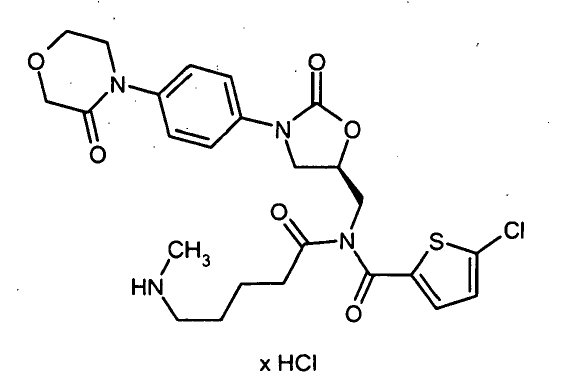

### Stufe a):

1.96 g (4.5 mmol) der Verbindung (A) werden in 70 ml DMF gelöst, mit 323 mg (13.5 mmol) Natriumhydrid versetzt, und die Mischung 30 min bei RT gerührt. Dann gibt man 5.1 g (18 mmol) des Intermediats 8A, gelöst in 10 ml DMF, hinzu. Man rührt weitere 15 min bei RT und versetzt den Ansatz dann mit 20 ml Wasser. Danach wird eingeengt und der Rückstand in 500 ml Ethylacetat aufgenommen. Man schüttelt dreimal mit 300 ml einer 5%-igen Natriumhydrogencarbonat-Lösung aus. Die Ethylacetat-Phase wird abgetrennt und eingeengt. Der Rückstand wird mit 40 ml einer Dichlormethan/Diethylether-Mischung (2:1) verrührt und anschließend filtriert. Die verbleibende Lösung wird im Vakuum eingeengt. Der Rückstand wird dann 2 h lang mit 100 ml einer gesättigten Lösung von Chlorwasserstoff in Dichlormethan verrührt, wobei der initial entstandene Enolester gespalten wird. Anschließend wird eingeengt und der verbleibende Rückstand durch Flash-Chromatographie an Kieselgel mit Ethylacetat/Toluol 5:1 als Eluent gereinigt. Die entsprechenden Fraktionen werden eingeengt, und man erhält 721 mg (23.5% d. Th.) der Z-geschützten Zwischenstufe.
HPLC (Methode 2): Rₜ =5.54 min.

### Stufe b):

720 mg (1.05 mmol) des oben erhaltenen Z-geschützten Intermediats werden in 20 ml wasserfreier Trifluoressigsäure über Nacht gerührt. Der Ansatz wird danach im Hochvakuum eingeengt, wobei die Temperatur bei etwa 20°C gehalten wird. Der Rückstand wird in 100 ml auf pH 3 eingestellte Salzsäure aufgenommen und je zweimal mit 100 ml Dichlormethan und 100 ml Ethylacetat ausgeschüttelt. Die wässrige Phase wird eingeengt und der Rückstand über präparative HPLC gereinigt. Nach Einengen der entsprechenden Fraktionen und Lyophilisation aus Salzsäure (pH 3) wird die Zielverbindung erhalten.
Ausbeute: 20 mg (3.3% d. Th.)
HPLC (Methode 2): Rₜ = 4.29 min;
LC-MS (Methode 5): Rₜ = 1.27 min; m/z =549 (M+H)⁺.

### Beispiel 18

### 5-Chlor-N-[6-(methylamino)hexanoyl]-N-({(5S)-2-oxo-3-[4-(3-oxomorpholin-4-yl)phenyl]-1,3-oxazolidin-5-yl}methyl)thiophen-2-carboxamid-Hydrochlorid

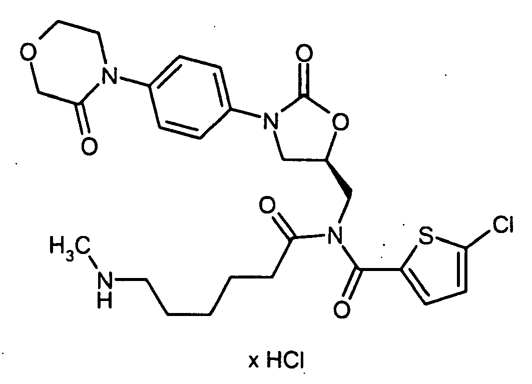

### Stufe a):

2 g (4.59 mmol) der Verbindung (A) werden in 70 ml DMF gelöst, mit 330 mg (13.8 mmol) Natriumhydrid versetzt, und die Mischung 30 min bei RT gerührt. Dann gibt man 5.1 g (18 mmol) des Intermediats 10A, gelöst in 10 ml DMF, hinzu. Man rührt weitere 15 min bei RT und versetzt den Ansatz dann mit 20 ml Wasser. Danach wird eingeengt und der Rückstand 2 h lang mit 100 ml einer gesättigten Lösung von Chlorwasserstoff in Dichlormethan verrührt, wobei der initial entstandene Enolester gespalten wird. Anschließend wird eingeengt und der Rückstand in 600 ml Ethylacetat aufgenommen. Man schüttelt dreimal mit einer 10%-igen Natriumcarbonat-Lösung aus. Die Ethylacetat-Phase wird abgetrennt und eingeengt. Der Rückstand wird mit 150 ml einer Dichlormethan/Diethylether-Mischung (2:1) verrührt und anschließend filtriert. Die verbleibende Lösung wird im Vakuum eingeengt und der Rückstand durch Flash-Chromatographie an Kieselgel mit Ethylacetat/Toluol 5:1 als Eluent gereinigt. Die entsprechenden Fraktionen werden eingeengt,
wobei das Produkt noch leicht verunreinigt erhalten wird. Eine erneute Reinigung durch Flash-Chromatographie an Kieselgel mit Acetonitril/Dichlormethan 1:1 als Eluent führt dann zu 572 mg (18% d. Th.) der sauberen Z-geschützten Zwischenstufe.
HPLC (Methode 2): Rₜ = 5.68 min.

### Stufe b):

572 mg (0.82 mmol) des oben erhaltenen Intermediats werden in 50 ml wasserfreier Trifluoressigsäure für 6 h im Ultraschallbad behandelt. Der Ansatz wird danach im Hochvakuum eingeengt, wobei die Temperatur bei etwa 20°C gehalten wird. Der Rückstand wird in 100 ml auf pH 3 eingestellte Salzsäure aufgenommen und filtriert. Die wässrige Phase wird eingeengt und der Rückstand über präparative HPLC gereinigt. Nach Einengen der entsprechenden Fraktionen und Lyophilisation aus Salzsäure (pH 3) werden 283 mg (58% d. Th.) der Zielverbindung erhalten.
HPLC (Methode 2): Rₜ = 4.35 min;
LC-MS (Methode 6): Rₜ = 1.24 min; m/z = 563 (M+H)⁺.

### Beispiel 19

### N-(4-Aminobutanoyl)-5-chlor-N-({(5S)-2-oxo-3-[4-(3-oxomorpholin-4-yl)phenyl]-1,3-oxazolidin-5-yl}methyl)thiophen-2-carboxamid-Hydrochlorid

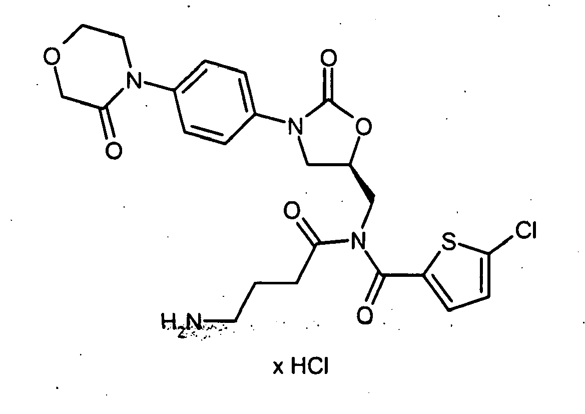

### Stufe a):

1.33 g (3.06 mmol) der Verbindung (A) werden in 75 ml DMF gelöst, mit 220 mg (9.2 mmol) Natriumhydrid versetzt, und die Mischung 30 min bei RT gerührt. Dann gibt man 11.5 g (30.6 mmol) des Intermediats 14A, gelöst in 10 ml DMF, hinzu. Man rührt weitere 15 min bei RT und versetzt den Ansatz dann mit 20 ml Wasser. Danach wird eingeengt und der Rückstand in 300 ml Ethylacetat aufgenommen. Man schüttelt dreimal mit 300 ml einer 10%-igen Natriumcarbonat-Lösung aus. Die organische Phase wird abgetrennt, eingeengt und der Rückstand in 50 ml Dichlormethan aufgenommen. Nach kurzen Verrühren werden ungelöste Bestandteile abfiltriert und die Dichlormethan-Phase eingeengt. Der Rückstand wird durch Flash-Chromatographie an Kieselgel mit Ethylacetat/Toluol 5:1 als Eluent gereinigt. Die produkthaltigen Fraktionen, die ein bis-acyliertes Nebenprodukt (m/z = 1113) enthalten, werden eingeengt. Anschließend wird der Rückstand 2 h lang mit 10 ml einer gesättigten Lösung von Chlorwasserstoff in Dichlormethan verrührt, wobei der initial entstandene Enolester gespalten wird. Anschließend wird eingeengt und der verbleibende Rückstand erneut durch Flash-Chromatographie an Kieselgel mit Ethylacetat/Toluol 5:1 als Eluent gereinigt. Die entsprechenden Fraktionen werden eingeengt, und man erhält 151 mg (7% d. Th.) des zweifach geschützten Intermediats.
HPLC (Methode 2): Rₜ = 5.83 min;
LC-MS (Methode 6): Rₜ = 2.61 min; m/z = 775 (M+H)⁺.

### Stufe b):

151 mg (0.2 mmol) der oben erhaltenen Zwischenstufe werden in 8 ml wasserfreier Trifluoressigsäure über Nacht bei RT gerührt. Der Ansatz wird danach im Hochvakuum eingeengt, wobei die Temperatur bei etwa 20°C gehalten wird. Der Rückstand wird in 100 ml auf pH 3 eingestellte Salzsäure aufgenommen und mit 75 ml Dichlormethan sowie anschließend zweimal mit Ethylacetat ausgeschüttelt. Die wässrige Phase wird eingeengt und der Rückstand aus Salzsäure (pH 3) lyophilisiert.
Ausbeute: 70 mg (64% d. Th.)
HPLC (Methode 2): Rₜ = 4.13 min;
LC-MS (Methode 5): Rₜ =1.38 min; m/z = 521 (M+H)⁺.

### Beispiel 20

### N-(5-Aminopentanoyl)-5-chlor-N-({(5S)-2-oxo-3-[4-(3-oxomorpholin-4-yl)phenyl]-1,3-oxazolidin-5-yl}methyl)thiophen-2-carboxamid-Hydrochlorid

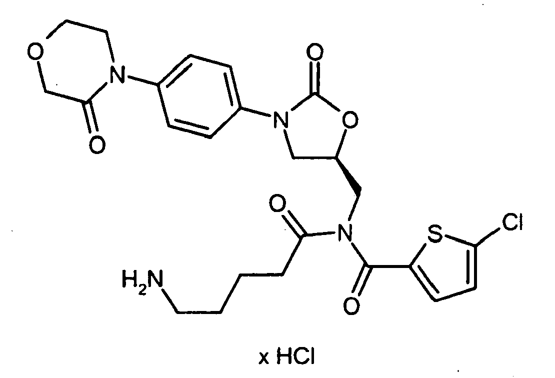

### Stufe a):

2.83 g (6.5 mmol) der Verbindung (A) werden in 100 ml DMF gelöst, mit 468 mg (19.5 mmol) Natriumhydrid versetzt, und die Mischung 30 min bei RT gerührt. Dann gibt man 7.6 g (19.5 mmol) des Intermediats 12A, gelöst in 10 ml DMF, hinzu. Man rührt weitere 15 min bei RT und versetzt den Ansatz dann mit 20 ml Wasser. Danach wird eingeengt und der Rückstand 1 h lang mit 150 ml einer gesättigten Lösung von Chlorwasserstoff in Dichlormethan verrührt, wobei der initial entstandene Enolester gespalten wird. Anschließend wird eingeengt und der Rückstand in 700 ml Ethylacetat aufgenommen. Man schüttelt zweimal mit je 200 ml einer 10%-igen Natriumcarbonat-Lösung aus. Die organische Phase wird abgetrennt, eingeengt und der Rückstand in 30 ml Ethylacetat sowie 30 ml Diethylether aufgenommen. Nach kurzem Verrühren werden ungelöste Bestandteile abfiltriert und die organische Phase eingeengt. Der Rückstand wird durch Flash-Chromatographie an Kieselgel mit Ethylacetat/Toluol 4:1 als Eluent gereinigt. Die entsprechenden Fraktionen werden eingeengt und der Rückstand in 10 ml Ethylacetat aufgenommen. Man gibt 100 ml kalten Diethylether hinzu und lässt 30 min bei 0°C stehen. Man filtriert ab und behandelt den Rückstand nochmals mit 100 ml Diethylether. Nach erneutem Abfiltrieren wird der Rückstand gesammelt und getrocknet. Man erhält 1 g (20% d. Th.) des zweifach geschützten Intermediats.
HPLC (Methode 2): Rₜ = 5.92 min;
LC-MS (Methode 6): Rₜ = 2.68 min; m/z = 789 (M+H)⁺.

### Stufe b):

1.g (1.3 mmol) der erhaltenen Zwischenstufe werden in 70 ml wasserfreier Trifluoressigsäure 6h lang im Ultraschallbad behandelt. Der Ansatz wird danach im Hochvakuum eingeengt, wobei die Temperatur bei etwa 20°C gehalten wird. Der Rückstand wird in 350 ml auf pH 3 eingestellte Salzsäure aufgenommen und nach 15-minütigem Verrühren bei RT mit 100 ml Dichlormethan ausgeschüttelt. Die wässrige Phase wird abgetrennt und anschließend nochmals mit 100 ml Ethylacetat ausgeschüttelt. Die wässrige Phase wird abgetrennt, dann zur Entfernung restlichen Ethylacetats kurz im Hochvakuum andestilliert und schließlich lyophilisiert.
Ausbeute: 586 mg (81% d. Th.)
HPLC (Methode 2): Rₜ = 4.2 min;
LC-MS (Methode 6): Rₜ = 1.17 min; m/z =535 (M+H)⁺.

### Beispiel 21

### N-(6-Aminohexanoyl)-5-chlor-N-({(5S)-2-oxo-3-[4-(3-oxomorpholin-4-yl)phenyl]-1,3-oxazolidin-5-yl}methyl)thiophen-2-carboxamid-Hydrochlorid

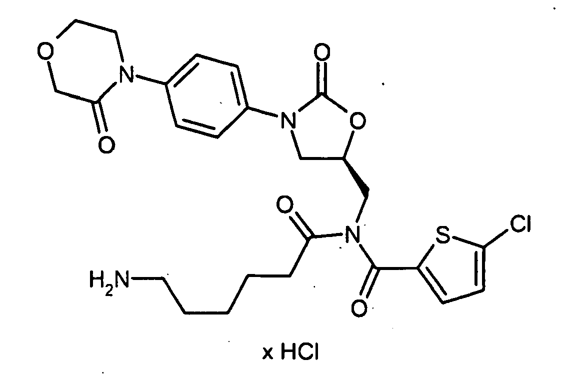

### Stufe a):

1.6 g (3.7 mmol) der Verbindung (A) werden in 50 ml DMF gelöst, mit 263 mg (11 mmol) Natriumhydrid versetzt, und die Mischung 30 min bei RT gerührt. Dann gibt man 14.1 g (36.6 mmol) des Intermediats 13A, gelöst in 10 ml DMF, hinzu. Man rührt weitere 15 min bei RT und versetzt den Ansatz dann mit 20 ml Wasser. Man engt ein und nimmt den Rückstand in 500 ml Ethylacetat auf. Man schüttelt dreimal mit je 100 ml einer 10%-igen Natriumcarbonat-Losung aus. Die organische Phase wird abgetrennt, eingeengt und der Rückstand in 15 ml Dichlormethan/Diethylether (2:1) aufgenommen. Nach kurzen Verrühren werden ungelöste Bestandteile abfiltriert und die organische Phase eingeengt. Der Rückstand wird durch Flash-Chromatographie an Kieselgel mit Ethyläcetat/Toluol 5:1 als Eluent gereinigt. Man erhält 256 mg (9% d. Th.) des zweifach geschützten Intermediats.
HPLC (Methode 2): Rₜ = 6.07 min;
LC-MS (Methode 5): Rₜ = 2.92 min; m/z =803 (M+H)⁺.

### Stufe b):

256 mg (0.32 mmol) der oben erhaltenen Zwischenstufe werden in 10 ml wasserfreier Trifluoressigsäure über Nacht bei Raumtemperatur gerührt. Der Ansatz wird danach im Hochvakuum eingeengt, wobei die Temperatur bei etwa 20°C gehalten wird. Der Rückstand wird in 100 ml auf pH 3 eingestellte Salzsäure aufgenommen und nach 15-minütigem Verrühren bei RT mit 100 ml Dichlormethan ausgeschüttelt. Die wässrige Phase wird abgetrennt und anschließend nochmals mit 100 ml Ethylacetat ausgeschüttelt. Die wässrige Phase wird eingeengt und der Rückstand durch präparative HPLC gereinigt. Die entsprechenden Fraktionen werden eingeengt und der Rückstand aus Salzsäure (pH 3) lyophilisiert.
Ausbeute: 29 mg (16% d. Th.)
HPLC (Methode 2): Rₜ = 4.28 min;
LC-MS (Methode 6): Rₜ = 1.24 min; m/z = 549 (M+H)⁺.

### Beispiel 22

### N-[4-(Butylamino)butanoyl]-5-chlor-N-({(5S)-2-oxo-3-[4-(3-oxomorpholin-4-yl)phenyl]-1,3-oxazolidin-5-yl}methyl)thiophen-2-carboxamid-Hydrochlorid

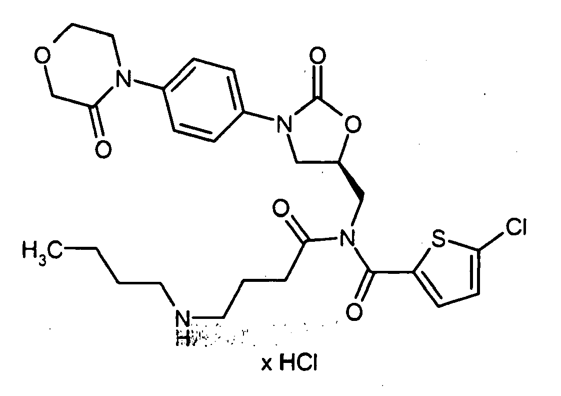

### Stufe a):

3.215 g (7.38 mmol) der Verbindung (A) werden in 60 ml DMF gelöst, mit 531 mg (22.1 mmol) Natriumhydrid versetzt, und die Mischung 30 min bei RT gerührt. Dann gibt man 6.9 g (22.1 mmol) des Intermediats 15A, gelöst in 10 ml DMF, hinzu. Man rührt weitere 10 min bei RT und versetzt den Ansatz dann mit 10 ml Wasser. Danach wird eingeengt und der Rückstand über Nacht mit 70 ml einer gesättigten Lösung von Chlorwasserstoff in Dichlornlethan verrührt, wobei der initial entstandene Enolester gespalten wird. Man filtriert und engt die verbleibende Lösung ein. Der Rückstand wird in 600 ml Ethylacetat aufgenommen und dreimal mit 100 ml einer 10%-igen Natriumcarbonat-Lösung sowie einmal mit Wasser ausgeschüttelt. Die Ethylacetat-Phase wird abgetrennt und eingeengt. Der Rückstand wird durch Flash-Chromatographie an Kieselgel mit Acetonitril/Dichlormethan 1:1 als Eluent gereinigt. Die entsprechenden Fraktionen werden eingeengt. Das verbleibende harzige Produkt wird in 20 ml Ethylacetat aufgenommen und mit 40 ml kaltem Diethylether versetzt. Man filtriert ab und wäscht den verbleibenden Rückstand mit Diethylether. Nach Trocknen im Hochvakuum erhält man 1.7 g (32.4% d. Th.) des Z-geschützten Intermediats.
HPLC (Methode 2): Rₜ = 6.0 min;
LC-MS (Methode 12): Rₜ = 3.9 min; m/z = 711 (M+H)⁺.

### Stufe b):

1.7 g (2.39 mmol) des geschützten Intermediats werden in 50 ml wasserfreier Trifluoressigsäure aufgenommen und 5 h lang mit Ultraschall behandelt. Der Ansatz wird danach im Hochvakuum eingeengt, wobei die Temperatur bei etwa 20°C gehalten wird. Der Rückstand wird in 200 ml auf pH 3 eingestellte Salzsäure aufgenommen und dreimal mit 25 ml Ethylacetat ausgeschüttelt. Die wässrige Phase wird lyophilisiert, anschließend nochmals in Salzsäure (pH 3) aufgenommen, filtriert und erneut lyophilisiert.
Ausbeute: 450 mg (31 % d. Th.)
HPLC (Methode 2): Rₜ = 4.57 min;
LC-MS (Methode 13): Rₜ = 2.81 min; m/z = 577 (M+H)⁺.

### Beispiel 23

### N-[(2-Aminoethoxy)acetyl]-5-chlor-N-({(5S)-2-oxo-3-[4-(3-oxomorphin-4-yl)phenyl]-1,3-oxazolidin-5-yl}methyl)thiophen-2-carboxamid-Hydrochlorid

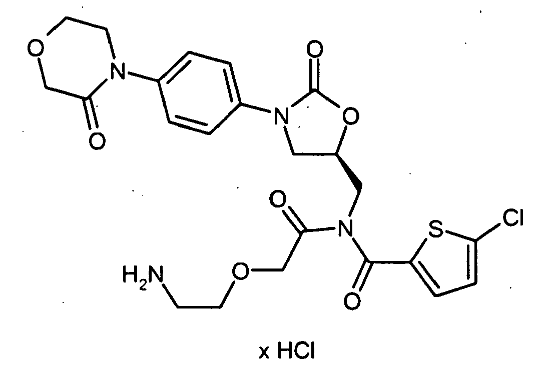

### Stufe a):

589.6 mg (1.35 mmol) der Verbindung (A) werden in 25 ml DMF gelöst, mit 97 mg (4 mmol) Natriumhydrid versetzt, und die Mischung 30 min bei RT gerührt. Dann gibt man 2.65 g (6.76 mmol) des Intermediats 16A, gelöst in 4 ml DMF, hinzu. Man rührt weitere 10 min bei RT und versetzt den Ansatz dann mit 5 ml Wasser. Danach wird eingeengt und der Rückstand über Nacht mit 150 ml einer gesättigten Lösung von Chlorwasserstoff in Dichlormethan verrührt. Anschließend wird wieder eingeengt und der Rückstand in 200 ml Ethylacetat aufgenommen. Man schüttelt zweimal mit je 50 ml einer 10%-igen Natriumcarbonat-Lösung aus. Die organische Phase wird abgetrennt, eingeengt und der Rückstand mit 30 ml Ethylacetat verrührt. Ungelöste Bestandteile werden abfiltriert und die organische Phase eingeengt. Der Rückstand wird durch Flash-Chromatographie an Kieselgel mit Acetonitril/Dichlormethan 1:1 als Eluent gereinigt. Die produkthaltigen Fraktionen werden eingeengt und der Rückstand erneut durch präparative HPLC (Methode 1) gereinigt. Die entsprechenden Fraktionen werden eingeengt und getrocknet. Man erhält 30 mg (3% d. Th.) des zweifach geschützten lntermediats.
HPLC (Methode 2): Rₜ = 5.71 min;
LC-MS (Methode 12): Rₜ = 3.74 min; m/z =791 (M+H)⁺.

### Stufe b):

30 mg (0.038 mmol) der geschützten Zwischenstufe werden in 50 ml wasserfreier Trifluoressigsäure über Nacht gerührt. Der Ansatz wird danach im Hochvakuum eingeengt, wobei die Temperatur bei etwa 20°C gehalten wird. Der Rückstand wird in 30 ml auf pH 3 eingestellte Salzsäure aufgenommen und die Mischung mit 20 ml Dichlormethan versetzt. Man trennt die Phasen und schüttelt die wässrige Phase erneut mit 20 ml Dichlormethan sowie anschließend mit 20 ml Ethylacetat aus. Die wässrige Phase wird abgetrennt, dann zur Entfernung restlichen Ethylacetats kurz im Hochvakuum andestilliert und schließlich lyophilisiert.
Ausbeute: 17 mg (78% d. Th.)
HPLC (Methode 2): Rₜ = 4.14 min;
LC-MS (Methode 12): Rₜ= 1.67 min; m/z =537 (M+H)⁺.

### B. Bestimmung von Löslichkeit, Stabilität und Freisetzungsverhalten

### a) Bestimmung der Löslichkeit:

Die Prüfsubstanz wird in Wasser oder verdünnter Salzsäure (pH 4) suspendiert. Diese Suspension wird 24 h bei Raumtemperatur geschüttelt. Nach Ultra-Zentrifugation bei 224000g für 30 min wird der Überstand mit DMSO verdünnt und per HPLC analysiert. Quantifiziert wird über eine ZweiPunkt-Kalibrationskurve der Testverbindung in DMSO.

### HPLC Methode:

Agilent 1100 mit DAD (G1315A), quat. Pumpe (G1311A), Autosampler CTC HTS PAL, Degaser (G1322A) und Säulenthermostat (G1316A); Säule: Zorbax Extend-C18 3.5 µ; Temperatur: 40°C; Eluent A: Wasser + 5 ml Perchlorsäure/Liter, Eluent B: Acetonitril; Flussrate: 0.7 ml/min; Gradient: 0-0.5 min 98% A, 2% B; Rampe 0.5-4.5 min 10% A, 90% B; 4.5-6 min 10% A, 90% B; Rampe 6.5-6.7 min 98% A, 2% B; 6.7-7.5 min 98% A, 2% B.

in Tabelle 1 sind die Loslichkertswerte repräsentativer Ausführungsbeispiele in verdünnter Salzsäure (pH 4) dargestellt:

**Tabelle 1**

| **Beispiel Nr.** | **Löslichkeit [mg/Liter]** |
|---|---|
| 1 | 320 |
| 2 | >500 |
| 3 | 340 |
| 4 | 480 |
| 5 | 330 |
| 6 | 860 |
| 7 | 340 |
| 10 | 680 |
| 11 | 960 |
| 12 | 350 |

Eine Zersetzung der Beispielverbindungen in diesen Lösungen wird nicht beobachtet.
Die Löslichkeit der zu Grunde liegenden Wirksubstanz [Verbindung (A)] in verdünnter Salzsäure (pH 4) wird in diesem Test mit 8.1 mg/Liter bestimmt.

In 5% Dextrose, die mit Salzsäure auf pH 4 eingestellt ist, wird für die Verbindung aus Beispiel 13 eine Löslichkeit von 2.70 g/Liter ermittelt; für die Verbindungen aus den Beispielen 20, 21 und 22 werden Löslichkeiten gemessen, die oberhalb von 3 g/Liter liegen.

### b) Stabilität in Puffer bei verschiedenen pH-Werten:

0.3 mg der Testsubstanz werden in einem 2 ml-HPLC-Vial eingewogen und mit 0.5 ml Acetonitril versetzt. Zum Lösen der Substanz wird das Probengefäß für ca. 10 Sekunden ins Ultraschallbad gegeben. Anschließend werden 0.5 ml der jeweiligen Pufferlösung zugefügt und die Probe erneut im Ultraschallbad behandelt.

Eingesetzte Pufferlösungen:
pH 4.0: 1 Liter Millipore-Wasser wird mit 1 N Salzsäure äuf pH 4.0 eingestellt;
pH 7.4: 90 g Natriumchlorid, 13.61 g Kaliumdihydrogenphosphat und 83.35 g 1 M Natronlauge werden auf 1 Liter mit Millipore-Wasser aufgefiillt und dann 1:10 verdünnt.

Über einen Zeitraum von 24 Stunden bei 37°C werden stündlich jeweils 5 µl der Probenlösung per HPLC auf ihren Gehalt an unveränderter Testsubstanz analysiert. Quantifiziert wird über die Flächenprozente der entsprechenden Peaks.

### HPLC-Methode:

Agilent 1100 mit DAD (G1314A), binärer Pumpe (G1312A), Autosampler (G1329A), Säulenofen (G1316A), Thermostat (G1330A); Säule: Kromasil 100 C18, 125 mm x 4.6 mm, 5 µm; Säulentemperatur: 30°C; Eluent A: Wasser + 5 ml Perchlorsäure/Liter, Eluent B: Acetonitril.

### Gradient 1:

0-1.0 min 98% A, 2% B → 1.0-13.0 min 50% A, 50% B→ 13.0-17.0 min 10% A, 90% B→17.0-18.0 min 10% A, 90% B → 18.0-19.5 98% A, 2% B→ 19.5-23.0 min 98% A, 2% B; Flussrate: 2.0 ml/min; UV-Detektion: 210 nm.

### Gradient 2:

0-3.0 min 78% A, 22% B → 3.0-15.0 min 78% A, 22% B → 15.0-17.0 min 10% A, 90% B→ 17.0-18.0 min 10% A, 90% B → 18.0-20.0 98% A, 2% B→ 20.0-23.0 min 98% A, 2% B; Flussrate: 2.0 ml/min; UV-Detektion: 210 nm.

### Gradient 3:

0-3.0 min 70% A, 30% B → 3.0-15.0 min 70% A, 30% B→ 15.0-17.0 min 10% A, 90% B→ 17.0-18.0 min 10% A, 90% B → 18.0-20.0 98% A, 2% B→ 20.0-23.0 min 98% A, 2% B; Flussrate: 2.0 ml/min; UV-Detektion: 210 nm.

In Tabelle 2 sind zu repräsentativen Ausführungsbeispielen die Verhältnisse der Peakflächen (F) zu den jeweiligen Zeitpunkten im Verhältnis zu den Peakflächen beim Startzeitpunkt dargestellt:

**Tabelle 2**

| **Beispiel Nr.** | **Puffer pH** | **% Testsubstanz nach 4 h** **[F(t=4h) x 100 / F(t=0h)]** | **% Testsubstanz nach 24 h** **[F(t=24h) x 100 / F(t=0h)]** |
|---|---|---|---|
| 1 | 4 | 100 | 99 |
| 1 | 7.4 | 49 | 3 |
| 2 | 4 | 100 | 100 |
| 2 | 7.4 | 96 | 80 |
| 3 | 4 | 100 | 99 |
| 3 | 7.4 | 88 | 48 |
| 4 | 4 | 100 | 100 |
| 4 | 7.4 | 31 | 0 |
| 5 | 4 | 100 | 94 |
| 5 | 7.4 | 68 | 14 |
| 6 | 4 | 100 | 98 |
| 6 | 7.4 | 90 | 59 |
| 7 | 4 | 100 | 99 |
| 7 | 7.4 | 48 | 3 |
| 8 | 4 | 99 | 81 |
| 8 | 7.4 | 2 | 0 |
| 10 | 4 | 100 | 99 |
| 10 | 7.4 | 8 | 0 |
| 11 | 4 | 100 | 99 |
| 11 | 7.4 | 10 | 0 |
| 12 | 4 | 100 | 98 |
| 12 | 7.4 | 0 | 0 |
| 13 | 4 | 99 | 91 |
| 14 | 4 | 57 | 0 |
| 14 | 7.4 | 0 | 0 |
| 17 | 4 | 83 | 55 |
| 18 | 4 | 100 | 99 |
| 19 | 4 | 100 | 97 |
| 19 | 7.4 | 0 | 0 |
| 20 | 4 | 98 | 90 |
| 20 | 7.4 | 0 | 0 |
| 21 | 4 | 100 | 100 |
| 21 | 7.4 | 92 | 66 |
| 22 | 4 | 100 | 98 |

In diesem Test wird bei pH 7.4 gleichzeitig mit der Abnahme des Gehalts an Testsubstanz eine Zunahme der Wirkstoff-Verbindung (A) festgestellt.

### c) In vitro-Stabilität in Rattenplasma (HPLC-Detektion):

1 mg Substanz wird in 1.25 ml Dimethylsulfoxid gelöst. Anschließend werden 1.25 ml Wasser zugegeben. 500 µl dieser Probenlösung werden mit 500 µl 37°C warmem Rattenplasma versetzt und geschüttelt. Es wird sofort eine erste Probe (10 µl) für die HPLC-Analyse gezogen. Im Zeitraum bis zu 2 h nach Inkubationsbeginn werden nach 2, 5, 10, 30, 60 und 90 min weitere Aliquote entnommen und der Gehalt der jeweiligen Prüfsubstanz und der daraus freigesetzten Wirkstoff-Verbindung (A) bestimmt.

### HPLC-Methode:

Agilent 1100 mit DAD (G1314A), binärer Pumpe (G1312A), Autosampler (G 1329A), Säulenofen (G1316A), Thermostat (G1330A); Säule: Kromasil 100 C18, 250 mm x 4.6 mm, 5 µm; Säulentemperatur: 30°C; ELuent A: Wasser + 5 ml Perchlorsäure/Liter, Eluent B: Acetonitril.

### Gradient:

0-0.5 min 98% A, 2% B → 0.5-3.0 min 73% A, 27% B → 3.0-18.0 min 73% A, 27% B → 18.0-20.0 min 10% A, 90% B → 20.0-21.0 90% A, 10% B → 21.0-22.5.0 min 98% A, 2% B → 22.5-25.0 min 98% A, 2% B; Flussrate: 2.0 ml/min; UV-Detektion:248 nm.

### Ergebnis:

Die Verbindungen aus den Beispielen 13, 17, 20, 22 und 23 werden in diesem Test mit einer Halbwertszeit von unter 2 min unter Freisetzung der Wirkstoff-Verbindung (A) abgebaut.

### d) In vitro-Stabilität in Ratten- und Humanplasma (LC/MS-MS-Detektion):

Ein definiertes Plasmavolumen (z.B. 2.0 ml) wird in einem geschlossenen Reagenzglas im Wasserbad auf 37°C erwärmt. Nach Erreichen der Soll-Temperatur wird eine definierte Menge der Prüfsubstanz als Lösung zugegeben (Volumen des Lösungsmittels max. 2% des Plasmavolumens). Das Plasma wird geschüttelt und eine erste Probe (50-100 µl) sofort entnommen. Im Zeitraum bis 2 h nach Inkubationsbeginn werden anschließend 4-6 weitere Aliquote entnommen.

Die Plasmaproben werden zur Proteinfällung mit Acetonitril versetzt. Nach Zentrifugation werden Prüfsubstanz und gegebenenfalls bekannte Spaltprodukte der Prüfsubstanz im Überstand mit einer geeigneten LC/MS-MS-Methode quantitativ bestimmt.

Stabilitätsbestimmungen in heparinisiertem Ratten- oder Humanblut werden wie für Plasma beschrieben durchgeführt.

In Tabelle 3 sind die zu verschiedenen Zeitpunkten bestimmten Konzentrationen c der Verbindung aus Beispiel 6 und der daraus freigesetzten Wirkstoff-Verbindung (A) im Plasma von Wistar-Ratten dargestellt:

**Tabelle 3**

| **Inkubationszeit** **[h]** | **c [mg/Liter]** **Beispiel 6** | **c [mg/Liter]** **Verbindung (A)** |
|---|---|---|
| 0 | 0.403 | 0.0789 |
| 0.08 | 0.0756 | 0.155 |
| 0.17 | 0.0079 | 0.175 |
| 0.5 | n.b. | 0.178 |
| 1 | *n.b.* | 0.186 |
| 2 | *n. b.* | 0.187 |

In Tabelle 4 sind die zu verschiedenen Zeitpunkten bestimmten Konzentrationen c der Verbindung aus Beispiel 13 und der daraus freigesetzten Wirkstoff-Verbindung (A) im Plasma von Wistar-Ratten dargestellt:

**Tabelle 4**

| **Inkubationszeit** **[h]** | **c [mg/Liter]** **Beispiel 13** | **c [mg/Liter]** **Verbindung (A)** |
|---|---|---|
| 0 | 0.5 | 0.178 |
| 0.08 | *n.b.* | 0.237 |
| 0.17 | *n.b.* | 0.247 |
| 0.5 | *n.b.* | 0.257 |
| 1 | *n. b.* | 0.275 |

| | | |
|---|---|---|
| *n.b.* = nicht bestimmbar (unterhalb der Detektionsgrenze). | | |

### e) i.v.-Pharmakokinetik in Wistar-Ratten:

Am Tag vor der Substanzgabe wird den Versuchstieren (männliche Wistar-Ratten, Körpergewicht 200-250 g) unter Isofluran^{®}-Narkose ein Katheter für die Blutgewinnung in die Vena Jugularis implantiert.

Am Versuchstag wird eine definierte Dosis der Prüfsubstanz als Lösung mit einer Hamilton^{®}-Glasspritze in die Schwanzvene appliziert (Bolusgabe, Applikationsdauer <10 s). Innerhalb von 24 h nach Substanzgabe werden sequentiell Blutproben (8-12 Zeitpunkte) über den Katheter entnommen. Zur Plasmagewinnung werden die Proben in heparinisierten Röhrchen zentrifugiert. Pro Zeitpunkt wird ein definiertes Plasmavolumen zur Proteinfällung mit Acetonitril versetzt. Nach Zentrifugation werden Prüfsubstanz und gegebenenfalls bekannte Spaltprodukte der Prüfsubstanz im Überstand mit einer geeigneten LC/MS-MS-Methode quantitativ bestimmt.

Die Berechnung pharmakokinetischer Kenngrößen der Prüfsubstanz bzw. der daraus freigesetzten Wirkstoff-Verbindung (A) wie AUC, Cₘₐₓ, T_{1/2} (Halbwertszeit) und CL (Clearance) erfolgt aus den gemessenen Plasmakonzentrationen.

### f) Hepatozytenassay zur Bestimmung der metabolischen Stabilität:

Die metabolische Stabilität der Testverbindungen gegenüber Hepatozyten wird bestimmt, indem die Verbindungen bei niedrigen Konzentrationen (bevorzugt unter 1 µM) und bei niedrigen Zellzahlen (bevorzugt bei 1 * 10⁶ Zellen/ml) inkubiert werden, um möglichst lineare kinetische Bedingungen im Versuch sicherzustellen. Sieben Proben aus der Inkubationslösung werden in einem festgelegten Zeitraster für die LC-MS-Analytik entnommen, um die Halbwertszeit (d.h. den Abbau) der Verbindung zu bestimmen. Aus dieser Halbwertszeit werden unterschiedliche "Clearance"-Parameter (CL) und "Fₘₐₓ"- Werte berechnet (s.u.).

Die CL- und Fₘₐₓ-Werte stellen ein Maß für den Phase 1- und Phase 2-Metabolismus der Verbindung in den Hepatozyten dar. Um den Einfluss des organischen Lösungsmittels auf die Enzyme in den Inkubationsansätzen möglichst klein zu halten, wird dessen Konzentration im Allgemeinen auf 1% (Acetonitril) bzw. 0.1% (DM SO) begrenzt.

Für alle Spezies und Rassen wird mit einer Hepatozyten-Zellzahl in der Leber von 1.1 * 10⁸ Zellen/g Leber gerechnet. CL-Parameter, deren Berechnung auf Halbwertszeiten beruhen, die über die Inkubationszeit hinausgehen (üblicherweise 90 Minuten), können nur als grobe Richtwerte angesehen werden.

Die berechneten Parameter und deren Bedeutung sind:
- **Fₘₐₓ well-stirred [%]**: maximal mögliche Bioverfügbarkeit nach oraler Applikation
- *Berechnung:*: (1-CL_{blood} well-stirred/QH) *100
- **CL_{blood} well-stirred [L/(h*kg)]**: berechnete Blut-Clearance (well stirred-Modell)
- *Berechnung:*: (QH * CL'_{intrinsic}) / (QH + CL'_{intrinsic})
- **CL'_{intrinsic} [ml/(min*kg)]**: maximale Fähigkeit der Leber (der Hepatozyten), eine Verbindung zu metabolisieren (unter der Annahme, dass der Leberblutfluss nicht geschwindigkeitslimitierend ist)
- *Berechnung*: CL'_{intrinsic.apparent} * speziesspezifische Hepatozytenzahl [1.1 * 10⁸/g Leber] * speziesspezifisches Lebergewicht [g/kg]
- **CL'_{intrinsic,apparent} [ml/(min*mg)]**: normiert die Eliminationskonstante, indem diese durch die eingesetzte Hepatozyten-Zellzahl x (x * 10⁶/ml) dividiert wird
- *Berechnung:*: kₑₗ [1/min] / (Zellzahl [x * 10⁶] / Inkubationsvolumen [ml])
(QH = speziesspezifischer Leberblutfluss).

### g) Bestimmung der antithrombotischen Wirkung in einem arteriovenösen Shunt-Modell in Ratten:

Nüchterne männliche Ratten (Stamm: HSD CPB:WU) werden durch intraperitoneale Gabe einer Rompun/Ketavet-Lösung narkotisiert (12 mg/kg / 50 mg/kg). Die Thrombusbildung wird in einem arteriovenösen Shunt in Anlehnung an die von P.C. Wong *et al.* beschriebene Methode *[*Thrombosis Research 83 (2), 117-126 (1996)] ausgelöst. Dazu werden die linke Vena jugularis und die rechte Arteria carotis freipräpariert. In die Arterie wird ein 8 cm langer Polyethylenkatheter (PE60, Fa. Becton-Dickinson), gefolgt von einem 6 cm langen Tygonschlauch (R-3606, ID 3.2 mm, Fa. Kronlab), der zur Erzeugung einer thrombogenen Oberfläche einen aufgerauhten und zu einer Doppelschlinge gelegten Nylonfaden (60 x 0.26 mm, Fa. Berkley Trilene) enthält, eingebunden. In die Vena jugularis wird ein 2 cm langer Polyethylenkatheter (PE60, Fa. Becton-Dickinson) eingebunden und über einen 6 cm langen Polyethylenkatheter (PE160, Fa. Becton-Dickinson) mit dem Tygonschlauch verbunden. Die Schläuche werden mit physiologischer Kochsalzlösung vor Öffnung des Shuntes gefüllt. Der extrakorporale Kreislauf wird 15 min lang aufrechterhalten. Dann wird der Shunt entfernt und der Nylonfaden mit dem Thrombus sofort gewogen. Das Leergewicht des Nylonfadens ist vor Versuchsbeginn ermittelt worden. Die Prüfsubstanz (als Lösung in physiologischer Kochsalzlösung, die mit 0.1 N Salzsäure auf pH 4 eingestellt ist) wird vor Anlegung des extrakorporalen Kreislaufs als Bolus-Injektion verabreicht.

### C. Ausführungsbeispiele für pharmazeutische Zusammensetzungen

Die erfindungsgemäßen Verbindungen können beispielsweise folgendermaßen in pharmazeutische Zubereitungen überführt werden:

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%, die jeweils auf einen pH-Wert von 3-5 eingestellt sind) gelöst. Die Lösung wird gegebenenfalls steril filtriert und/oder in sterile und pyrogenfreie Injektionsbehältnisse abgefüllt.

## Patentansprüche

1. Verbindung der Formel (I) 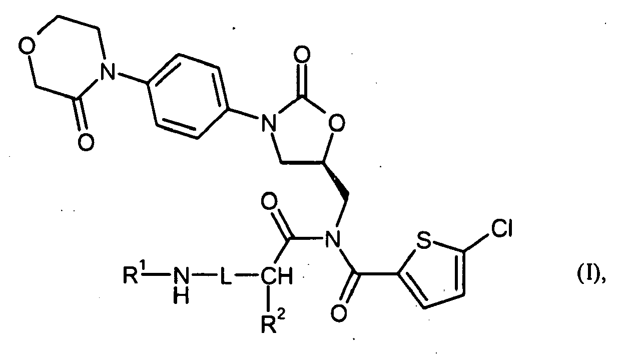 in welcher
R¹ für Wasserstoff oder (C₁-C₄)-Alkyl, das mit Hydroxy oder (C₁-C₄)-Alkoxy substituiert sein kann steht,
R² für Wasserstoff oder (C₁-C₄)-Alkyl steht
und
L für eine (C₁-C₄)-Alkandiyl-Gruppe, in welcher eine CH₂-Gruppe gegen ein O-Atom ausgetauscht sein kann, oder für eine Gruppe der Formel 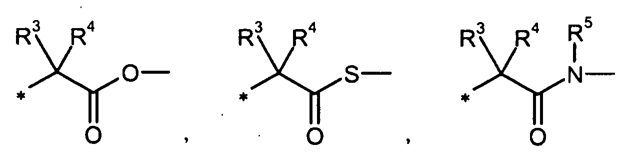 oder 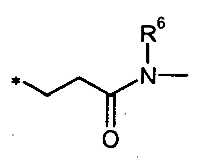 steht, worin
* die Verknüpfungsstelle mit dem N-Atom bedeutet,
R³ die Seitengruppe einer natürlichen α-Aminosäure oder ihrer Homologe oder Isomere bedeutet
oder
R³ mit R¹ verknüpft ist und beide zusammen eine (CH₂)₃- oder (CH₂)₄-Gruppe bilden,
R⁴ Wasserstoff oder Methyl bedeutet,
R⁵ (C₁-C₄)-Alkyl bedeutet
und
R⁶ Wasserstoff oder (C₁-C₄)-Alkyl bedeutet,
sowie ihre Salze, Solvate und Solvate der Salze.

2. Verbindung der Formel (I) nach Anspruch 1, in welcher
R¹ für Wasserstoff oder (C₁-C₄)-Alkyl steht,
R² für Wasserstoff steht
und
L für eine (C₂-C₄)-Alkandiyl-Gruppe oder für eine Gruppe der Formel 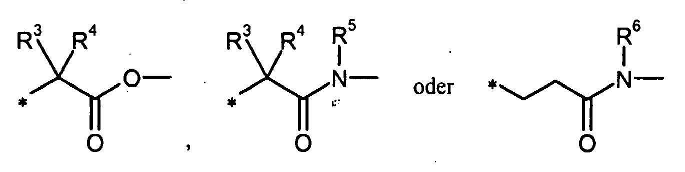 steht, worin
* die Verknüpfungsstelle mit dem N-Atom bedeutet,
R³ Wasserstoff, Methyl, Propan-2-yl, Propan-1-yl, Imidazol-4-ylmethyl, Hydroxymethyl, 1-Hydroxyethyl, Carbamoylmethyl, 2-Carbamoylethyl, 4-Aminobutan-1-yl, 3-Aminopropan-1-yl oder 3-Guanidinopropan-1-yl bedeutet
oder
R³ mit R¹ verknüpft ist und beide zusammen eine (CH₂)₃- oder (CH₂)₄-Gruppe bilden,
R⁴ Wasserstoff oder Methyl bedeutet,
R⁵ Methyl bedeutet
und
R⁶ Wasserstoff oder Methyl bedeutet,
sowie ihre Salze, Solvate und Solvate der Salze.

3. Verbindung der Formel (I) nach Anspruch 1 oder 2, in welcher
R¹ für Wasserstoff, Methyl oder n-Butyl steht,
R² für Wasserstoff steht
und L für eine CH₂CH₂-Gruppe oder für eine Gruppe der Formel 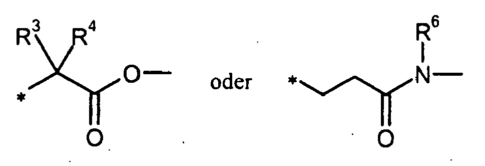 steht, worin
* die Verknüpfungsstelle mit dem N-Atom bedeutet,
R³ Wasserstoff, Methyl, Propan-2-yl, Propan-1-yl, Imidazol-4-ylmethyl, Hydroxymethyl, 1-Hydroxyethyl, Carbamoylmethyl, 2-Carbamoylethyl, 4-Aminobulan-1-yl, 3-Aminopropan-1-yl oder 3-Guanidinopropan-1-yl bedeutet
oder
R³ mit R¹ verknüpft ist und beide zusammen eine (CH₂)₃- oder (CH₂)₄-Gruppe bilden,
R⁴ Wasserstoff oder Methyl bedeutet
und
R⁶ Wasserstoff oder Methyl bedeutet,
sowie ihre Salze, Solvate und Solvate der Salze.

4. Verfahren zur Herstellung von Verbindungen der Formel (I), wie in den Ansprüchen 1 bis 3 definiert, **dadurch gekennzeichnet, dass** man entweder
[A] die Verbindung (A) 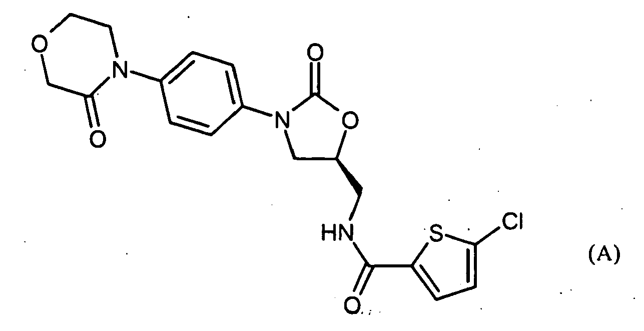 zunächst in einem inerten Lösungsmittel in Gegenwart einer Base mit einer Verbindung der Formel (II) 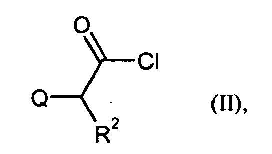 in welcher R² die in den Ansprüchen 1 bis 3 angegebene Bedeutung hat
und
Q für eine Abgangsgruppe wie beispielsweise Chlor, Brom oder Iod steht,
in eine Verbindung der Formel (III) 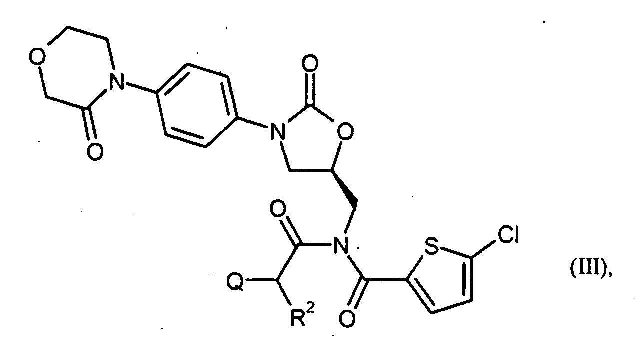 in welcher Q und R² die oben angegebenen Bedeutungen haben,
überführt, diese dann in einem inerten Lösungsmittel mit dem Caesiumsalz einer α-Aminocarbonsäure oder α-Aminothiocarbonsäure der Formel (IV) 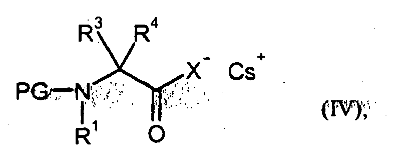 in welcher R¹, R³ und R⁴ jeweils die in den Ansprüchen 1 bis 3 angegebenen Bedeutungen haben,
PG für eine Amino-Schutzgruppe wie beispielsweise *tert*.-Butoxycarbonyl (Boc) oder Benzyloxycarbonyl (Z) steht
und
X für O oder S steht,
zu einer Verbindung der Formel (V) 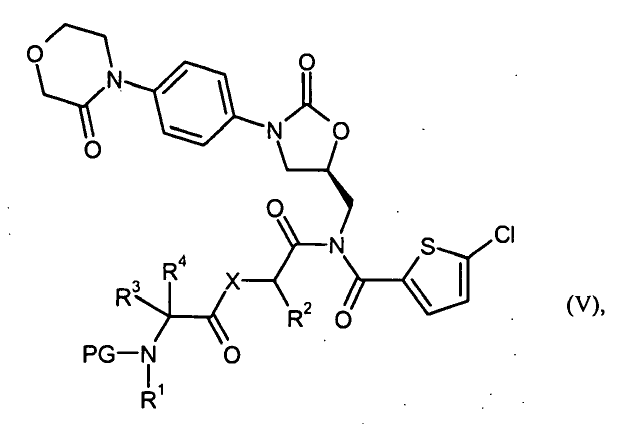
in welcher R', R², R³, R⁴, PG und X jeweils die oben angegebenen Bedeutungen haben,
umsetzt und nachfolgend die Schutzgruppe PG unter Erhalt einer Verbindung der Formel (I-A) 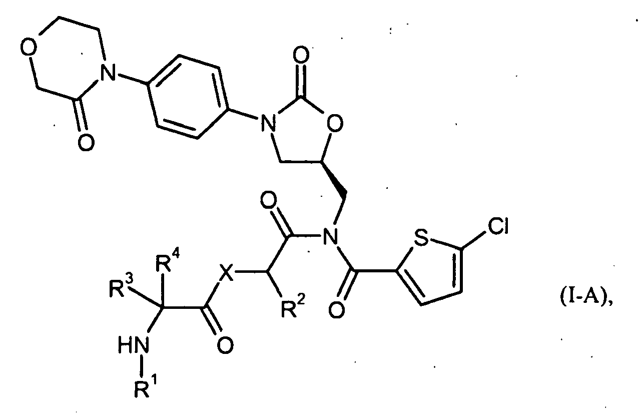 in welcher R¹, R², R³, R⁴ und X jeweils die oben angegebenen Bedeutungen haben, entfernt
oder
[B] Verbindung (A) in einem inerten Lösungsmittel in Gegenwart einer Base mit einer Verbindung der Formel (VI) 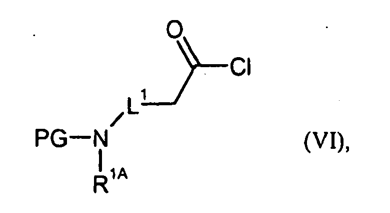 in welcher PG die oben angegebene Bedeutung hat,
R^{1A} für (C₁-C₄)-Alkyl, das mit Hydroxy oder (C₁-C₄)-Alkoxy substituiert sein kann, steht
und
L¹ für eine (C₁-C₄)-Alkandiyl-Gruppe, in welcher eine CH₂-Gruppe gegen ein O-Atom ausgetauscht sein kann, steht,
zu einer Verbindung der Formel (VII) 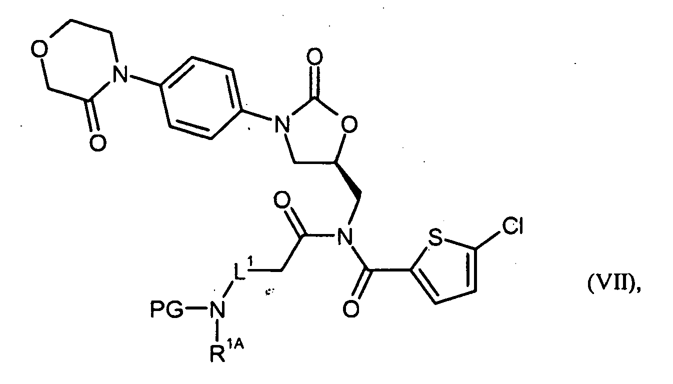 in welcher R^{1A}, L¹ und PG jeweils die oben angegebenen Bedeutungen haben, umsetzt und anschließend die Schutzgruppe PG unter Erhalt einer Verbindung der Formel (I-B) 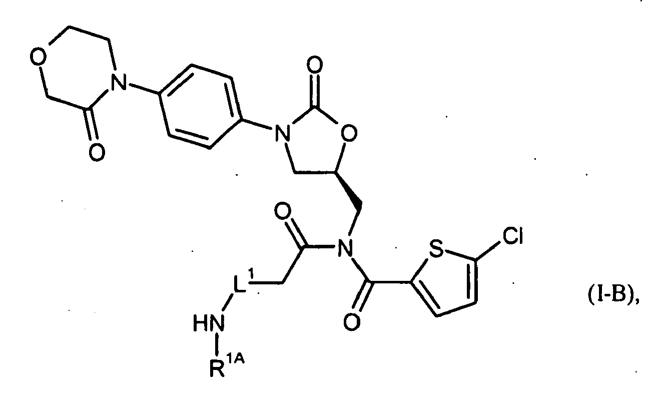 in welcher R^{1A} und L¹ die oben angegebenen Bedeutungen haben,
entfernt
oder
[C] die Verbindung (B) 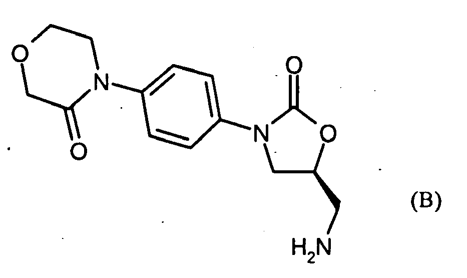 zunächst in eine Verbindung der Formel (VIII) 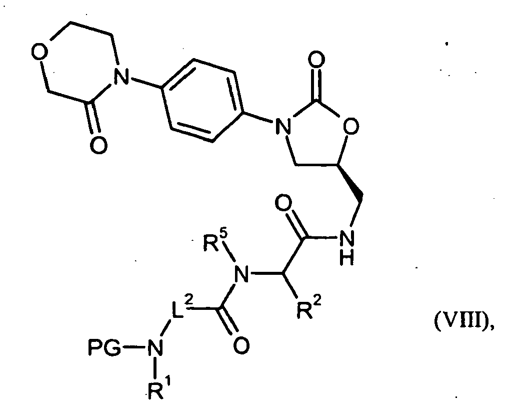 in welcher PG, R¹, R² und R⁵ jeweils die in den Ansprüchen 1 bis 3 angegebenen Bedeutungen haben
und
L² für eine (CH₂)₂- oder CR³R⁴-Gruppe steht, worin R³ und R⁴ jeweils die in den Ansprüchen 1 bis 3 angegebenen Bedeutungen haben,
überführt, diese dann in einem inerten Lösungsmittel in Gegenwart einer Base mit einer Verbindung der Formel (IX) 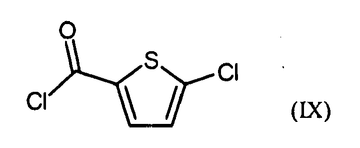 zu einer Verbindung der Formel (X) 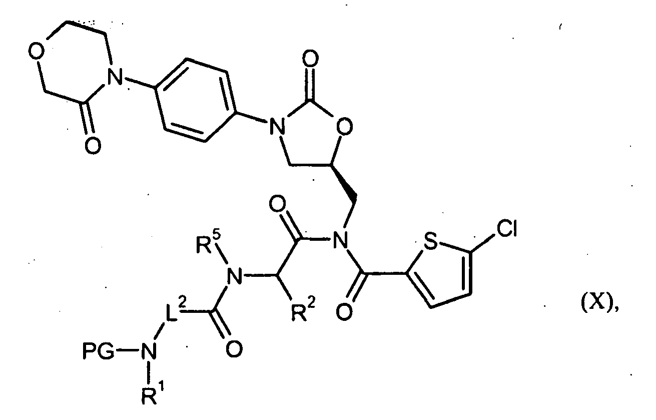 in welcher PG, L², R¹, R² und R⁵ jeweils die oben angegebenen Bedeutungen haben,
umsetzt und nachfolgend die Schutzgruppe PG unter Erhalt einer Verbindung der Formel (I-C) 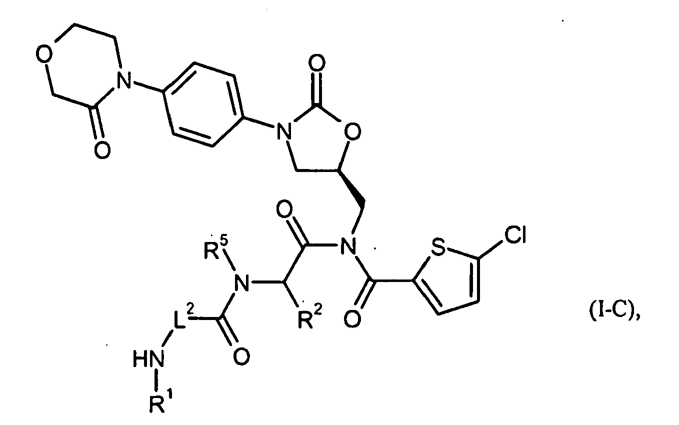 in welcher L², R', R² und R⁵ jeweils die oben angegebenen Bedeutungen haben, entfernt
oder
[D] Verbindung (A) in einem inerten Lösungsmittel in Gegenwart einer Base mit einer Verbindung der Formel (XI) 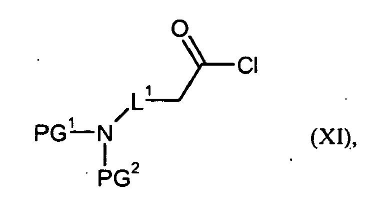
in welcher
L¹ für eine (C₁-C₄)-Alkandiyl-Gruppe, in welcher eine CH₂-Gruppe gegen ein O-Atom ausgetauscht sein kann, steht
und
PG¹ und PG² unabhängig voneinander für eine Amino-Schutzgruppe wie beispielsweise *tert*.-Butoxycarbonyl (Boc), Benzyloxycarbonyl (Z) oder p-Methoxybenzyl (PMB) stehen und gleich oder verschieden sein können,
zu einer Verbindung der Formel (XII) 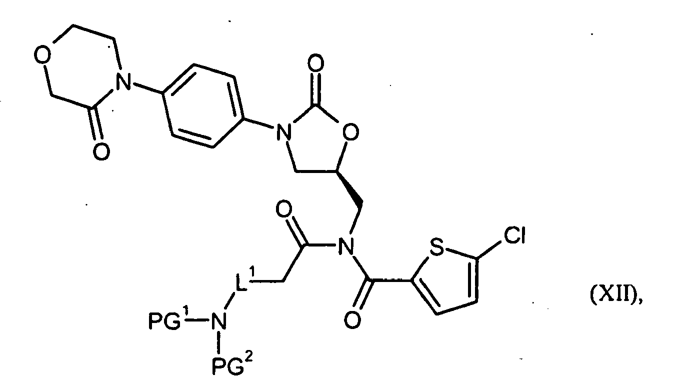 in welcher L¹, PG¹ und PG² jeweils die oben angegebenen Bedeutungen haben, umsetzt und anschließend die Schutzgruppen PG¹ und PG², gleichzeitig oder sequentiell, unter Erhalt einer Verbindung der Formel (I-D) 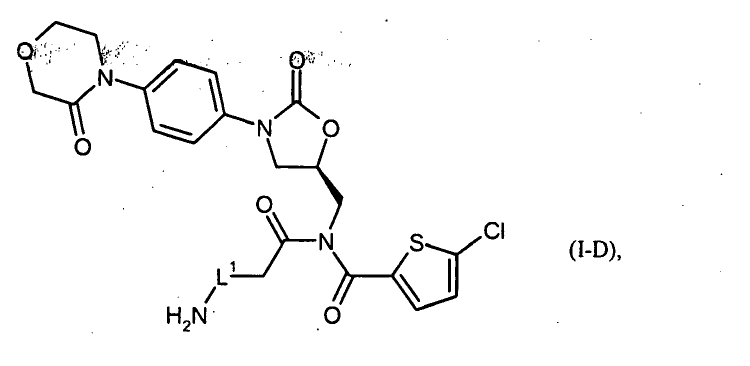 in welcher L¹ die oben angegebene Bedeutung hat,
entfernt
und die jeweils resultierenden Verbindungen der Formel (I-A), (I-B), (I-C) bzw. (I-D) gegebenenfalls mit den entsprechenden (i) Lösungsmitteln und/oder (ii) Säuren in ihre Solvate, Salze und/oder Solvate der Salze überführt.

5. Verbindung der Formel (I), wie in einem der Ansprüchen 1 bis 3 definiert, zur Behandlung und/oder Prophylaxe von Krankheiten.

6. Verwendung einer Verbindung der Formel (I), wie in einem der Ansprüche 1 bis 3 definiert, zur Herstellung eines Arzneimittels zur Behandlung und/oder Prophylaxe von thromboembolischen Erkrankungen.

7. Arzneimittel enthaltend eine Verbindung der Formel (I), wie in einem der Ansprüche 1 bis 3 definiert, gegebenenfalls in Kombination mit einem inerten, nicht-toxischen, pharmazeutisch geeigneten Hilfsstoff.

8. Arzneimittel enthaltend eine Verbindung der Formel (I), wie in einem der Ansprüche 1 bis 3 definiert, in Kombination mit einem weiteren Wirkstoff.

9. Arzneimittel nach Anspruch 7 oder 8 zur Behandlung und/oder Prophylaxe von thromboembolischen Erkrankungen.

10. Arzneimittel nach einem der Ansprüche 7 bis 9 zur intravenösen Anwendung.

11. Verwendung mindestens einer Verbindung der Formel (I), wie in einem der Ansprüche 1 bis 3 definiert, oder eines Arzneimittels, wie in einem der Ansprüche 7 bis 10 definiert zur Herstellung eines Arzneimittels zur Behandlung und/oder Prophylaxe von thromboembolischen Erkrankungen bei Menschen und Tieren.

## Claims

1. Compound of the formula (I) 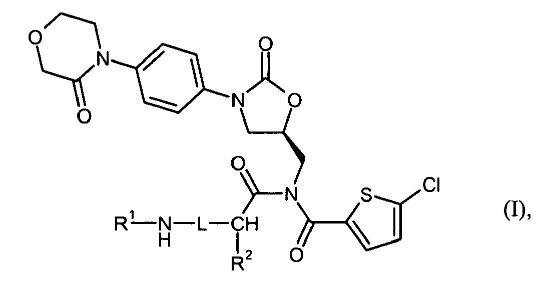 in which
R¹ is hydrogen or (C₁-C₄)-alkyl which may be substituted by hydroxy or (C₁-C₄)-alkoxy,
R² is hydrogen or (C₁-C₄)-alkyl,
and
L is a (C₁-C₄)-alkanediyl group in which one CH₂ group may be replaced by an O atom, or is a group of the formula 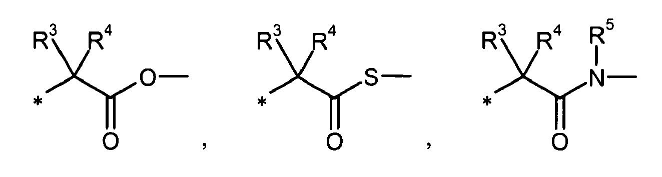 or 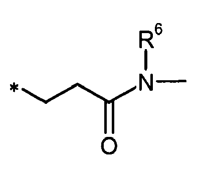 in which
* means the point of linkage to the N atom,
R³ is the side group of a natural α-amino acid or its homologues or isomers,
or
R³ is linked to R¹ and the two together form a (CH₂)₃ or (CH₂)₄ group,
R⁴ is hydrogen or methyl,
R⁵ is (C₁-C₄)-alkyl,
and
R⁶ is hydrogen or (C₁-C₄)-alkyl,
and the salts, solvates and solvates of the salts thereof.

2. Compound of the formula (I) according to Claim 1, in which
R¹ is hydrogen or (C₁-C₄)-alkyl,
R² is hydrogen,
and
L is a (C₂-C₄)-alkanediyl group or is a group of the formula 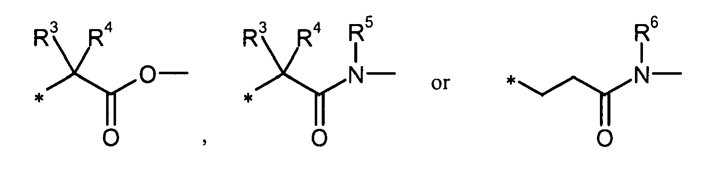 in which
* means the point of linkage to the N atom,
R³ is hydrogen, methyl, propan-2-yl, propan-1-yl, imidazol-4-yl-methyl, hydroxymethyl, 1-hydroxyethyl, carbamoylmethyl, 2-carbamoylethyl, 4-aminobutan-1-yl, 3-aminopropan-1-yl or 3-guanidinopropan-1-yl,
or
R³ is linked to R¹ and the two together form a (CH₂)₃ or (CH₂)₄ group,
R⁴ is hydrogen or methyl,
R⁵ is methyl,
and
R⁶ is hydrogen or methyl,
and the salts, solvates and solvates of the salts thereof.

3. Compound of the formula (I) according to Claim 1 or 2, in which
R¹ is hydrogen, methyl or n-butyl,
R² is hydrogen,
and
L is a CH₂CH₂ group or is a group of the formula 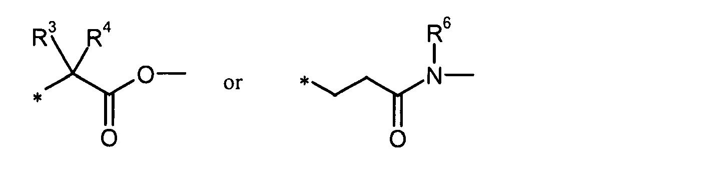 in which
* means the point of linkage to the N atom,
R³ is hydrogen, methyl, propan-2-yl, propan-1-yl, imidazol-4-yl-methyl, hydroxymethyl, 1-hydroxyethyl, carbamoylmethyl, 2-carbamoylethyl, 4-aminobutan-1-yl, 3-aminopropan-1-yl or 3-guanidinopropan-1-yl,
or
R³ is linked to R¹ and the two together form a (CH₂)₃ or (CH₂)₄ group,
R⁴ is hydrogen or methyl,
and
R⁶ is hydrogen or methyl,
and the salts, solvates and solvates of the salts thereof.

4. Process for preparing compounds of the formula (I) as defined in Claims 1 to 3, **characterized in that** either
[A] the compound (A) 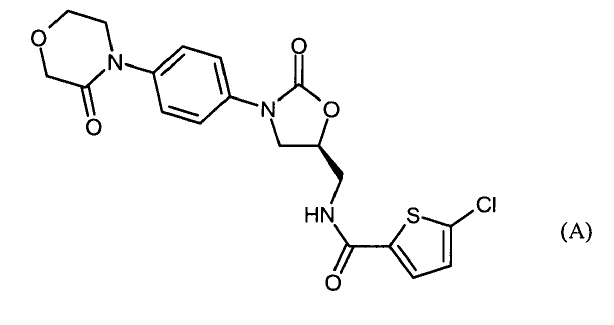 is initially converted in an inert solvent in the presence of a base with a compound of the formula (II) 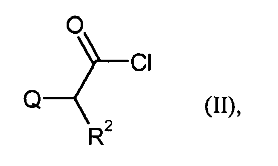 in which R² has the meaning indicated in Claims 1 to 3,
and
Q is a leaving group such as, for example, chlorine, bromine or iodine,
into a compound of the formula (III) 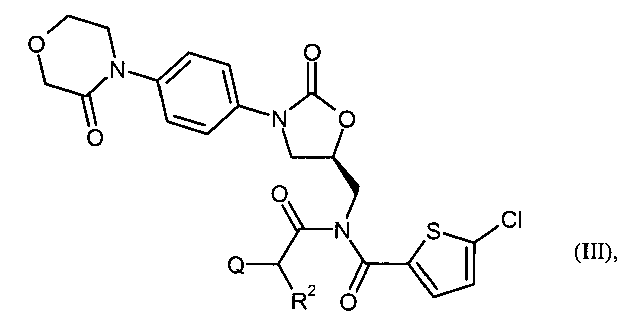 in which Q and R² have the meanings indicated above,
the latter is then reacted in an inert solvent with the caesium salt of an α-amino carboxylic acid or α-amino thiocarboxylic acid of the formula (IV) 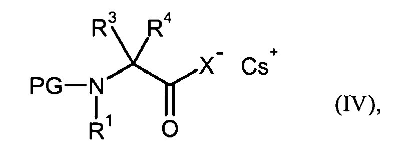 in which R¹, R³ and R⁴ each have the meanings indicated in Claims 1 to 3, PG is an amino protective group such as, for example, *tert-*butoxycarbonyl (Boc) or benzyloxycarbonyl (Z),
and
X is O or S,
to give a compound of the formula (V) 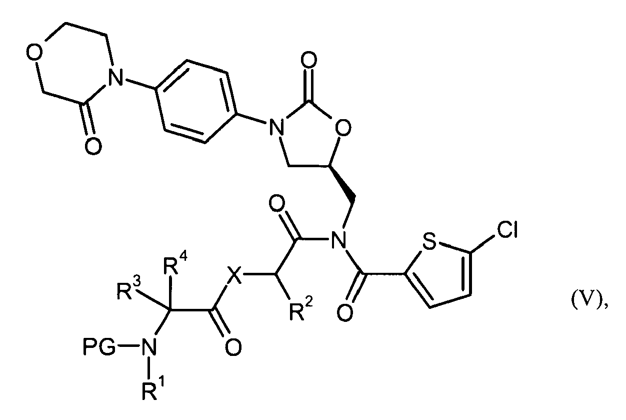 in which R¹, R², R³, R⁴, PG and X each have the meanings indicated above,
and subsequently the protective group PG is removed to result in a compound of the formula (I-A) 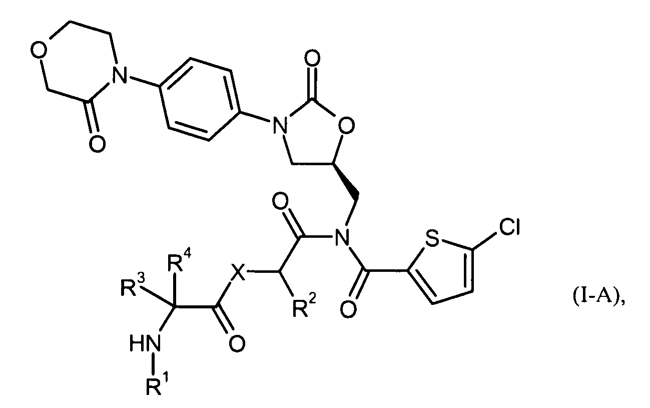 in which R¹, R², R³, R⁴ and X each have the meanings indicated above,
or
[B] compound (A) is reacted in an inert solvent in the presence of a base with a compound of the formula (VI) 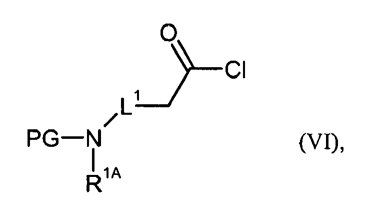 in which PG has the meaning indicated above,
R^{1A} is (C₁-C₄)-alkyl which may be substituted by hydroxy or (C₁-C₄)-alkoxy,
and
L¹ is a (C₁-C₄)-alkanediyl group in which one CH₂ group may be replaced by an O atom,
to give a compound of the formula (VII) 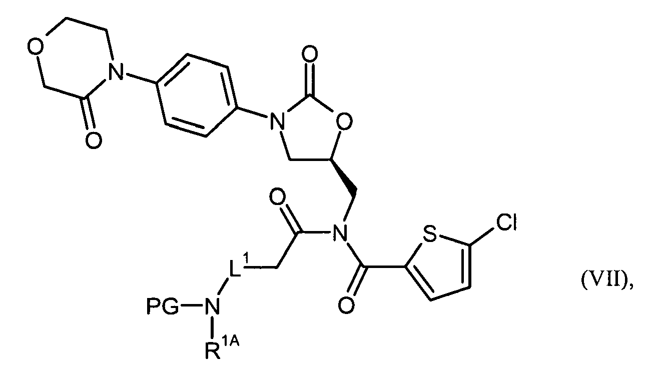 in which R^{1A}, L¹ and PG each have the meanings indicated above,
and subsequently the protective group PG is removed to result in a compound of the formula (I-B) 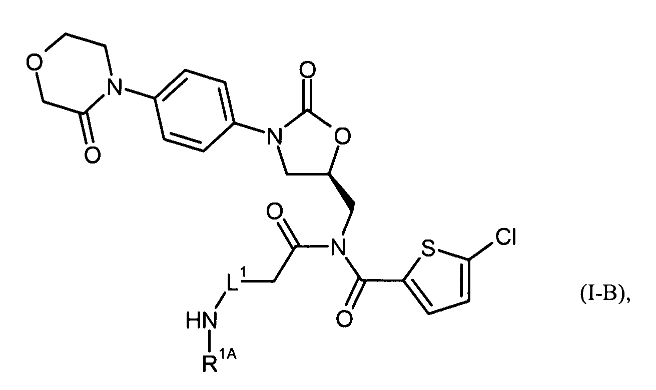 in which R^{1A} and L¹ have the meanings indicated above,
or
[C] the compound (B) 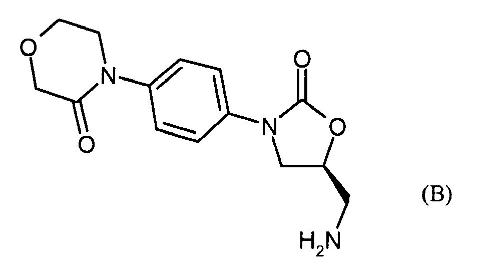 is initially converted into a compound of the formula (VIII) 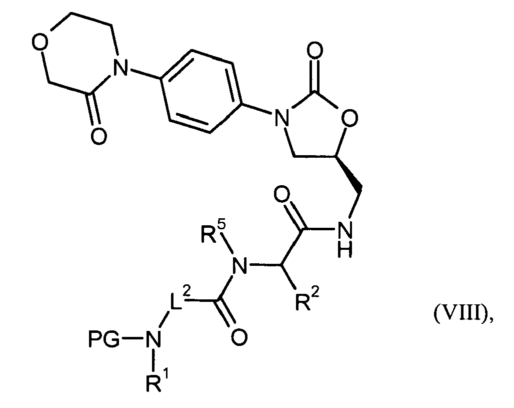 in which PG, R¹, R² and R⁵ each have the meanings indicated in Claims 1 to 3,
and
L² is a (CH₂)₂ or CR³R⁴ group in which R³ and R⁴ each have the meanings indicated in Claims 1 to 3,
the latter is then reacted in an inert solvent in the presence of a base with a compound of the formula (IX) 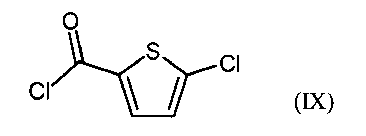 to give a compound of the formula (X) 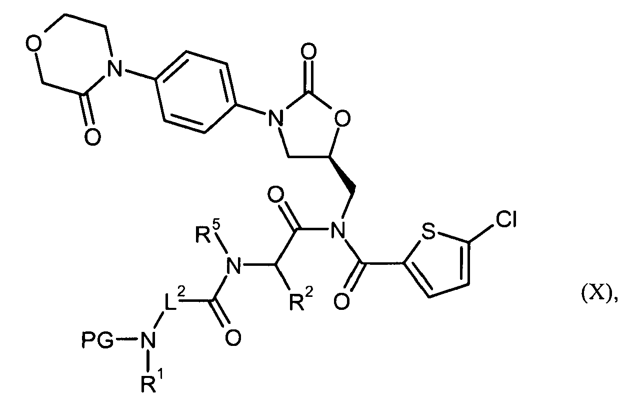 in which PG, L², R¹, R² and R⁵ each have the meanings indicated above, and subsequently the protective group PG is removed to result in a compound of the formula (I-C) 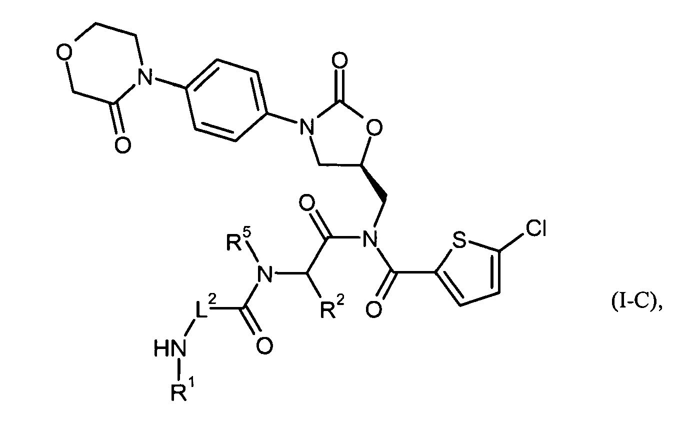 in which L², R¹, R² and R⁵ each have the meanings indicated above,
or
[D] compound (A) is reacted in an inert solvent in the presence of a base with a compound of the formula (XI) 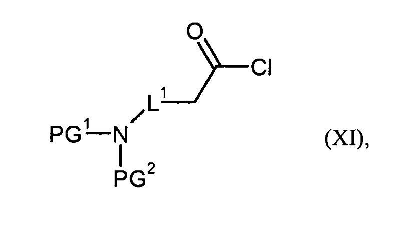 in which
L¹ is a (C₁-C₄)-alkanediyl group in which one CH₂ group may be replaced by an O atom,
and
PG¹ and PG² are independently of one another an amino protective group such as, for example, tert-butoxycarbonyl (Boc), benzyloxycarbonyl (Z) or p-methoxybenzyl (PMB) and may be identical or different,
to give a compound of the formula (XII) 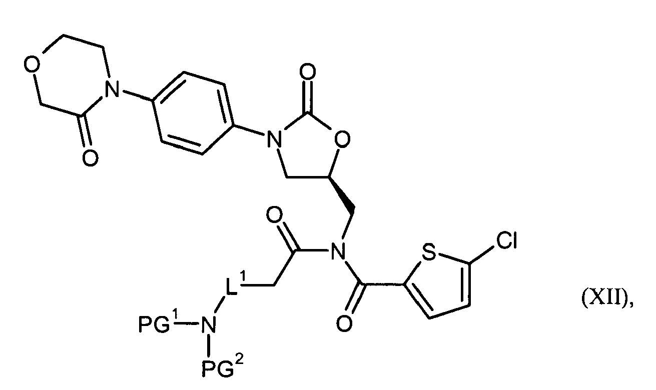
in which L¹, PG¹ and PG² each have the meanings indicated above,
and subsequently the protective groups PG¹ and PG² are removed, simultaneously or sequentially, to result in a compound of the formula (I-D) 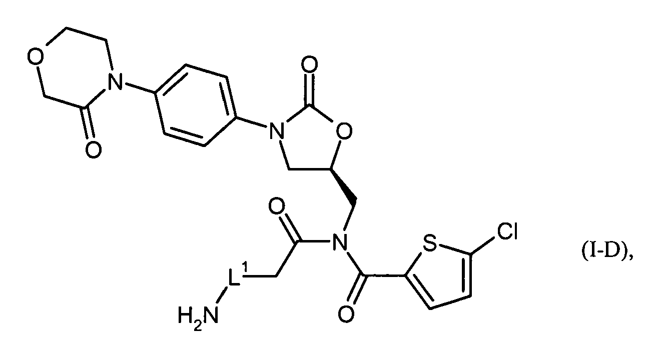 in which L¹ has the meaning indicated above,
and the compounds of the formula (I-A), (I-B), (I-C) and (I-D) resulting in each case are converted where appropriate with the appropriate (i) solvents and/or (ii) acids into the solvates, salts and/or solvates of the salts thereof.

5. Compound of the formula (I) as defined in any of Claims 1 to 3 for the treatment and/or prophylaxis of diseases.

6. Use of a compound of the formula (I) as defined in any of Claims 1 to 3 for the manufacture of a medicament for the treatment and/or prophylaxis of thromboembolic disorders.

7. Medicament comprising a compound of the formula (I) as defined in any of Claims 1 to 3, where appropriate in combination with an inert, non-toxic, pharmaceutically suitable excipient.

8. Medicament comprising a compound of the formula (I) as defined in any of Claims 1 to 3 in combination with a further active ingredient.

9. Medicament according to Claim 7 or 8 for the treatment and/or prophylaxis of thromboembolic disorders.

10. Medicament according to any of Claims 7 to 9 for intravenous use.

11. Use of at least one compound of the formula (I) as defined in any of Claims 1 to 3, or a medicament as defined in any of Claims 7 to 10 for preparing a medicament for the treatment and/or prophylaxis of thromboembolic disorders in humans and animals.

## Revendications

1. Composé de formule (I) 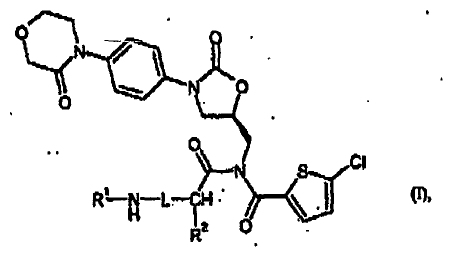 dans laquelle
R¹ désigne l'hydrogène ou un alkyle (en C₁-C₄), qui peut être substitué par un hydroxy ou un alcoxy (en C₁-C₄),
R² représente l'hydrogène ou un alkyle (en C₁-C₄)
et
L est un groupe alcanediyle (en C₁-C₄), dans lequel un groupe CH₂ peut être échangé contre un atome d'O, ou un groupe de la formule 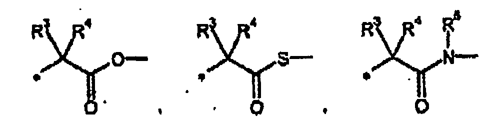 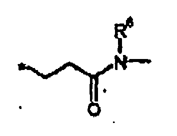 dans laquelle
* désigne le point de liaison avec l'atome de N,
R³ représente le groupe latéral d'un acide alpha-aminé naturel ou d'homologues ou d'isomères de celui-ci
ou
R³ est lié avec R¹ et ils forment tous deux ensemble un groupe (CH₂)₃ ou (CH₂)₄, R⁴ correspond à l'hydrogène ou à un méthyle,
R⁵ désigne un alkyle (en C₁-C₄),
et
R⁶ est l'hydrogène ou un alkyle (en C₁-C₄),
ainsi que leurs sels, solvates et solvates de sels.

2. Composé de formule (I) selon la revendication 1, dans laquelle
R¹ désigne l'hydrogène ou un alkyle (en C₁-C₄),
R² représente l'hydrogène
et
L est un groupe alcanediyle (en C₂-C₄) ou groupe de formule 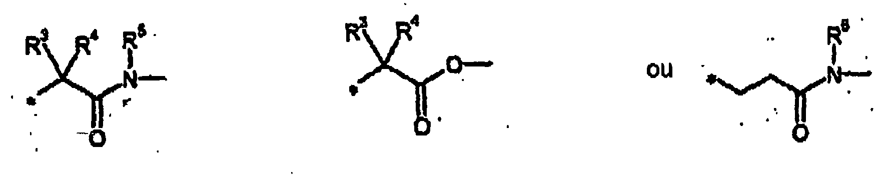 dans laquelle
* désigne le point de liaison avec l'atome de N,
R³ représente l'hydrogène, un groupe méthyle, propane-2yle, propane-1-yle, imidazol-4-ylméthyle, hydroxyméthyle, 1-hydroxyéthyle, carbamoylméthyle, 2-carbamoyléthyle, 4-aminobutane-1-yle, 3-aminopropane-1-yle ou 3-guanidinopropane-1-yle
ou
R³ est lié avec R¹ et ils forment tous deux ensemble un groupe (CH₂)₃ ou (CH₂)₄,
R⁴ correspond à l'hydrogène ou à un méthyle,
R⁵ désigne un méthyle,
et
R⁸ est l'hydrogène ou un méthyle,
ainsi que leurs sels, solvates et solvates de sels.

3. Composé de formule (I) selon la revendication 1 ou 2, dans laquelle
R¹ désigne l'hydrogène, un méthyle ou un n-butyle,
R² représente l'hydrogène
et
L est un groupe CH₂CH₂ ou un groupe de formule 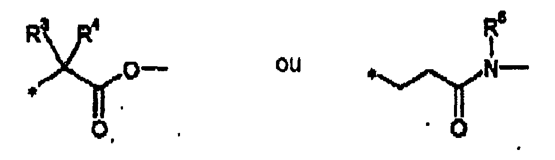 dans laquelle
* * désigne le point de liaison avec l'atome de N,
R³ représente l'hydrogène, un groupe méthyle, propane-2-yle, propane-1-yle, imidazol-4-ylméthyle, hydroxyméthyle, 1-hydroxyéthyle, carbamoylméthyle, 2-carbamoyléthyle, 4-aminobutane-1-yle, 3-aminopropane-1-yle ou 3-guanidinopropane-1-yle
ou
R³ est lié avec R¹ et ils forment tous deux ensemble un groupe (CH₂)₃ ou (CH₂)₄,
R⁴ correspond à l'hydrogène ou à un méthyle,
et
R⁸ est l'hydrogène ou un méthyle,
ainsi que leurs sels, solvates et solvates de sels

4. Procédé de production de composés de formule (I), tels qu'ils sont définis dans les revendications 1 à 3, **caractérisé en ce que**, soit,
[A] le composé (A) 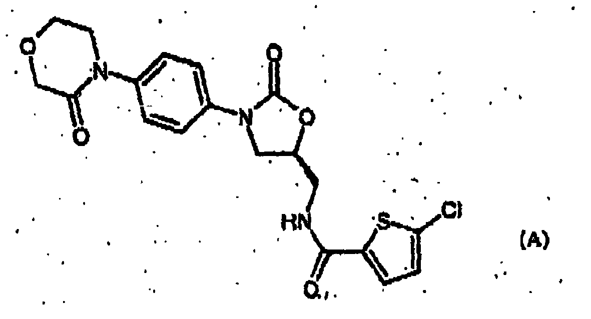 est tout d'abord transformé dans un solvant inerte en présence d'une base avec un composé de formule (II) 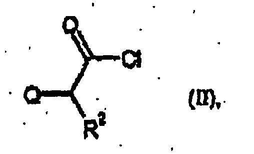 dans laquelle R² possède la signification indiquée dans les revendications 1 à 3
et
Q représente un groupe de départ, tel qu'un chloro, un bromo
ou un iodo,
en un groupe de formule (III) 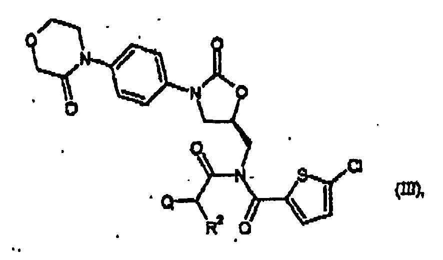 dans laquelle Q et R² possèdent la signification mentionnée ci-dessus, lequel composé est ensuite mis à réagir dans un solvant inerte avec le sel de césium d'un acide alpha-aminé ou d'un acide alpha-aminothiocarboxylique de formule (IV), 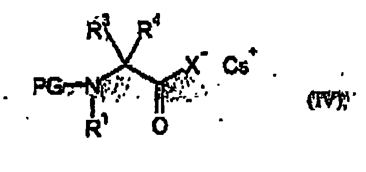 dans laquelle R¹, R³ et R⁴ possèdent chacun la signification indiquée dans les revendications 1 à 3,
PG est un groupe protecteur de la fonction amine, tel qu'un tert.-butoxycarbonyle (Boc) ou un benzyloxycarbonyle (Z),
et
X désigne O ou S,
pour donner un composé de formule (V) 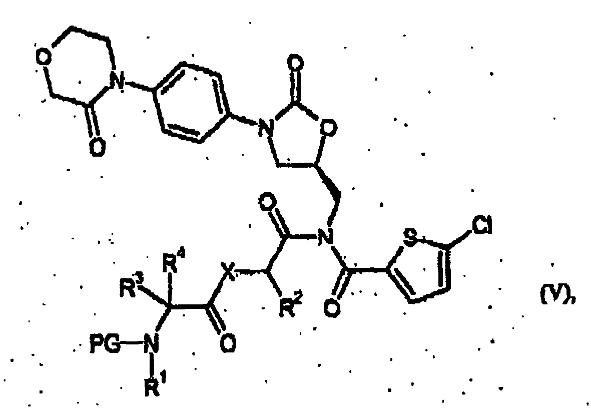 dans laquelle R¹, R², R³, R⁴, PG et X possèdent chacun la signification indiquée ci-dessus,
et le groupe protecteur PG est ensuite éliminé pour donner un composé de formule (I-A) 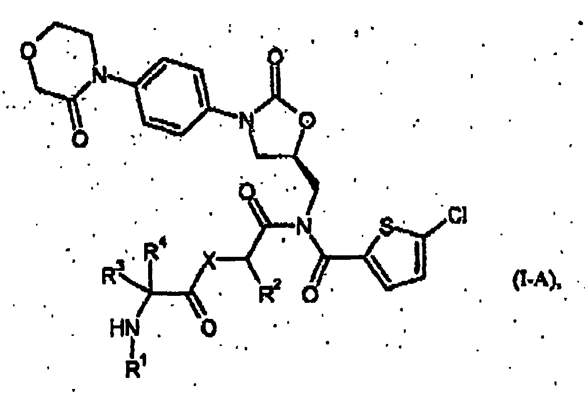 dans laquelle R¹, R², R³, R⁴ et X possèdent chacun la signification indiquée ci-dessus,
ou
[B] le composé (A) est mis à réagir dans un solvant inerte en présence d'une base avec un composé de formule (VI) 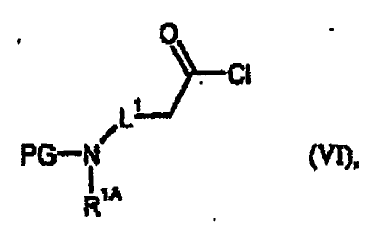 dans laquelle PG possède la signification indiquée ci-dessus,
R^{1A} désigne un alkyle (en C₁-C₄), qui peut être substitué par un hydroxy ou un alcoxy (en C₁-C₄)
et
L¹ représente un groupe alcanediyle (en C₁-C₄), dans lequel un groupe CH₂ peut être échangé contre un atome d'O,
pour donner un composé de formule (VII) 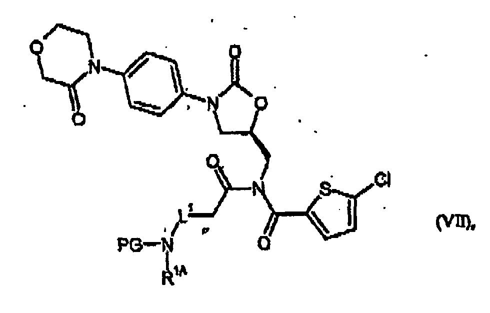 dans laquelle R^{1A}, L' et PG possèdent chacun la signification indiquée ci-dessus,
et le groupe protecteur PG est ensuite éliminé pour donner un composé de formule (I-B) 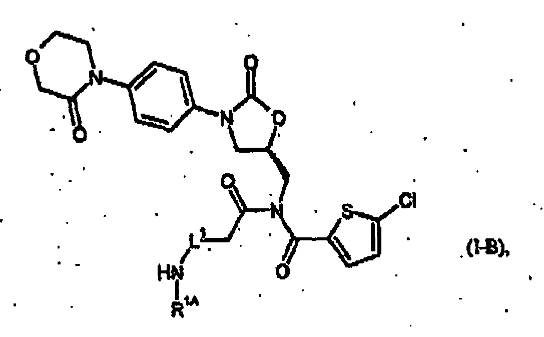 dans laquelle R^{1A} et L¹ possèdent la signification indiquée ci-dessus
ou
[C] le composé (B) 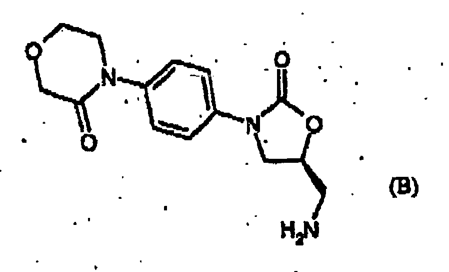 est tout d'abord transformé en un composé de formule (VIII) 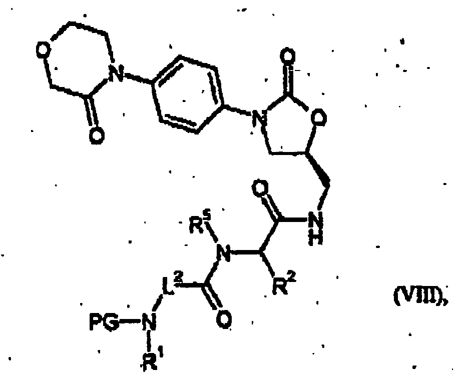 dans laquelle PG, R¹, R² et R⁵ possèdent chacun la signification indiquée dans les revendications 1 à 3
et
L² désigne un groupe (CH₂)₂ ou CR³R⁴, dans lequel R³ et R⁴ possèdent chacun la signification indiquée dans les revendications 1 à 3,
lequel est ensuite mis à réagir dans un solvant inerte en présence d'une base avec un composé de formule (IX) 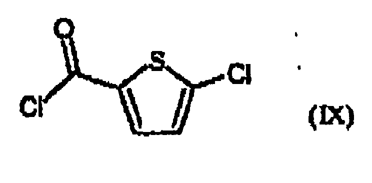 pour donner un composé de formule (X) 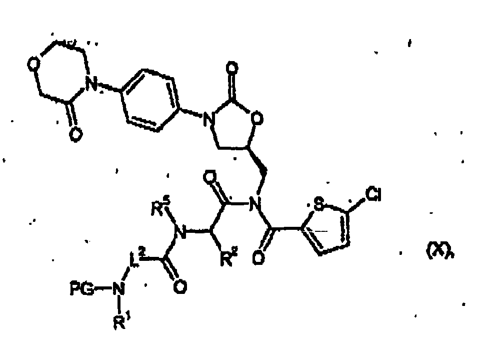 dans laquelle PG, L², R¹, R² et R⁵ possèdent chacun la signification indiquée ci-dessus,
et le groupe protecteur PG est ensuite éliminé pour donner un composé de formule (I-C), 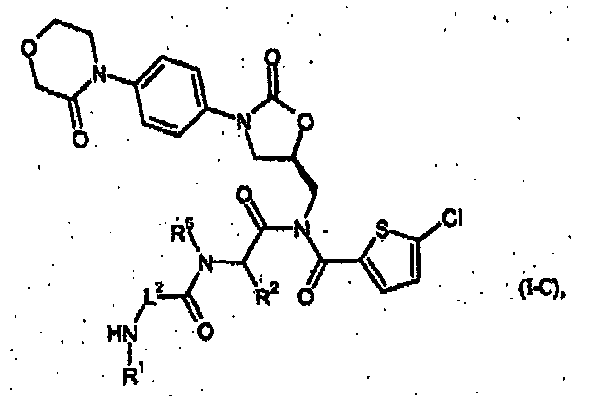 dans laquelle PG, L², R¹, R² et R⁵ possèdent chacun la signification indiquée ci-dessus
ou
[D] le composé (A) est mis à réagir dans un solvant inerte en présence d'une base avec un composé de formule (XI) 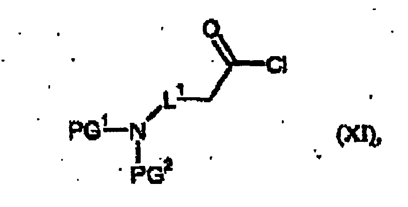
dans laquelle
L¹ représente un groupe alcanediyle (en C₁-C₄), dans lequel un groupe CH₂ peut être échangé contre un atome d'O,
et
PG¹ et PG² représentent indépendamment l'un de l'autre un groupe protecteur de la fonction amine, tel qu'un tert.-butoxycarbonyle (Boc), un benzyloxycarbonyle (Z) ou un p-méthoxybenzyle (PMB) et peuvent être identiques ou différents,
pour donner un composé de formule (XII) 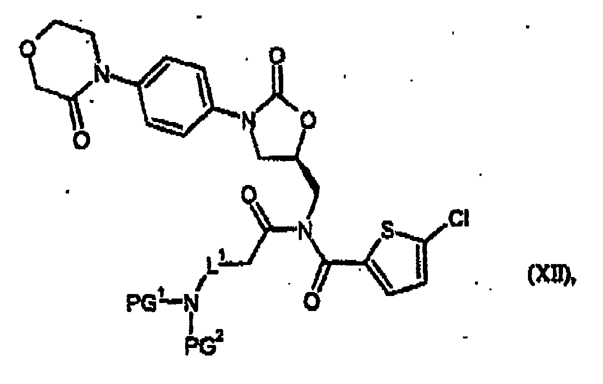 dans laquelle L¹, PG¹ et PG² possèdent chacun la signification indiquée ci-dessus,
et les groupes protecteurs PG¹ et PG² sont ensuite éliminés, simultanément ou consécutivement, pour donner un composé de formule (I-D) 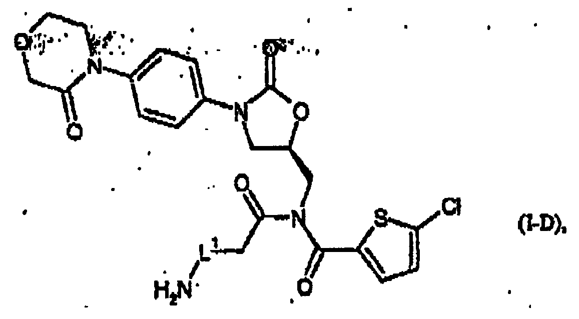 dans laquelle L¹ possède la signification indiquée ci-dessus
et les composés de formule (I-A), (I-B), (I-C) ou (I-D) résultants dans chaque cas sont transformés le cas échéant avec (i) les solvants et/ou (ii) les acides correspondants en leurs solvates, sels et/ou solvates de sels.

5. Composé de formule (I), tels que défini dans l'une des revendications 1 à 3, pour le traitement et/ou la prévention de maladies.

6. Utilisation d'un composé de formule (I), tel que défini dans l'une des revendications 1 à 3, pour la préparation d'un médicament destiné au traitement et/ou à la prévention de maladies thromboemboliques.

7. Médicament contenant un composé de formule (I), tel que défini dans l'une des revendications 1 à 3, le cas échéant en association avec un excipient inerte, non toxique, pharmaceutiquement compatible.

8. Médicament contenant un composé de formule (I), tel que défini dans l'une des revendications 1 à 3, en association avec un autre principe actif.

9. Médicament selon la revendication 7 ou 8 destiné au traitement et/ou à la prévention de maladies thromboemboliques.

10. Médicament selon l'une des revendications 7 à 9 destiné à l'administration intraveineuse.

11. Utilisation d'au moins un composé de formule (I), tel que défini dans l'une des revendications 1 à 3, ou d'un médicament, tel que défini dans l'une des revendications 7 à 10, pour la préparation d'un médicament destiné au traitement et/ou à la prévention de maladies thromboemboliques chez l'homme et l'animal.
